# EUROPEAN PATENT SPECIFICATION

(11) **EP 4 305 033 B1**
(45) Date of publication and mention of the grant of the patent: **07.05.2025**
(21) Application number: 22708933.1
(22) Date of filing: 01.03.2022
(51) Int. Cl.: C07D 471/14, C07D 487/14, A01N 43/40, A01N 43/653

(54) **TRICYCLIC PESTICIDAL COMPOUNDS**
TRICYCLISCHE PESTIZIDVERBINDUNGEN
COMPOSÉS PESTICIDES TRICYCLIQUES

(30) Priority: 09.03.2021 IN 202121009837; 21.04.2021 EP 21169545
(43) Date of publication of application: 17.01.2024
(73) Proprietor: BASF SE, 67056 Ludwigshafen am Rhein (DE)
(72) Inventor: SCHROEDER, Birte, 67056 Ludwigshafen (DE); SHAIKH, Rizwan Shabbir, Mumbai 400705 (IN); MAITY, Pulakesh, Mumbai 400705 (IN); ADISECHAN, Ashokkumar, Mumbai 400705 (IN)
(74) Representative: BASF IP Association
(86) International application number: PCT/EP2022/055029
(87) International publication number: WO 2022/189189

(56) References cited:
- WO-A1-01/07441
- WO-A1-02/12246
- WO-A1-2017/167832

## Description

The invention relates to compounds of formula (I) or an agrochemically or verterinarily acceptable salt, stereoisomer, tautomer, or N-oxide thereof 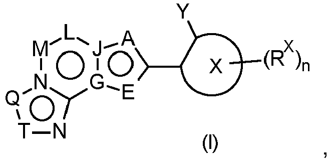 wherein the variables are as defined below. The invention also relates to the use of compounds of formula (I) as an agrochemical pesticide; to pesticidal mixtures comprising a compound of formula (I) and another agrochemically active ingredient; to agrochemical or veterinary compositions comprising a compound of formula (I) or the pesticidal mixture and a liquid or solid carrier; and to seed comprising a compound of formula (I) or the pesticidal mixture. The invention also relates to methods for controlling invertebrate pests, infestation, or infection by invertebrate pests by application of the compounds of formula (I) or the pesticidal mixtures comprising them.

Invertebrate pests and in particular insects, arachnids and nematodes destroy growing and harvested crops and attack wooden dwelling and commercial structures, thereby causing large economic loss to the food supply and to property. Accordingly, there is an ongoing need for new agents for combating invertebrate pests.

WO2017/167832A1 discloses bicyclic compounds and their use as agrochemical pesticides, whereas tricyclic compounds are not described.

Due to the ability of target pests to develop resistance to pesticidally active agents, there is an ongoing need to identify further compounds, which are suitable for combating invertebrate pests such as insects, arachnids and nematodes. Furthermore, there is a need for new compounds having a high pesticidal activity and showing a broad activity spectrum against a large number of different invertebrate pests, especially against difficult to control insects, arachnids and nematodes. There is furthermore a need to find compounds that display a higher efficacy as compared with known pesticides, which reduces the application rates and costs for the applicant, and decreases the environmental effects on soil and ground water.

It is therefore an object of the present invention to identify and provide compounds, which exhibit a high pesticidal activity and have a broad activity spectrum against invertebrate pests.

It has been found that these objects can be achieved by substituted tricyclic compounds of formula I as depicted and defined below, including their stereoisomers, their salts, in particular their agriculturally or veterinarily acceptable salts, their tautomers and their N-oxides.

Therefore, the invention provides in a first aspect compounds of formula (I), or an agrochemically or veterinarily acceptable salt, stereoisomer, tautomer, or N-oxide thereof

Compounds of formula (I), or an agrochemically or veterinarily acceptable salt, stereoisomer, tautomer, or N-oxide thereof 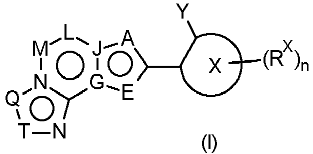 wherein the variables in formula (I) have the following meaning
- A: is CH, N, or NH;
- E: is N, O, S, NR^{E}, or CR^{E};
- G, J: are independently C or N, provided that only one of E or G is N;
- L: is N or CR^{L};
- M: is N or CR^{M};
- Q: is N or CR^{Q}
- T: is N or CR^{T};
- X: is phenyl, or a 5- or 6-membered heteroaryl;
- Y: is SR^{Y1}, S(O)R^{Y1}, S(O)₂R^{Y1}, S(=O)(=NR^{Y2})R^{Y1}, or S(=NR^{Y2})(=NR^{Y3})R^{Y1};
R^{E}, R^{L}, R^{M}, R^{Q} and R^{T} are independently H, halogen, N₃, CN, NO₂, SCN, SF₅; C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are halogenated or non-halogenated,
   C(=O)OR¹, NR²R³, C₁-C₆-alkylen-NR²R³, O-C₁-C₆-alkylen-NR²R³, C₁-C₆-alkylen-CN, NH-C₁-C₆-alkylen-NR²R³, C(=O)NR²R³, C(=O)R⁴, SO₂NR²R³, S(=O)ₘR⁵, OR⁶, SR⁶, or CH₂R⁶;
   phenyl, which is unsubstituted or substituted with one or more, same or different substituents R¹¹;
      R¹ H;
      C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which groups are halogenated or non-halogenated;
      C₁-C₆-alkylen-NR²R³, C₁-C₆-alkylen-CN, or CH₂R⁶; or phenyl, which is unsubstituted, or substituted with one or more, same or different substituents R¹¹;
      R¹¹ is halogen, N₃, OH, CN, NO₂, SCN, SF₅;
      C₁-C₆-alkyl, C₁-C₆-alkoxy, C₂-C₆-alkenyl , C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are halogenated or non-halogenated;
      R² is H;
      C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted, or substituted with one or more, same or different substitutent selected from halogen, CN and HO.
      C(=O)R²¹, C(=O)OR²¹, C(=O)NR²¹, C₁-C₆-alkylen-CN, or CH₂R⁶; or
      phenyl, which is unsubstituted or substituted with one or more, same or different substituents R¹¹;
         R²¹ is H;
         C₁-C₆-alkyl, C₁-C₆-haloalkyl, C₂-C₆-alkenyl, C₂-C₆-alkynyl, C₁-C₆-alkoxy-C₁-C₄-alkyl, C₃-C₆-cycloalkyl;
         C₃-C₆-cycloalkyl-C₁-C₄-alkyl, C₃-C₆-cycloalkoxy-C₁-C₄ alkyl, phenyl, or a saturated, partially-, or fully unsaturated 5- or 6-membered heterocycle, wherein the cyclic moieties are unsubstituted or substituted with one or more, same or different substituents R¹¹;

      R³ is H;
      C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are halogenated or non-halogenated;
      C₁-C₆-alkylen-CN, or CH₂R⁶;
      phenyl, which is unsubstituted or substituted with one or more, same or different substituents R¹¹; or
      NR²R³ may also form an N-bound, saturated 3- to 8-membered heterocycle, which in addition to the nitrogen atom may have 1 or 2 further heteroatoms or heteroatom moieties selected from O, S(=O)ₘ, NH, and N-C₁-C₆-alkyl, and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;
      R⁴ is H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which are unsubstituted or substituted with one or more, same of different substituents selected from halogen, CN, and OH;
      CH₂R⁶, or phenyl, which is unsubstituted, or substituted with one or more, same or different substituents R¹¹;
      R⁵ is C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which groups are halogenated or non-halogenated;
      C₁-C₆-alkylen-NR²R³, C₁-C₆-alkylen-CN, CH₂R⁶; or
      phenyl, which is unsubstituted, or substituted with one or more, same or different substituents R¹¹;
      R⁶ is phenyl, which is unsubstituted or substituted with one or more, same or different substituents R¹¹;
each R^{X} is independently halogen, N₃, OH, CN, NO₂, SCN, SF₅;
   C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are unsubstituted or substituted with CN or halogen;
   C(=O)OR¹, NR²R³, C(=O)NR²R³, C(=O)R⁴, SO₂NR²R³, S(=O)ₘR¹, OR⁶, SR⁶, CH₂R⁶, OC(=O)R⁴, NR³C(=O)R⁴, OC(=O)OR¹, OC(=O)NR²R³, OC(=O)SR¹, OC(=S)NR²R³, OC(=S)SR¹, ONR²R³, ON=CR¹R⁴, N=CR¹R⁴, NNR², NR³C(=O)R⁷, SC(=O)SR¹, SC(=O)NR²R³, C(=S)R⁶, C(=S)OR⁴, C(=NR²)R⁴, C(R⁸)=NO(R⁹);
   phenyl, which is unsubstituted or substituted with one or more, same or different substituents R¹¹;
   a 5- or 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents R³¹, and wherein said N- and S-atoms are independently oxidized, or non-oxidized; or
   a group of formula (S) 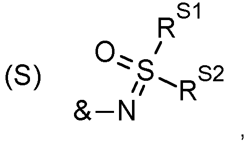
   wherein each R^{S1}, R^{S2} is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or halogenated;
   a 3- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said N- and S-atoms are independently oxidized, or non-oxidized;
   phenyl, which is unsubstituted, or substituted with one or more, same or different substituents selected from halogen, C₁-C₃-alkyl, C₁-C₃-alkoxy, C₁-C₃-haloalkyl, and C₁-C₃-haloalkoxy;
   or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to;
   R³¹ is halogen, N₃, OH, CN, NO₂, SCN, SF₅;
      C₁-C₆-alkyl, C₁-C₆-haloalkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₁-C₆-alkoxycarbonyl, C₃-C₆-cycloalkyl;
      C₃-C₆-cycloalkoxy, C₃-C₆-cycloalkyl-C₁-C₄-alkyl, C₃-C₆-cycloalkoxy-C₁-C₄ alkyl, phenyl, or a saturated, partially-, or fully unsaturated 5- or 6-membered heterocycle, wherein the cyclic moieties are unsubstituted or substituted with one or more, same or different substituents R¹¹; or
      two geminal substituents R³¹ form together with the atom to which they are bound a group =O or =S;
   each R⁷ is independently C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from CN and halogen;
   each R⁸ is independently H, CN, OH, C₁-C₆-alkyl, C₂-C₆-alkenyl, C₂-C₆-alkynyl, C₁-Cₐ-alkoxy-C₁-C₄-alkyl, C₃-C₆-cycloalkyl, C₃-C₆-cycloal kyl-C₁-C₄-alkyl, C₃-C₆-cycloalkoxy-C₁-C₄-alkyl, which groups are unsubstituted or substituted with halogen;
      phenyl or benzyl, wherein the phenyl ring is unsubstituted or substituted with one or more, same or different substituents R¹¹;
   each R⁹ is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen and CN;
      phenyl or benzyl, wherein the phenyl ring is unsubstituted or substituted with one or more, same or different substitutents R¹¹;
   each R^{Y1} R^{Y2}, R^{Y3} is independently selected from H, C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more, same or different substituents selected from halogen and CN;
      a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, CN, C₁-C₃-alkyl, C₁-C₃-alkoxy, C₁-C₃-haloalkyl, C₁-C₃-haloalkoxy, and wherein said N- and S-atoms are independently oxidized, or non-oxidized;
      phenyl, which is unsubstituted, or substituted with one or more, same or different substituents selected from halogen, CN, C₁-C₃-alkyl, C₁-C₃-alkoxy, C₁-C₃-haloalkyl, C₁-C₃-haloalkoxy;
      or two substituents R^{Y1}, R^{Y2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, CN, C₁-C₃-alkyl, C₁-C₃-alkoxy, C₁-C₃-haloalkyl, C₁-C₃-haloalkox, and wherein said heterocycle comprises no, one or more, same or different heteroatoms O, N, or S in addition to the sulfur atom to which R^{Y1} and R^{Y2} are bound to;

the index n is 0, 1, 2, 3, or 4 if X is phenyl or a 6-membered heteroaryl;
   or 0, 1, 2, or 3 if X is a 5-membered heteroaryl; and
the index m is 0, 1, or 2.

The tricyclic compounds of the formula (I), and their agriculturally acceptable salts are highly active against animal pest, *i.e.* harmful arthropodes and nematodes, especially against insects and acaridae which are difficult to control by other means.

Moreover, the present invention discloses the following embodiments (wherein methods for treatment of the human or animal body by surgery or therapy are excluded):
- compositions comprising at least one compound of formula (I) as defined above;
- agricultural and veterinary compositions comprising an amount of at least one compound of formula (I) or an enantiomer, diasteromer or salt thereof as defined above;
- methods for combating invertebrate pests, infestation, or infection by invertebrate pests, which method comprises contacting said pest or its food supply, habitat or breeding grounds with a pesticidally effective amount of at least one compound of formula (I) as defined above or a composition thereof;
- methods for controlling invertebrate pests, infestation, or infection by invertebrate pests, which method comprises contacting said pest or its food supply, habitat or breeding grounds with a pesticidally effective amount of at least one compound of formula (I) as defined above or a composition comprising at least one compound of formula (I);
- methods for preventing or protecting against invertebrate pests comprising contacting the invertebrate pests, or their food supply, habitat or breeding grounds with substituted imidazolium compounds of the general formula (I) as defined above or a composition comprising at least one compound of formula (I) as defined above or a composition comprising at least one compound of formula (I);
- methods for protecting crops, plants, plant propagation material and/or growing plants from attack or infestation by invertebrate pests comprising contacting or treating the crops, plants, plant propagation material and growing plants, or soil, material, surface, space, area or water in which the crops, plants, plant propagation material is stored or the plant is growing, with a pesticidally effective amount of at least one compound of formula (I) as defined above or a composition comprising at least one compound of formula (I);
- non-therapeutic methods for treating animals infested or infected by parasites or preventing animals of getting infected or infested by parasites or protecting animals against infestation or infection by parasites which comprises orally, topically or parenterally administering or applying to the animals a parasiticidally effective amount of a compound of formula (I) as defined above or a composition comprising at least one compound of formula (I);
- methods for treating, controlling, preventing or protecting animals against infestation or infection by parasites by administering or applying orally, topically or parenterally to the animals a substituted imidazolium compound of the general formula (I) as defined above or a composition comprising at least one compound of formula (I);
- seed comprising a compound of formula (I) as defined above, in an amount of from 0.1 g to 10 kg per 100 kg of seed;
- the use of the compounds of formula (I) as defined above for protecting growing plants or plant propagation material from attack or infestation by invertebrate pests;
- the use of compounds of formula (I) or the enantiomers, diastereomers or veterinary acceptable salts thereof for combating parasites in and on animals;
- a process for the preparation of a veterinary composition for treating, controlling, preventing or protecting animals against infestation or infection by parasites which comprises adding a parasiticidally effective amount of an compound of formula (I) or the enantiomers, diastereomers and/or veterinary acceptable salt thereof to a carrier composition suitable for veterinary use;
- the use of a compound of formula (I) or the enantiomers, diastereomers and/or veterinary acceptable salt thereof for the preparation of a medicament for treating, controlling, preventing or protecting animals against infestation or infection by parasites.

All the compounds of formula (I) if applicable their stereoisomers, their tautomers, their salts or their N-oxides as well as compositions thereof are particularly useful for controlling invertebrate pests, in particular for controlling arthropods and nematodes and especially insects. Therefore, the invention relates to the use of a compound of formula (I) as an agrochemical pesticide, preferably for combating or controlling invertebrate pests, in particular invertebrate pests of the group of insects, arachnids or nematodes.

The term "compound(s) according to the invention" or "compound(s) of formula (I)" as used in the present invention refers to and comprises the compound(s) as defined herein and/or stereoisomer(s), salt(s), tautomer(s) or N-oxide(s) thereof. The term "compound(s) of the present invention" is to be understood as equivalent to the term "compound(s) according to the invention", therefore also comprising stereoisomer(s), salt(s), tautomer(s) or N-oxide(s) of compounds of formula (I).

Ther terms "tricyclic scaffold" or "tricyclic moiety" relate to the following moiety of formula (I) 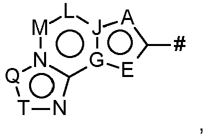 wherein "#" means the remainder of formula (I) and wherein the other variables have a meaninga as defined form formula (I). For the avoidance of doubt, the circles in the three adjoined rings mean that the tricyclic scaffold is fully unsaturated, typically aromatic

The term "composition(s) according to the invention" or "composition(s) of the present invention" encompasses composition(s) comprising at least one compound of formula (I) according to the invention as defined above, therefore also including a stereoisomer, an agriculturally or veterinary acceptable salt, tautomer or an N-oxide of the compounds of formula (I).

The compounds of the present invention may be amorphous or may exist in one or more different crystalline states (polymorphs) or modifications which may have a different macroscopic properties such as stability or show different biological properties such as activities. The present invention includes both amorphous and crystalline compounds of the formula (I), mixtures of different crystalline states or modifications of the respective compound of formula ()), as well as amorphous or crystalline salts thereof.

The compounds of the formula (I) have one or, depending on the substitution pattern, more centers of chirality, in which case they are present as mixtures of enantiomers or diastereomers. The invention provides both the single pure enantiomers or pure diastereomers of the compounds of formula (I), and their mixtures and the use according to the invention of the pure enantiomers or pure diastereomers of the compound of formula (I) or its mixtures. Suitable compounds of the formula (I) also include all possible geometrical stereoisomers (cis/trans isomers) and mixtures thereof. Cis/trans isomers may be present with respect to an alkene, carbon-nitrogen double-bond or amide group. The term "stereoisomer(s)" encompasses both optical isomers, such as enantiomers or diastereomers, the latter existing due to more than one center of chirality in the molecule, as well as geometrical isomers (cis/trans isomers). The present invention relates to every possible stereoisomer of the compounds of formula (I), *i.e.* to single enantiomers or diastereomers, as well as to mixtures thereof.

Depending on the substitution pattern, the compounds of the formula (I) may be present in the form of their tautomers. Hence the invention also relates to the tautomers of the formula (I) and the stereoisomers, salts, tautomers and N-oxides of said tautomers.

Salts of the compounds of the formula (I) are preferably agriculturally and/or veterinary acceptable salts. They can be formed in a customary method, e.g. by reacting the compound with an acid of the anion in question if the compound of formula (I) has a basic functionality or by reacting an acidic compound of formula (I) with a suitable base.

Suitable agriculturally or veterinary useful salts are especially the salts of those cations or the acid addition salts of those acids whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethyl-ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formulae I with an acid of the corresponding anion, preferably of hydrochloric acid, hydrobromic acid, sulfuric acid, phosphoric acid or nitric acid.

The term "N-oxide" includes any compound of the present invention which has at least one tertiary nitrogen atom that is oxidized to an N-oxide moiety.

The organic moieties groups mentioned in the above definitions of the variables are - like the term halogen - collective terms for individual listings of the individual group members. The prefix Cₙ-Cₘ indicates in each case the possible number of carbon atoms in the group. "Halogen" will be taken to mean F, Cl, Br, and I, preferably F.

The term "substituted with", e.g. as used in "partially, or fully substituted with" means that one or more, e.g. 1, 2, 3, 4 or 5 or all of the hydrogen atoms of a given radical have been replaced by one or more, same or different substituents, such as a halogen, in particular F. Accordingly, for substituted cyclic moieties, e.g. 1-cyanocyclopropyl, one or more of the hydrogen atoms of the cyclic moiety may be replaced by one or more, same or different substituents.

Likewise, the term "halogenated" means that one or more, e.g. 1, 2, 3, 4, or 5 or all of the hydrogen atoms of a given radical have been replaced by one or more, same or different halogen atoms, such as F.

The term "Cₙ-Cₘ-alkyl" as used herein (and also in Cₙ-Cₘ-alkylamino, di-Cₙ-Cₘ-alkylamino, Cₙ-Cₘ-alkylaminocarbonyl, di-(Cₙ-Cₘ-alkylamino)carbonyl, Cₙ-Cₘ-alkylthio, Cₙ-Cₘ-alkylsulfinyl and Cₙ-Cₘ-alkylsulfonyl) refers to a branched or unbranched saturated hydrocarbon group having n to m, e.g. 1 to 10 carbon atoms, preferably 1 to 6 carbon atoms, for example methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "Cₙ-Cₘ-haloalkyl" as used herein (and also in Cₙ-Cₘ-haloalkylsulfinyl and Cₙ-Cₘ-haloalkylsulfonyl) refers to a straight-chain or branched alkyl group having n to m carbon atoms, e.g. 1 to 10 in particular 1 to 6 carbon atoms (as mentioned above), where some or all of the hydrogen atoms in these groups may be replaced by halogen atoms as mentioned above, for example C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like. The term C₁-C₁₀-haloalkyl in particular comprises C₁-C₂-fluoroalkyl, which is synonym with methyl or ethyl, wherein 1, 2, 3, 4 or 5 hydrogen atoms are substituted with fluorine atoms, such as fluoromethyl, difluoromethyl, trifluoromethyl, 1-fluoroethyl, 2-fluoroethyl, 2,2-difluoroethyl, 2,2,2-trifluoroethyl and pentafluoromethyl.

Similarly, "Cₙ-Cₘ-alkoxy" and "Cₙ-Cₘ-alkylthio" (or Cₙ-Cₘ-alkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen (or sulfur linkages, respectively) at any bond in the alkyl group. Examples include C₁-C₄-alkoxy such as methoxy, ethoxy, propoxy, isopropoxy, butoxy, sec-butoxy, isobutoxy and tert-butoxy, further C₁-C₄-alkylthio such as methylthio, ethylthio, propylthio, isopropylthio, and n-butylthio.

Accordingly, the terms "Cₙ-Cₘ-haloalkoxy" and "Cₙ-Cₘ-haloalkylthio" (or Cₙ-Cₘ-haloalkyl-sulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group, where some or all of the hydrogen atoms in these groups may be replaced by halogen atoms as mentioned above, for example C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy, further C₁-C₂-haloalkylthio, such as chloromethylthio, bromomethylthio, dichloromethylthio, trichlorome-thylthio, fluoromethylthio, difluoromethylthio, trifluoromethylthio, chlorofluoromethylthio, dichloro-fluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluo-roethylthio and the like. Similarly, the terms C₁-C₂-fluoroalkoxy and C₁-C₂-fluoroalkylthio refer to C₁-C₂-fluoroalkyl which is bound to the remainder of the molecule via an oxygen atom or a sulfur atom, respectively.

The term "C₂-Cₘ-alkenyl" as used herein intends a branched or unbranched unsaturated hydrocarbon group having 2 to m, e.g. 2 to 10 or 2 to 6 carbon atoms and a double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "C₂-Cₘ-alkynyl" as used herein refers to a branched or unbranched unsaturated hydrocarbon group having 2 to m, e.g. 2 to 10 or 2 to 6 carbon atoms and containing at least one triple bond, such as ethynyl, propynyl, 1-butynyl, 2-butynyl, and the like.

The term "Cₙ-Cₘ-alkoxy-Cₙ-Cₘ-alkyl" as used herein refers to alkyl having n to m carbon atoms, e.g. like specific examples mentioned above, wherein one hydrogen atom of the alkyl radical is replaced by an Cₙ-Cₘ-alkoxy group; wherein the value of n and m of the alkoxy group are independently chosen from that of the alkyl group.

The suffix "-carbonyl" in a group or "C(=O)" denotes in each case that the group is bound to the remainder of the molecule via a carbonyl C=O group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl.

The term "aryl" as used herein refers to a mono-, bi- or tricyclic aromatic hydrocarbon radical such as phenyl or naphthyl, in particular phenyl (also referred as to C₆H₅ as subsitituent).

The term "C₃-Cₘ-cycloalkyl" as used herein refers to a monocyclic ring of 3- to m-membered saturated cycloaliphatic radicals, e.g. cyclopropyl, cyclobutyl, cyclopentyl, cyclohexyl, cycloheptyl, cyclooctyl and cyclodecyl.

The term "alkylcycloalkyl" denotes as well as the term "alkyl which may be substituted with cycloalkyl" an alkyl group which is substituted with a cycloalkyl ring, wherein alkyl and cycloakyl are as herein defined.

The term "cycloalkylalkyl" denotes as well as the term "cycloalkyl which may be substituted with alkyl" a cycloalkyl ring which is substituted with an alkyl group, wherein alkyl and cycloakyl are as herein defined.

The term "alkylcycloalkylalkyl" denotes as well as the term "alkylcycloalkyl which may be substituted with alkyl" an alkylcycloalkyl group which is substituted with an alkyl, wherein alkyl and alkylcycloakyl are as herein defined.

The term " C₃-Cₘ-cycloalkenyl" as used herein refers to a monocyclic ring of 3- to m-membered partially unsaturated cycloaliphatic radicals.

The term "cycloalkylcycloalkyl" denotes as well as the term "cycloalkyl which may be substituted with cycloalkyl" a cycloalkyl substitution on another cycloalkyl ring, wherein each cycloalkyl ring independently has from 3 to 7 carbon atom ring members and the cycloalkyls are linked through one single bond or have one common carbon atom. Examples of cycloalkylcycloalkyl include cyclopropylcyclopropyl (e.g. 1,1'-bicyclopropyl-2-yl), cyclohexylcyclohexyl wherein the two rings are linked through one single common carbon atom (e.g. 1,1'-bicyclohexyl-2-yl), cyclohexylcyclopentyl wherein the two rings are linked through one single bond (e.g. 4-cyclopentylcyclohexyl) and their different stereoisomers such as (1R,2S)-1, 1'-bicyclopropyl-2-yl and (1R,2R)-1,1'-bicyclopropyl-2-yl.The term "carbocycle" or "carbocyclyl" includes, unless otherwise indicated, in general a 3- to 12-membered, preferably a 3- to 8-membered or a 5- to 8-membered, more preferably a 5- or 6-membered mono-cyclic, ring comprising 3 to 12, preferably 3 to 8 or 5 to 8, more preferably 5 or 6 carbon atoms.

The carbocyclic radicals may be saturated, partially unsaturated, or fully unsaturated. Preferably, the term "carbocycle" covers cycloalkyl and cycloalkenyl groups as defined above, for example cyclopropane, cyclobutane, cyclopentane and cyclohexane rings. When it is referred to "fully unsaturated" carbocycles, this term also includes "aromatic" carbocycles. In certain preferred embodiments, a fully unsaturated carbocycle is an aromatic carbocycle as defined below, preferably a 6-membered aromatic carbocycle.

The term "heteroaryl" or "aromatic heterocycle" or "aromatic heterocyclic ring" includes mono-cyclic 5- or 6-membered heteroaromatic radicals comprising as ring members 1, 2, 3 or 4 heteroatoms selected from N, O and S. Examples of 5- or 6-membered heteroaromatic radicals include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxa-diazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl. The term "heteroaryl" also includes bicyclic 8 to 10-membered heteroaromatic radicals comprising as ring members 1, 2 or 3 heteroatoms selected from N, O and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimid-azolyl and the like. These fused heteroaryl radicals may be bonded to the remainder of the molecule via any ring atom of 5- or 6-membered heteroaromatic ring or via a carbon atom of the fused phenyl moiety.

The terms "heterocycle", "heterocyclyl" or "heterocyclic ring" includes, unless otherwise indicated, in general 3- to 12-membered, preferably 3- to 8-membered, 3- to 7-membered, or 5- to 8-membered, more preferably 5- or 6-membered, in particular 6-membered monocyclic heterocyclic radicals. The heterocyclic radicals may be saturated, partially unsaturated, or fully unsaturated. As used in this context, the term "fully unsaturated" also includes "aromatic". In a preferred embodiment, a fully unsaturated heterocycle is thus an aromatic heterocycle, preferably a 5- or 6-membered aromatic heterocycle comprising one or more, e.g. 1, 2, 3, or 4, preferably 1, 2, or 3 heteroatoms selected from N, O and S as ring members. Examples of aromatic heterocycles are provided above in connection with the definition of "heteroaryl". Unless otherwise indicated, "heteroaryls" are thus covered by the term "heterocycles". The heterocyclic non-aromatic radicals usually comprise 1, 2, 3, 4 or 5, preferably 1, 2 or 3 heteroatoms selected from N, O and S as ring members, where S-atoms as ring members may be present as S, SO or SO₂. Examples of 5- or 6-membered heterocyclic radicals comprise saturated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxothiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxodihydrothiopyranyl, S-dioxodihydrothiopyranyl, tetrahydrothiopyranyl, S-oxotetra-hydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-onyl and the like.

The erms "alkylene", "alkenylene", and "alkynylene" refer to alkyl, alkenyl, and alkynyl as defined above, respectively, which are bonded to the remainder of the molecule, via two atoms, preferably via two carbon atoms, of the respective group, so that they represent a linker between two moieties of the molecule. In particular, the term "alkylene" may refer to alkyl chains such as CH₂CH₂, -CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂. Similarly, "alkenylene" and "alkynylene" may refer to alkenyl and alkynyl chains, respectively.

The term "5- to 6-membered carbocyclic ring" as used herein refers to cyclopentane and cyclohexane rings.

Examples of 5- or 6-membered saturated heterocyclic rings include: 2-tetrahydrofuranyl, 3-tetrahydrofuranyl, 2-tetrahydrothienyl, 3-tetrahydrothienyl, 2-pyrrolidinyl, 3-pyrrolidinyl, 3-pyrazolidinyl, 4-pyrazolidinyl, 5-pyrazolidinyl, 2-imidazolidinyl, 4-imidazolidinyl, 2-oxazolidinyl, 4-oxazolidinyl, 5-oxazolidinyl, 3-isoxazolidinyl, 4-isoxazolidinyl, 5-isoxazolidinyl, 2-thiazolidinyl, 4-thiazolidinyl, 5-thiazolidinyl, 3-isothiazolidinyl, 4-isothiazolidinyl, 5-isothiazolidinyl, 1,2,4-oxadiazolidin-3-yl, 1,2,4-oxadiazolidin 5 yl, 1,2,4-thiadiazolidin-3-yl, 1,2,4-thiadiazolidin-5-yl, 1,2,4-triazolidin-3-yl,-1,3,4-oxadiazolidin-2-yl, 1,3,4-thiadiazolidin-2-yl, 1,3,4-triazolidin-2-yl, 2-tetrahydropyranyl, 4-tetrahydropyranyl, 1,3-dioxan-5-yl, 1,4-dioxan-2-yl, 2-piperidinyl, 3-piperidinyl, 4-piperidinyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl, 2-morpholinyl, 3-morpholinyl, 2-thiomorpholinyl, 3-thiomorpholinyl, 1-oxothiomorpholin-2-yl, 1-oxothiomorpholin-3-yl, 1,1-dioxothiomorpholin-2-yl, 1,1-dioxothiomorpholin-3-yl.

Examples of 5- or 6-membered partially unsaturated heterocyclyl or heterocyclic rings include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin 3 yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3 dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl.

Examples of 5- or 6-membered fully unsaturated heterocyclic (heteroaryl) or heteroaromatic rings are: 2-furyl, 3-furyl, 2-thienyl, 3-thienyl, 2-pyrrolyl, 3-pyrrolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

A "C₂-Cₘ-alkylene" is divalent branched or preferably unbranched saturated aliphatic chain having 2 to m, e.g. 2 to 7 carbon atoms, for example CH₂CH₂, -CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂.

### Preparation Methods

The compounds of formula (I) can be prepared by standard methods of organic chemistry. If certain derivatives cannot be prepared by the processes outlined below, they can be obtained by derivatization of other compounds of formula (I) that are accessible by these methods. The definition of variable for the following compounds and intermediates is - unless otherwise provided - as defined for formula (I). The preferred meanings of variables according as defined herein is also valid for the following formulae (1) to (20).

Compounds of formula (11) are generally very useful building blocks for the synthesis of certain compounds of formula (I). Such compounds are accessible via a cascade of preparation steps as displayed under General Scheme 1 below:

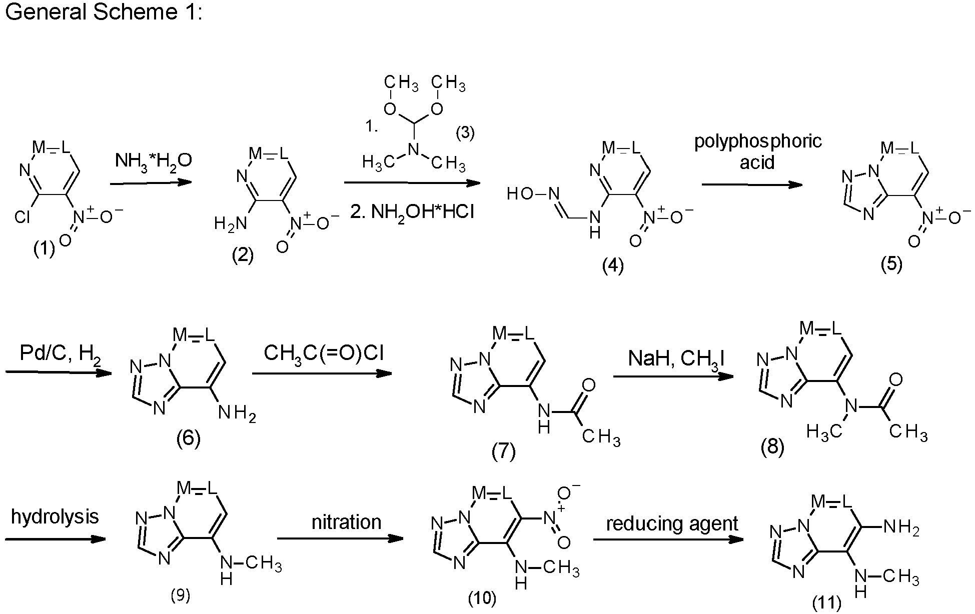

In a first step, compounds of formula (1) may be reacted with NH₃ to yield compounds of formula (2). The reaction is typically carried out in a polar solvent at a temperature of from 50 to 120 °C. Suitable solvents include halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, CH₃OC(CH₃)₃ (MTBE), CH₃OCH₃ (DME), CH₃OCH₂CH₂OCH₃, dioxane, anisole, and tetrahydrofurane (THF); nitriles, preferably C₁-C₆-nitriles, such as CH₃CN, and CH₃CH₂CN; alcohols, preferably C₁-C₄-alcohols, such as CH₃OH, CH₃CH₂OH, CH₃CH₂CH₂OH, CH₃CH(OH)CH₃, CH₃(CH₂)₃OH, and C(CH₃)₃OH; amides and urea derivatives, preferably dimethyl formamide (DMF), N-methyl-2-pyrrolidone (NMP), dimethyl acetamide (DMA), 1,3-dimethyl-2-imidazolidinone (DMI), 1,3-dimethyl-3,4,5,6-tetrahydro-2(1H)-pyrimidinone (DMPU), hexamethylphosphamide (HMPA); moreover dimethyl sulfoxide (DMSO), sulfolane, H₂O, and mixtures thereof.

Compounds of formula (3) may then be reacted first with a compound of formula (4) at a temperature of from 50 to 120 °C in a polar solvent. Suitable solvents include halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, MTBE, DME, CH₃OCH₂CH₂OCH₃, dioxane, anisole, and THF; nitriles, preferably C₁-C₆-nitriles, such as CH₃CN, and CH₃CH₂CN; alcohols, preferably C₁-C₄-alcohols, such as CH₃OH, CH₃CH₂OH, CH₃CH₂CH₂OH, CH₃CH(OH)CH₃, CH₃(CH₂)₃OH, and C(CH₃)₃OH; amides and urea derivatives, preferably DMF, NMP, DMA, DMI, DMPU, HMPA; moreover DMSO, sulfolane, H₂O, and mixtures thereof. Second, NH₂OH may be added to the reaction mixture to yield compounds of formula (4). Typically, NH₂OH is added at a temperature of from 0 to 40 °C, which temperature may then be raised to 60 to 120 °C.

Compounds of formula (4) may then be converted to compounds of formula (5) in a condensation reaction in the presence of a catalyst at a temperature of from 80 to 150 °C in a polar solvent. Suitable solvents include halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, MTBE, DME, CH₃OCH₂CH₂OCH₃, dioxane, anisole, and THF; esters, preferably esters of aliphtic C₁-C₆-alcohols with aliphatic C₁-C₆-carboxylic acids, esters of aromatic C₆-C₁₀-alcohols with aromatic C₆-C₁₀-carboxylic adcids, cyclic esters of ω-hydroxy-C₁-C₆-carboxylic acids, such as CH₃C(O)OCH₂CH₃, CH₃C(O)OCH₃, CH₃C(O)OCH₂CH₂CH₂CH₃, CH₃C(O)OCH(CH₃)CH₂CH₃, CH₃C(O)OC(CH₃), CH₃CH₂CH₂C(O)OCH₂CH₃, CH₃CH(OH)C(O)OCH₂CH₃, CH₃CH(OH)C(O)OCH₃, CH₃C(O)OCH₂CH(CH₃)₂, CH₃C(O)OCH(CH₃)₂, CH₃CH₂C(O)OCH₃, benzyl benzoate, and γ-butyrolactone; carbonates, such as ethylene carbonate, propylene carbonate, CH₃CH₂OC(O)OCH₂CH₃, and CH₃OC(O)OCH₃; nitriles, preferably C₁-C₆-nitriles, such as CH₃CN, and CH₃CH₂CN; ketones, preferably C₁-C₆-alkyl-C₁-C₆-alkyl ketones, such as CH₃C(O)CH₃, CH₃C(O)CH₂CH₃, CH₃CH₂C(O)CH₂CH₃, and CH₃C(O)C(CH₃)₃ (MTBK); alcohols, preferably C₁-C₄-alcohols, such as CH₃OH, CH₃CH₂OH, CH₃CH₂CH₂OH, CH₃CH(OH)CH₃, CH₃(CH₂)₃OH, and C(CH₃)₃OH; amides and urea derivatives, preferably DMF,NMP, DMA, DMI, DMPU, HMPA; moreover DMSO, sulfolane, and H₂O. Suitable catalysts in include strong inorganic acids like polyphosphoric acid, H₂SO₄.

Compounds of formula (5) may then be hydrated with H₂ in the presence of a catalyst in an inert solvent. Typical catalysts include Pd on C. Suitable solvents include halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, MTBE, DME, CH₃OCH₂CH₂OCH₃, dioxane, anisole, and THF; alcohols, preferably C₁-C₄-alcohols, such as CH₃OH, CH₃CH₂OH, CH₃CH₂CH₂OH, CH₃CH(OH)CH₃, CH₃(CH₂)₃OH, and C(CH₃)₃OH; H₂O, and mixtures thereof.

Compounds of formula (6) may then be methylated by introducing a suitable protection group at the primary amine, such as an acetate group (compounds of formula (7)), followed by a methylation step (to yield compounds of formula (8) and a hydrolysis step to yield compounds of formula (9). Such reactions are known to the skilled person and may be carried out under reaction conditions known in prior art.

Compounds of formula (9) are then nitrated under routine conditions, e.g. by addition of HNO₃ in H₂SO₄ to yield compounds of formula (10).

Compounds of formula (10) may finally be converted to compounds of formula (11) by addition of a reducing agent in an inert solvent. Suitable reducing agents include LiAlH₄, LiBH₄, BH₃, or SnCl₂ in a protic solvent. Such reaction conditions are equally routine methods for the skilled person.

Compounds of formula (11) are then be reacted with a carbonic acid compound of formula (12) in the presence of a coupling agent to yield compounds of formula (13) 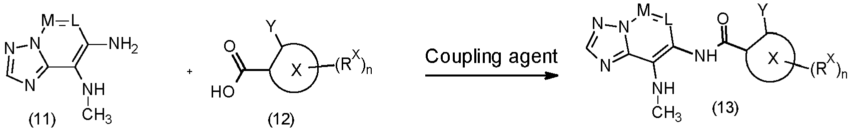 Typical Coupling Agents are hexafluorophosphate azabenzotriazole tetramethyl uronium (HA-TU), 3-[Bis(dimethylamino)methyliumyl]-3H-benzotriazol-1-oxide hexafluorophosphate (HBTU), or *O*-(1*H*-6-Chlorobenzotriazole-1-yl)-1,1,3,3-tetramethyluronium hexafluorophosphate (HCTU). The reaction may be carried out in a polar aprotic solvent, such as DMF, in the presence of a base. Compounds of formula (12) are commercially available or may be prepared by methods as inter alia described in WO2016/071214A1, EP Application Number 2115353132.2, and EP Application Number 20168197.0.

Compounds of formula (13) are then treated with an Acid Catalyst, such as CH₃COOH, or toluene sulfonic acid, to produce compounds of formula (14), which fall under the definition of compounds of formula (I), in a condensation reaction: 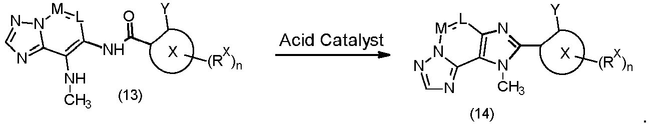 The reaction is typically carried out at elevated temperatures of from 60 to 180 °C in a polar solvent. Typically, the Acid Catalyst also serves as the solvent for the reaction.

Other suitable building blocks for the preparation of compounds of formula (I) are compounds of formula (15), which are accessible as depicted under General Scheme 2 below:

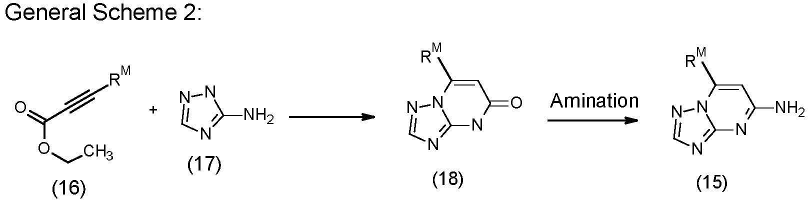

In a first step, compounds of formula (15) are reacted with compounds of formula (17) to yield compounds of formula (18). Such reactions have been described in Journal of Chemistry (2019), 43(25), 9961-9968. The reaction is typically carried out in a two-step approach. In a first step, compounds of formula (16) and compounds of formula (17) are reacted in an inert polar solvent at a temperature of from 60 to 150 °C. Typical solvents include halogenated hydrocarbons, preferably halogenated aliphatic C₁-C₆-alkanes, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, preferably C₁-C₆-cycloalkyl ethers, C₁-C₆-alkyl-C₁-C₆-alkyl ethers and C₁-C₆-alkyl-C₆-C₁₀-aryl ethers, such as CH₃CH₂OCH₂CH₃, (CH₃)₂CHOCH(CH₃)₂, MTBE, DME, CH₃OCH₂CH₂OCH₃, dioxane, anisole, and THF, and mixtures thereof. Typically, the reaction is carried out in a microwave reactor.
In a second step, a base is added to the reaction mixture at 20 to 30 °C to yield compounds of formula (18). Suitable bases include inorganic bases, such as alkali metal and alkaline earth metal hydroxides, such as LiOH, NaOH, KOH, and Ca(OH)₂; alcoholates, such as alkali methanolate, alkali ethanolate, alkali isopropanolate, alkali tert-butanolate, and mixtures thereof.

Subsequently, compounds of formula (18) may be reacted with an aminating agent to yield compounds of formula (15). Reactions of this kind have are routine for the skilled person and may for example be carried out as described in Org. Lett. 2020, 22, 16, 6547-6551; Org. Lett. 2006, 8, 11, 2425-2428 and US6046211A, Example 310.

Compounds of formula (15) may then be reacted with compounds of formula (19) to yield compounds of formula (20) falling under the definition of compounds of formula (I)

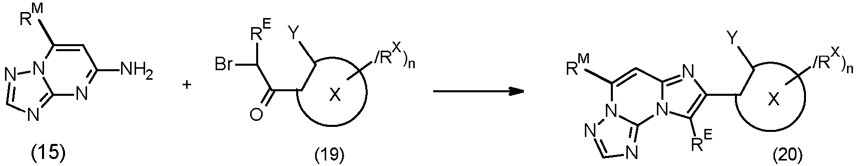

Reactions of this type have been described in EP3257853A1 and WO2018206479. The reaction is typically carried out under elevated temperatures of from 50-160 °C in an inert solvent. Suitable solvents are aliphatic hydrocarbons, such as pentane, hexane, cyclohexane, or petrol ether; aromatic hydrocarbons, such as benzene, toluene, o-, m-, and p-xylene; halogenated hydrocarbons, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, such as CH₃CH₂OCH₂CH₃, (CH₃)₂CHOCH(CH₃)₂, MTBE, DME, CH₃OCH₂CH₂OCH₃, CH₃OC(CH₃)₂CH₂CH₃, dioxane, anisole, 2-methyltetrahydrofuran, THF, and diethylene glycol; nitriles, such as CH₃CN, and CH₃CH₂CN; alcohols, such as CH₃OH, CH₃CH₂OH, CH₃CH₂CH₂OH, CH₃CH(OH)CH₃, CH₃(CH₂)₃OH, and C(CH₃)₃OH, CH₂(OH)CH₂(OH), CH₃CH(OH)CH₂OH; amides and urea derivatives, such as DMF, NMP, DMA, DMI, DMPU, HMPA; moreover DMSO, sulfolane, and H₂O. Mixtures of the above solvents are also possible.

The reaction may be carried out in the presence of a catalyst, such as an acid or a base, preferably a base. Suitable bases are, in general, inorganic bases, such as LiOH, NaOH, KOH, and Ca(OH)₂; alkali metal and alkaline earth metal oxides, such as Li₂O, Na₂O, CaO, and MgO; alkali metal and alkaline earth metal hydrides, such as LiH, NaH, KH and CaH₂; alkali metal and alkaline earth metal carbonates, such as Li₂CO₃, K₂CO₃ and CaCO₃; alkali metal bicarbonates, such as NaHCO₃; organic bases, such as pyrrolidine; tertiary amines, such as diisopropylethylamine, trimethylamine, triethylamine, triisopropylamine and N-methylpiperidine, imidazol, pyridine; substituted pyridines, such as collidine, lutidine and 4-dimethylaminopyridine, and polycyclic amides and amidines, such as 1,8-diazabicycloundec-7-ene (DBU), 1,4-Diazabicyclo[2.2.2]octane (DABCO); alkali metal salts of secondary amines, such as alkali diisopropylamide, alkali bis(trimethylsilyl)amide, alkali tetramethylpiperidene; alcoholates, such as alkali methanolate, alkali ethanolate, alkali isopropanolate, alkali tert-butanolate; alkali metal - alkyl, and alkali metal - aryl salts, such as n-butyl lithium, tert-butyl lithium, phenyl lithium. Mixtures of the aforementioned bases are also possible. The bases are generally employed in catalytic amounts; however, they can also be used in equimolar amounts, in excess or, if appropriate, as solvent.

Compounds of formula (15) and compounds of formula (19) are typically reacted with one another in equimolar amounts. In terms of yield, it may be advantageous to employ an excess of compounds of formula (19).

Compounds of formula (19) are accessible by a variety of synthetic methods, such as described in EP Application Number 2115353132.2, EP Application Number 20168197.0, WO2016/071214A1, and WO 2018/206479A1.

By other way of example, compounds of formula (22), falling under the definition of compounds of formula (I), may be obtained as depicted in General Scheme 3 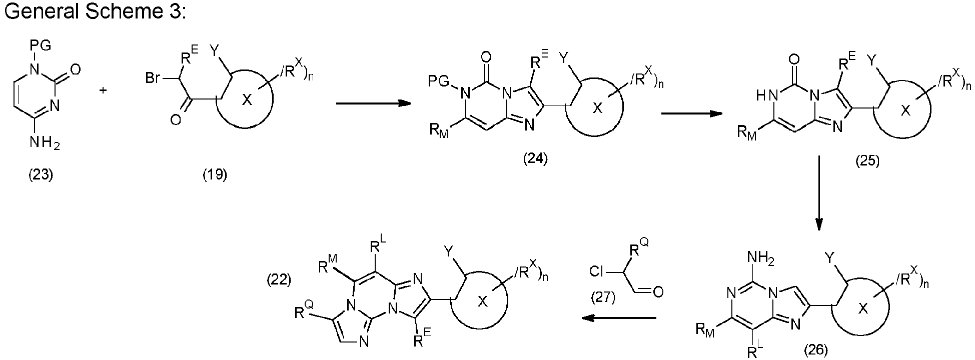 PG means protective group, e.g. benzyl.

In a first step, compounds of formula (23) are reacted with compounds (19) to obtain compounds (24). Reactions of this type have been described in EP3257853A1 and WO2018206479. The reaction is typically carried out under elevated temperatures of from 50-160 °C in an inert solvent. Suitable solvents are aliphatic hydrocarbons, such as pentane, hexane, cyclohexane, or petrol ether; aromatic hydrocarbons, such as benzene, toluene, o-, m-, and p-xylene; halogenated hydrocarbons, or halogenated aromatic C₆-C₁₀-hydrocarbons, such as CH₂Cl₂, CHCl₃, CCl₄, CH₂ClCH₂Cl, CCl₃CH₃, CHCl₂CH₂Cl, CCl₂CCl₂, or chlorobenzene; ethers, such as CH₃CH₂OCH₂CH₃, (CH₃)₂CHOCH(CH₃)₂, MTBE, DME, CH₃OCH₂CH₂OCH₃, CH₃OC(CH₃)₂CH₂CH₃, dioxane, anisole, 2-methyltetrahydrofuran, THF, and diethylene glycol; nitriles, such as CH₃CN, and CH₃CH₂CN; alcohols, such as CH₃OH, CH₃CH₂OH, CH₃CH₂CH₂OH, CH₃CH(OH)CH₃, CH₃(CH₂)₃OH, and C(CH₃)₃OH, CH₂(OH)CH₂(OH), CH₃CH(OH)CH₂OH; amides and urea derivatives, such as DMF, NMP, DMA, DMI, DMPU, HMPA; moreover DMSO, sulfolane, and H₂O. Mixtures of the above solvents are also possible.

The reaction may be carried out in the presence of a catalyst, such as an acid or a base, preferably a base. Suitable bases are, in general, inorganic bases, such as LiOH, NaOH, KOH, and Ca(OH)₂; alkali metal and alkaline earth metal oxides, such as Li₂O, Na₂O, CaO, and MgO; alkali metal and alkaline earth metal hydrides, such as LiH, NaH, KH and CaH₂; alkali metal and alkaline earth metal carbonates, such as Li₂CO₃, K₂CO₃ and CaCO₃; alkali metal bicarbonates, such as NaHCO₃; organic bases, such as pyrrolidine; tertiary amines, such as diisopropylethylamine, trimethylamine, triethylamine, triisopropylamine and N-me19etramethridine, imidazol, pyridine; substituted pyridines, such as collidine, lutidine and 4-dimethylaminopyridine, and polycyclic amides and amidines, such as 1,8-diazabicycloundec-7-ene (DBU), 1,4-Diazabicyclo[2.2.2]octane (DABCO); alkali metal salts of secondary amines, such as alkali diisopropylamide, alkali bis(trimethylsilyetramethylpiperidinemethylpiperidene; alcoholates, such as alkali methanolate, alkali ethanolate, alkali isopropanolate, alkali tert-butanolate; alkali metal - alkyl, and alkali metal - aryl salts, such as n-butyl lithium, tert-butyl lithium, phenyl lithium. Mixtures of the aforementioned bases are also possible. The bases are generally employed in catalytic amounts; however, they can also be used in equimolar amounts, in excess or, if appropriate, as solvent.

Compounds (23) can be prepared as described in WO2017/167832A1, e.g. Example C-1. Compounds (23) and compounds (19) are typically reacted with one another in equimolar amounts. In terms of yield, it may be advantageous to employ an excess of compounds (2).

In a second step, protective group "PG" is removed. This may be achieved by standard methods as described in "Greene's Protective Groups in Organic Synthesis", Wiley, 2006.

In a third step, the carbonyl group in compounds (25) is converted into an enamine goup in compounds (26). Typically, compounds (25) are first reacted with a suitable activating agent, such as triflateanhydride, followed by reaction with NH₃. The activation reaction is typically carried out in a suitable inert, aprotic solvent, such as a hydrocarbon, or a halogenated hydrocarbon, at a temperature of from -10 to 25 °C in the presence of a base, such as organic bases, especially pyridine. The subsequent reaction with NH₃ is then typically carried out in an organic polar solvent, such as halogenated hydrocarbons and alcohols, at a temperature of from 10 to 50 °C.

Finally, compounds (26) are reacted with compounds (27) to obtain compounds (22). The reaction is typically carried out in a polar solvent in the presence of a base. Suitable solvents include H₂O, ethers, alcohols, nitriles, amides and urea derivatives, DMSO, and sulfolane. Suitable bases are inorganic bases, preferably alkali metal and alkaline earth metal carbonates, such as Li₂CO₃, K₂CO₃ and CaCO₃. Typically, compounds (27) are applied in excess compared to compounds (26). They may, however, also be applied in equimolar amounts.

The reaction mixtures are worked up in a customary manner, for example by mixing with water, separating the phases and, if appropriate, chromatographic purification of the crude products. Some of the intermediates and end products are obtained in the form of colorless or slightly brownish viscous oils which are purified or freed from volatile components under reduced pressure and at moderately elevated temperature. If the intermediates and end products are obtained as solids, purification can also be carried out by recrystallization or digestion.

The N-oxides may be prepared from the inventive compounds according to conventional oxidation methods, e. g. by treating compounds of formula (I) with an organic peracid such as metachloroperbenzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus to be controlled.

A skilled person will readily understand that the preferences for the substituents, also in particular the ones given in the tables below for the respective substituents, given herein in connection with compounds of formula (I) apply for the intermediates accordingly. Thereby, the substituents in each case have independently of each other or more preferably in combination the meanings as defined herein.

### Preferences

Embodiments and preferred compounds of the present invention for use in pesticidal methods and for insecticidal application purposes are outlined in the following paragraphs. The remarks made below concerning preferred embodiments of the variables of compounds of formula (I) are valid both on their own in combination with each other. The variables of the compounds of formula (I) have the following meanings, these meanings, both on their own and in combination with one another, being particular embodiments of the compounds of the formula (I).

The variable A is CH, N, or NH. In one embodiment, A is N. In another embodiment, A is NH. The variable E is N, NH, O, S, or CR^{E}. In one embodiment, E is NR^{E} or CR^{E}. In another embodiment, A is N or NH, and E is NR^{E} or CR^{E}. In another embodiment, A is N and E is NR^{E}. In another embodiment, A is N and E is CR^{E}

The variables G and J are independently C or N, provided that only one of E or G is N. Typically, both G and J are C. In one embodiment, G is N and J is C.

The variable L is N or CR^{L}. In one embodiment, the variable L is N. In another embodiment, the variable L is CR^{L}, preferably wherein R^{L} is H, C₁-C₃-alkyl, C₁-C₃-haloalkyl, C₁-C₃-alkoxy, or C₁-C₃-haloalkoxy, more preferably wherein R^{L} is H, C₁-C₃-fluoroalkyl, or C₁-C₃-fluoroalkoxy, most preferably wherein R^{L} is H, CF₃ or OCF₃, especially preferably wherein R^{L} is H.

The variable M is N or CR^{M}. In one embodiment, the variable M is N. In another embodiment, the variable M is CR^{M}, preferably wherein R^{M} is H, C₁-C₃-alkyl, C₁-C₃-haloalkyl, C₁-C₃-alkoxy, or C₁-C₃-haloalkoxy, more preferably wherein R^{M} is H, C₁-C₃-fluoroalkyl, or C₁-C₃-fluoroalkoxy, most preferably wherein R^{M} is H, or OCF₃, especially preferably wherein R^{M} is H or CF₃.

The variable Q is N or CR^{Q}. In one embodiment, the variable Q is N. In another embodiment, the variable Q is CR^{Q}, preferably wherein R^{Q} is H, C₁-C₃-alkyl, C₁-C₃-haloalkyl, C₁-C₃-alkoxy,or C₁-C₃-haloalkoxy, more preferably wherein R^{Q} is H, C₁-C₃-fluoroalkyl, or C₁-C₃-fluoroalkoxy, most preferably wherein R^{Q} is H, CF₃, OCHF₂, or OCF₃, especially preferably wherein R^{Q} is H, CF₃, or OCF₃, such as CF₃.

The variable T is N or CR^{T}. In one embodiment, the variable Q is N. In another embodiment, the variable T is CR^{T}, preferably wherein R^{T} is H, C₁-C₃-alkyl, C₁-C₃-haloalkyl, C₁-C₃-alkoxy, or C₁-C₃-haloalkoxy, more preferably wherein R^{T} is H, C₁-C₃-fluoroalkyl, or C₁-C₃-fluoroalkoxy, most preferably wherein R^{T} is H, or CF₃, especially H.

Typically, only one of the variables Q and T is N. In one embodiment, both Q and T are C. In another embodiment, Q is N and T is CR^{T}.

Prefered combinations of variables A, E, G, J, L, M, Q, and T are presented below as *formulae* (I-A) to (I-M), wherein the variables have a meaning as defiend for formula (I).

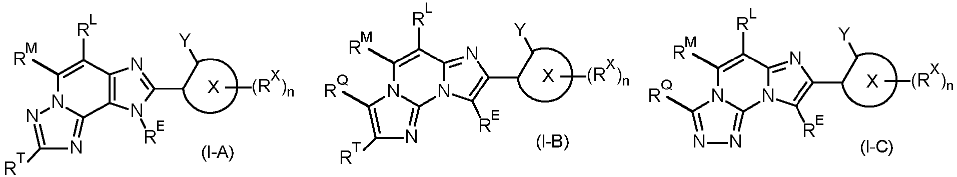

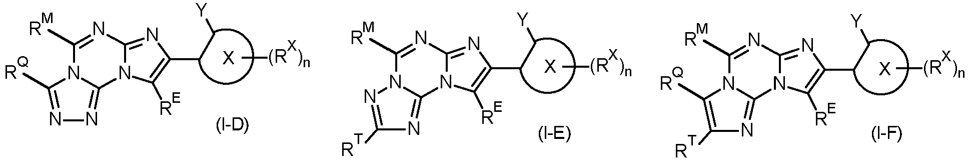

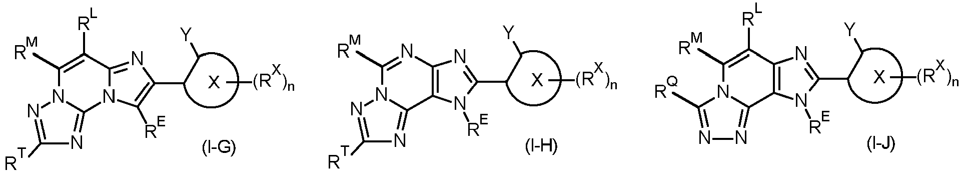

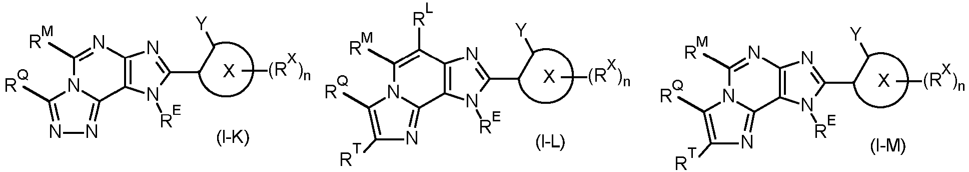

In one embodiment, compounds of formula (I) are compounds of formula (I-A). In another embodiment, compounds of formula (I) are compounds of formula (I-B). In another embodiment, compounds of formula (I) are compounds of formula (I-A) or (I-B). In another embodiment, compounds of formula (I) are compounds of formula (I-G). In another embodiment, compounds of formula (I) are compounds of formula (I-A), (I-B), or (I-G).

The variable Y is SR^{Y1}, S(O)R^{Y1}, S(O)₂R^{Y1}, S(=O)(=NR^{Y2})R^{Y1}, or S(=NR^{Y2})(=NR^{Y3})R^{Y1}. In one embodiment, the variable Y is S(O)₂R^{Y1}. In another embodiment, the variable Y is S(=O)(=NR^{Y2})R^{Y1}. In another embodiment, the variable Y is S(=NR^{Y2})(=NR^{Y3})R^{Y1}.

Each R^{Y1}, R^{Y2}, R^{Y3} is independently selected from H, C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more, same or different substituents selected from halogen and CN;
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, CN, C₁-C₃-alkyl, C₁-C₃-alkoxy, C₁-C₃-haloalkyl, C₁-C₃-haloalkoxy, and wherein said N- and S-atoms are independently oxidized, or non-oxidized;
phenyl, which is unsubstituted, or substituted with one or more, same or different substituents selected from halogen, CN, C₁-C₃-alkyl, C₁-C₃-alkoxy, C₁-C₃-haloalkyl, C₁-C₃-haloalkoxy;
or two substituents selected from R^{Y1}, R^{Y2}, R^{Y3} form, together with the S- or N-atom to which they are bound, a 5- or 6- membered saturated, partially unsaturated, or fully unsaturated heterocycle, which heterocycle is unsubstituted, or substituted with one or more, same or different substituents selected from halogen, CN, C₁-C₃-alkyl, C₁-C₃-alkoxy, C₁-C₃-haloalkyl, C₁-C₃-haloalkox, and wherein said heterocycle comprises no, one or more, same or different heteroatoms O, N, or S in addition to the S- or N-atoms to the substituents selected from R^{Y1}, R^{Y2,}, and R^{Y3} are bound to.

In another embodiment, each R^{Y1} R^{Y2}, R^{Y3} is independently selected from H, C₁-C₃-alkyl, which is unsubstituted, or substituted with one or more, same or different substituents selected from halogen; phenyl, which is unsubstituted, or substituted with one or more, same or different substituents selected from halogen, CN, C₁-C₃-alkyl, and C₁-C₃-haloalkyl; or two substituents selected from R^{Y1}, R^{Y2}, R^{Y3} form, together with the heteroatoms atom to which they are bound, a 5- or 6- membered partially unsaturated, or fully unsaturated heterocycle, which heterocycle is unsubstituted, or substituted with halogen, and wherein said heterocycle comprises no, one or more, same or different heteroatoms O, N, or S in addition to the heteroatoms to which the substituents selected from R^{Y1}, R^{Y2}, and R^{Y3} are bound to.

Typically, each R^{Y1}, R^{Y2}, R^{Y3} is selected from H, C₁-C₃-alkyl and C₁-C₃-haloalkyl. Preferably, R^{Y1} is selected from C₁-C₃-alkyl and C₁-C₃-haloalkyl, and R^{Y2} is selected from H, C₁-C₃-alkyl, and C₁-C₃-haloalkyl.

Accordingly, Y is preferably SO₂R^{Y1}, or S(=O)(=NR^{Y2})R^{Y1}, wherein R^{Y1} is C₁-C₃-alkyl or C₁-C₃-haloalkyl, and R^{Y2} is H, C₁-C₃-alkyl, or C₁-C₃-haloalkyl. More preferably, Y is SO₂R^{Y1}

The index n is 0, 1, 2, 3, or 4, if X is phenyl, or a 6-membered heteroaryl, or 0, 1, 2, or 3 if X is a 5-membered heteroaryl. Typically, n is 1. In one embodiment, n is 0. In another embodiment, n is 2. In another embodiment, n is 3.

R^{E}, R^{L}, R^{M}, R^{Q}, and R^{T} are independently H, halogen, N₃, CN, NO₂, SCN, SF₅; C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are halogenated or non-halogenated; C(=O)OR¹, NR²R³, C₁-C₆-alkylen-NR²R³, O-C₁-C₆-alkylen-NR²R³, C₁-C₆-alkylen-CN, NH-C₁-C₆-alkylen-NR²R³, C(=O)NR²R³, C(=O)R⁴, SO₂NR²R³, S(=O)ₘR⁵, OR⁶, C(=O)R⁶, SR⁶, or CH₂R⁶; or phenyl, which is unsubstituted or substituted with one or more, same or different substituents R¹¹.

R^{E} is typically H, halogen; C₁-C₃-alkyl, C₁-C₃-alkoxy, C₂-C₃-alkenyl, C₂-C₃-alkynyl, C₃-C₅-cycloalkyl, which groups are halogenated or non-halogenated. In one embodiment, R^{E} is H, C₁-C₁-C₃-alkyl, or C₁-C₃-haloalkyl. In another embodiment, R^{E} is H or CH₃. In another embodiment, R^{E} is CH₃.

R^{L} is typically H, halogen; C₁-C₃-alkyl, C₁-C₃-alkoxy, C₂-C₃-alkenyl, C₂-C₃-alkynyl, C₃-C₅-cycloalkyl, which groups are halogenated or non-halogenated. In one embodiment, R^{L} is H, C₁-C₁-C₃-alkyl, C₁-C₃-haloalkyl, C₁-C₃-alkoxy, or C₁-C₃-haloalkoxy. In another embodiment, R^{L} is H or CF₃. In another embodiment, R^{L} is H.

R^{M} is typically H, halogen; C₁-C₃-alkyl, C₁-C₃-alkoxy, C₂-C₃-alkenyl, C₂-C₃-alkynyl, C₃-C₅-cycloalkyl, which groups are halogenated or non-halogenated. In one embodiment, R^{M} is H, C₁-C₁-C₃-alkyl, C₁-C₃-haloalkyl, C₁-C₃-alkoxy, or C₁-C₃-haloalkoxy. In another embodiment, R^{M} is H or CF₃.

R^{Q} is typically H, halogen; C₁-C₃-alkyl, C₁-C₃-alkoxy, C₂-C₃-alkenyl, C₂-C₃-alkynyl, C₃-C₅-cycloalkyl, which groups are halogenated or non-halogenated. In one embodiment, R^{Q} is H, C₁-C₁-C₃-alkyl, C₁-C₃-haloalkyl, C₁-C₃-alkoxy, or C₁-C₃-haloalkoxy. In another embodiment, R^{Q} is H, CF₃, or OCF₃. In another embodiment, R^{Q} is H, CF₃ or OCF₃. In another embodiment, R^{Q} is H. In another embodiment, R^{Q} is CF₃.

R^{T} is typically H, halogen; C₁-C₃-alkyl, C₁-C₃-alkoxy, C₂-C₃-alkenyl, C₂-C₃-alkynyl, C₃-C₅-cycloalkyl, which groups are halogenated or non-halogenated. In one embodiment, R^{T} is H, C₁-C₁-C₃-alkyl, C₁-C₃-haloalkyl, C₁-C₃-alkoxy, or C₁-C₃-haloalkoxy. In another embodiment, R^{T} is H, CF₃, or OCF₃. In another embodiment, R^{T} is H, C₁-C₃-haloalkyl, or C₁-C₃-haloalkoxy. In antoher embodiment, R^{T} is H.

In one embodiment, R^{M}, R^{Q}, and R^{T} are independently H; or C₁-C₆-alkyl, C₁-C₆-alkoxy, C₂-C₆-alkenyl, C₂-C₆-alkynyl, C₃-C₆-cycloalkyl, C₃-C₆-cycloalkoxy, or C₁-C₆-alkyl-S(O)ₘ, which groups are halogenated or non-halogenated.

In another embodiment, R^{M}, R^{Q}, and R^{T}are independently H; or C₁-C₃-alkyl, C₁-C₃-alkoxy, C₂-C₃-alkenyl, C₂-C₃-alkynyl, C₃-C₆-cycloalkyl, or C₃-C₆-cycloalkoxy, which groups are halogenated or non-halogenated. In another embodiment, R^{M}, R^{Q}, and R^{T} are independently H; or C₁-C₃-alkyl, or C₁-C₃-alkoxy, which groups are halogenated or non-halogenated.

In another embodiment, R^{M}, R^{Q}, and R^{T}are independently H; or C₁-C₃-haloalkyl, or C₁-C₃-haloalkoxy. In another embodiment, R^{M}, R^{Q}, and R^{T}are independently H; or C₁-C₃-fluoroalkyl, or C₁-C₃-fluoroalkoxy, wherein at least one substituent R^{M}, R^{Q}, R^{T}, and R^{V} is not H.

In one embodiment, R^{L}, R^{M}, R^{Q}, and R^{T}are independently H; or C₁-C₆-alkyl, C₁-C₆-alkoxy, C₂-C₆-alkenyl, C₂-C₆-alkynyl, C₃-C₆-cycloalkyl, C₃-C₆-cycloalkoxy, or C₁-C₆-alkyl-S(O)ₘ, which groups are halogenated or non-halogenated.

In another embodiment, R^{L}, R^{M}, R^{Q}, and R^{T} are independently H; or C₁-C₃-alkyl, C₁-C₃-alkoxy, C₂-C₃-alkenyl, C₂-C₃-alkynyl, C₃-C₆-cycloalkyl, or C₃-C₆-cycloalkoxy, which groups are halogenated or non-halogenated. In another embodiment, R^{L}, R^{M}, R^{Q}, and R^{T} are independently H; or C₁-C₃-alkyl, or C₁-C₃-alkoxy, which groups are halogenated or non-halogenated.

In another embodiment, R^{L}, R^{M}, R^{Q}, and R^{T} are independently H; or C₁-C₃-haloalkyl, or C₁-C₃-haloalkoxy. In another embodiment, R^{L}, R^{M}, R^{Q}, and R^{T} are independently H; or C₁-C₃-fluoroalkyl, or C₁-C₃-fluoroalkoxy, wherein at least one substituent R^{L}, R^{M}, R^{Q}, R^{T}, and R^{V} is not H.

In one embodiment, R^{E} and R^{L} are independently H, halogen; C₁-C₄-alkyl, C₁-C₄-alkoxy, C₂-C₄-alkenyl, or C₂-C₄-alkynyl, which groups are halogenated or non-halogenated. In another embodiment, R^{E} and R^{L} are independently H, C₁-C₃-alkyl, or C₁-C₃-haloalkyl. In another embodiment, R^{E} and R^{L} are independently H, or C₁-C₃-alkyl. In another embodiment, R^{L} is H and R^{E} is H or C₁-C₃-alkyl.

The cycle X is phenyl, or a 5- or 6-membered heteroaryl, preferably phenyl or 2-pyridyl. For the avoidance of doubt, the cycle X is substituted with n substituents R^{X}. Also, for the avoidance of doubt, X is connected to Y and to the tricyclic system by direct chemical bonds to two adjacent ring members of X.

In one embodiment, X is phenyl. In another embodiment, X is a 5-membered heteroaryl. In another embodiment, X is a 6-membered hetary. In another embodiment, X is a 5-membered heteroaryl comprising one N-atom. In another embodiment, X is a 6-membered heteroaryl comprising at least one N-atom. In another embodiment, X is a 6-membered heteroaryl comprising two N-atoms.

Prefered 5- or 6-membered heteroaryls X are depicted below as *formulae* A-1 to A-48, wherein "&" stands for the connection to the trycyclic scaffold of compounds of formula (I). For the avoidance of doubt, the *formulae* A-1 to A-48 are preferred embodiments on their own and in combination for the following moiety of formula (I) 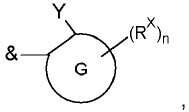 wherein "&" stands for the connection to the tricyclic scaffold in formula (I). In other words, the substituents Y and (R^{X})ₙ in formulae A1 to A48 are mere illustrations but are not part of the heteroaryl G.

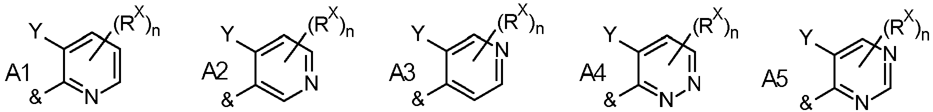

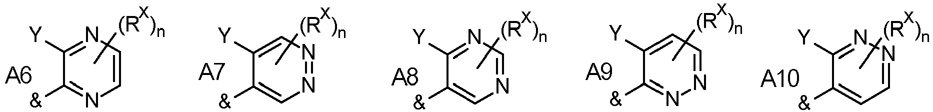

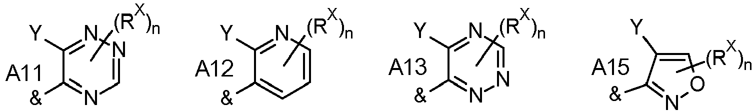

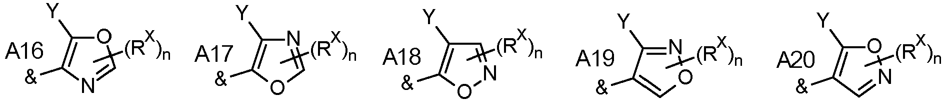

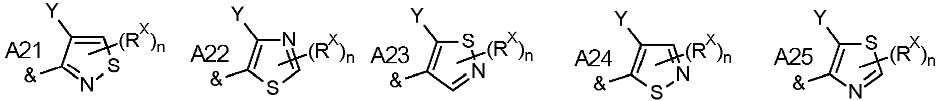

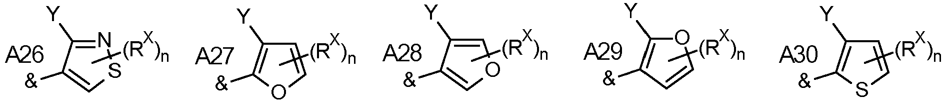

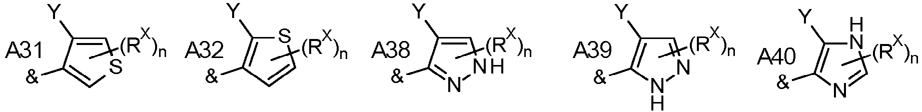

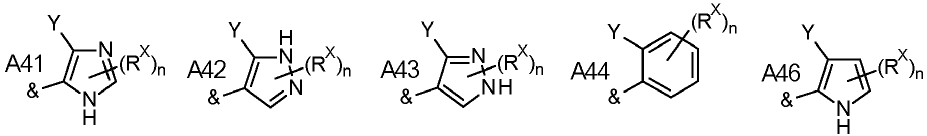

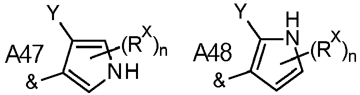

In one embodiment, X is selected from *formulae* A-1 to A-14. In one embodiment, X is selected from *formulae* A-1 to A-3. In another embodiment, X is A-1. In another embodiment, X is A-2. In another embodiment, X is A-3.

R¹ is H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which groups are halogenated or non-halogenated; C₁-C₆-alkylen-NR²R³, C₁-C₆-alkylen-CN, or CH₂R⁶; or phenyl, which is unsubstituted, or substituted with one or more, same or different substituents R¹¹.

In one embodiment, R¹ is phenyl, which is unsubstituted or substituted with one or more, same or different substituents R¹¹. In another embodiment, R¹ is H; C₁-C₆-alkyl, C₂-C₆-alkenyl, C₂-C₆-alkynyl, C₁-C₆-alkoxy-C₁-C₄-alkyl, C₃-C₆-Cycloalkyl, C₃-Calkyl-C₁-C₄-alkyl, or C₃-C₆-cycloalkoxy-C₁-C₄-alkyl, which groups are halogenated or non-halogenated. In another embodiment, R¹ is H; C₁-C₃-alkyl, C₂-C₃-alkenyl, C₂-C₃-alkynyl, which groups are halogenated or non-halogenated. In another embodiment, R¹ is C₁-C₃-alkyl or C₁-C₃-haloalkyl.

R¹¹ is halogen, N₃, OH, CN, NO₂, SCN, SF₅; C₁-C₆-alkyl, C₁-C₆-alkoxy, C₂-C₆-alkenyl , C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are halogenated or non-halogenated.

In one embodiment, R¹¹ is halogen, OH, CN, SF₅; C₁-C₃-alkyl, C₁-C₃-alkoxy, which groups are halogenated or non-halogenated. In one embodiment, R¹¹ is halogen; C₁-C₃-alkyl, or C₁-C₃-haloalkyl.

R² is H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are halogenated or non-halogenated; C(=O)R²¹, C(=O)OR²¹, C(=O)NR²¹, C₁-C₆-alkylen-CN, or CH₂R⁶; or phenyl, which is unsubstituted or substituted with one or more, same or different substituents R¹¹.

In one embodiment, R² is H; C₁-C₃-alkyl, C₂-C₃-alkenyl, C₂-C₃-alkynyl, which groups are halogenated or non-halogenated. In another embodiment, R² is H. In another embodiment, R² is H; C₁-C₃-alkyl, or C₁-C₃-haloalkyl.

R²¹ is H; C₁-C₆-alkyl, C₁-C₆-haloalkyl, C₂-C₆-alkenyl, C₂-C₆-alkynyl, C₁-C₆-alkoxy-C₁-C₄-alkyl, C₃-C₆-cycloalkyl; C₃-C₆-cycloalkyl-C₁-C₄-alkyl, C₃-C₆-cycloalkoxy-C₁-C₄ alkyl, phenyl, or a saturated, partially-, or fully unsaturated 5- or 6-membered heterocycle, wherein the cyclic moieties are unsubstituted or substituted with one or more, same or different substituents R¹¹. In one embodiment, R²¹ is H; C₁-C₃-alkyl, C₁-C₃-haloalkyl, or phenyl. In another embodiment, R²¹ is C₁-C₃-alkyl.

R³ is H; C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are halogenated or non-halogenated; C₁-C₆-alkylen-CN, or CH₂R^{a}; phenyl, which is unsubstituted or substituted with one or more, same or different substituents R¹¹; or NR²R³ may also form an N-bound, saturated 3- to 8-membered heterocycle, which in addition to the nitrogen atom may have 1 or 2 further heteroatoms or heteroatom moieties selected from O, S(=O)ₘ, NH, and N-C₁-C₆-alkyl, and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy. In one embodiment, R³ is H, C₁-C₃-alkyl, C₁-C₃-haloalkyl, or phenyl. In another embodiment, R³ is phenyl. In another embodiment, R³ is H. In another embodiment, R² is H and R³ is C₁-C₃-alkyl or phenyl.

R⁴ is selected from H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which are unsubstituted or substituted with halogen; CH₂R⁶, or phenyl, which is unsubstituted, or substituted with one or more, same or different substituents R¹¹. In one embodiment, R⁴ is H, C₁-C₃-alkyl, C₁-C₃-haloalkyl, or phenyl. In another embodiment, R⁴ is H. In another embodiment, R⁴ is C₁-C₃-alkyl, or C₁-C₃-haloalkyl. In another embodiment, R⁴ is C₃-C₆-cycloalkyl, which is unsubstituted or substituted with CN, preferably 1-cyanocyclopropyl.

R⁵ C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which groups are halogenated or non-halogenated; C₁-C₆-alkylen-NR²R³, C₁-C₆-alkylen-CN, CH₂R⁶; or phenyl, which is unsubstituted, or substituted with one or more, same or different substituents R¹¹. In one embodiment, R⁵ is C₁-C₆-alkyl, C₂-C₆-alkenyl, C₂-C₆-alkynyl, or phenyl, which groups are unhalogenated or halogenated. In another embodiment, R5 is C₁-C₃-alkyl or C₁-C₃-haloalkyl.

R⁶ is phenyl, which is unsubstituted or substituted with one or more, same or different substituents R¹¹. In one embodiment, R⁶ is phenyl. In another embodiment, R⁶ is phenyl that is unsub-situted or substituted with halogen, C₁-C₃-alkyl, C₁-C₃-haloalkyl, C₁-C₃-alkoxy, or C₁-C₃-haloalkoxy.

Each R^{X} is independently halogen, N₃, OH, CN, NO₂, SCN, SF₅;
C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are unsubstituted or substituted with CN or halogen;
C(=O)OR¹, NR²R³, C(=O)NR²R³, C(=O)R⁴, SO₂NR²R³, S(=O)ₘR¹, OR⁶, SR⁶, CH₂R⁶, OC(=O)R⁴, NR³C(=O)R⁴, OC(=O)OR¹, OC(=O)NR²R³, OC(=O)SR¹, OC(=S)NR²R³, OC(=S)SR¹, ONR²R³, ON=CR¹R⁴, N=CR¹R⁴, NNR², NR³C(=O)R⁷, SC(=O)SR¹, SC(=O)NR²R³, C(=S)R⁶, C(=S)OR⁴, C(=NR²)R⁴, C(R⁸)=N-O(R⁹);
phenyl, which is unsubstituted or substituted with one or more, same or different substituents R¹¹;
a 5- or 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents R³¹, and wherein said N- and S-atoms are independently oxidized, or non-oxidized; or a group of formula (S) 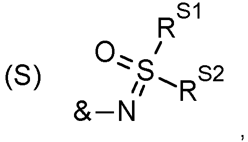
wherein each R^{S1}, R^{S2} is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or halogenated;
a 3- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said N- and S-atoms are independently oxidized, or non-oxidized; phenyl, which is unsubstituted, or substituted with one or more, same or different substituents selected from halogen, C₁-C₃-alkyl, C₁-C₃-alkoxy, C₁-C₃-haloalkyl, and C₁-C₃-haloalkoxy;
or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to.

Typically, each R^{X} is independently halogen, CN;
C₁-C₃-alkyl, C₁-C₃-alkoxy, C₃-C₆-cycloalkyl, C₃-C₆-cycloalkoxy, C₃-C₆-cycloalkyl-C₁-C₄-alkyl, which groups are unsubstituted or substituted with CN or halogen;
NR³C(=O)R⁷, C(R⁸)=N-O(R⁹);
phenyl, which is unsubstituted or substituted with one or more, same or different substituents R¹¹;
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents R³¹, and
wherein said N- and S-atoms are independently oxidized, or non-oxidized; or
a group of formula (S),
wherein each R^{S1}, R^{S2} is independently selected from C₁-C₆-alkyl, C₃-C₆-cycloalkyl, which groups are unsubstituted, or halogenated;
or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to.

In another embodiment, each R^{X} is independently halogen;
C₁-C₃-alkyl, C₁-C₃-alkoxy, which groups are unsubstituted or substituted with CN or halogen; NR³C(=O)R⁷, C(R⁸)=N-O(R⁹);
phenyl, which is unsubstituted or substituted with one or more, same or different substituents selected from halogen, C₁-C₃-alkyl, C₁-C₃-alkoxy, C₁-C₃-haloalkyl, and C₁-C₃-haloalkoxy;
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents selected from halogen, CN, C₁-C₃-alkyl, C₁-C₃-alkoxy, C₁-C₃-haloalkyl, and C₁-C₃-haloalkoxy, and wherein two substituents may form together with the carbon-atom to which they are bound a group (C=O), and wherein said N- and S-atoms are independently oxidized, or non-oxidized; or a group of formula (S),
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to.

In another embodiment, each R^{X} is independently halogen;
C₁-C₃-alkyl, C₁-C₃-alkoxy, which groups are unsubstituted or substituted with CN or halogen (such as 1-cyanoisopropyl);
NR³C(=O)R⁷, C(R⁸)=N-O(R⁹);
phenyl, which is unsubstituted or halogenated;
a 5- or 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents selected from halogen, CN, C₁-C₃-alkyl, C₁-C₃-alkoxy, C₁-C₃-haloalkyl, and C₁-C₃-haloalkoxy, and wherein two substituents may form together with the carbon-atom to which they are bound a group (C=O), and wherein said N- and S-atoms are independently oxidized, or non-oxidized; or a group of formula (S),
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 6-membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to.

In another embodiment, each R^{X} is independently halogen;
C₁-C₃-alkyl, which groups are unsubstituted or substituted with halogen;
phenyl, which is unsubstituted or halogenated.

In another embodiment, each R^{X} is independently halogen;
C₁-C₃-alkyl, C₃-C₆-cycloalkyl, or C₁-C₃-alkoxy, which groups are unsubstituted or substituted with CN;
NR³C(=O)R⁷;
phenyl, which is unsubstituted or halogenated;
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein two substituents may form together with the carbon-atom to which they are bound a group (C=O), and wherein said N- and S-atoms are independently oxidized, or non-oxidized; or a group of formula (S),
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to.

In another embodiment, each R^{X} is independently halogen;
C₁-C₃-alkyl, C₃-C₆-cycloalkyl, or C₁-C₃-alkoxy, which groups are unsubstituted or substituted with CN;
phenyl, which is unsubstituted or halogenated;
a 6-membered saturated, partially unsaturated, or fully unsaturated heterocyclic ring, wherein said heterocyclic ring comprises one or more, same or different heteroatoms O, N, or S, and wherein two substituents may form together with the carbon-atom to which they are bound a group (C=O); or
a group of formula (S),
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 5-membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy.

R³¹ is halogen, N₃, OH, CN, NO₂, SCN, SF₅; C₁-C₆-alkyl, C₁-C₆-haloalkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₁-C₆-alkoxycarbonyl, C₃-C₆-cycloalkyl; C₃-C₆-cycloalkoxy, C₃-C₆-cycloalkyl-C₁-C₄-alkyl, C₃-C₆-cycloalkoxy-C₁-C₄ alkyl, phenyl, or a saturated, partially-, or fully unsaturated 5- or 6-membered heterocycle, wherein the cyclic moieties are unsubstituted or substituted with one or more, same or different substituents R¹¹; or two geminal substituents R³¹ form together with the atom to which they are bound a group =O or =S.

In one embodiment, R³¹ is halogen, C₁-C₃-alkyl, C₁-C₃-haloalkyl, C₁-C₃-alkoxycarbonyl, or two geminal substituents form together with the atom to which they are bound ag roup =O. In one embodiment, R³¹ is halogen, C₁-C₃-alkyl, C₁-C₃-haloalkyl, or two geminal substituents form together with the atom to which they are bound a group =O.

In one embodiment, R³¹ is halogen, C₁-C₃-alkyl, C₁-C₃-haloalkyl, C₁-C₃-alkoxycarbonyl, or two geminal substituents form together with the atom to which they are bound ag roup =O. In one embodiment, R³¹ is halogen, C₁-C₃-haloalkyl, or two geminal substituents form together with the atom to which they are bound ag roup =O.

The index m is 0, 1, or 2. Typically, m is 0 or 2. In one embodiment, m is 2. In another embodiment, m is 0.

Each R⁷ is independently C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from CN and halogen. In one embodiment, each R⁹ is independently C₁-C₃-alkyl, or C₃-C₆-cycloalkyl, which groups are unsubstituted or substituted with one or more, same or different substituents selected from CN and halogen. In another embodiment, each R⁹ is independently C₃-C₆-cycloalkyl, which is unsubstituted or substituted with one or more, same or different substituents selected from CN. In another embodiment, R⁹ is 1-cyanocyclopropyl.

Each R⁸ is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more, same or different substituents R¹¹. In one embodiment, R⁸ is H, CN, C₁-C₃-alkyl, or C₁-C₃-haloalkyl. In another embodiment, R³ is CN.

Each R⁹ is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen and CN; phenyl or benzyl, wherein the phenyl ring is unsubstituted or substituted with one or more, same or different substitutents R¹¹. In one embodiment, each R⁹ is independently H, C₁-C₃-alkyl, or C₁-C₃-haloalkyl. In another embodiment, each R⁹ is independently C₁-C₃-alkyl.

Preferably, compounds of formula (I) are compounds of formulae (II.1), or (III.1) 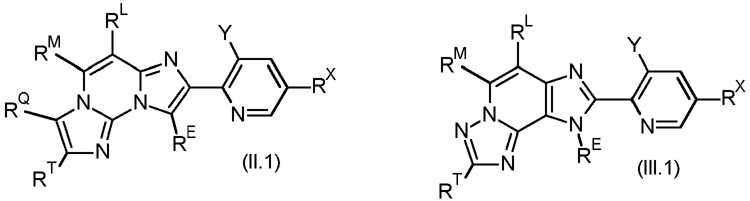 wherein all variables have a meaning as defined for formula (I).

In another embodiment, compounds of formula (I) are compounds of formulae (II.1), (III.1), or (IV.1) 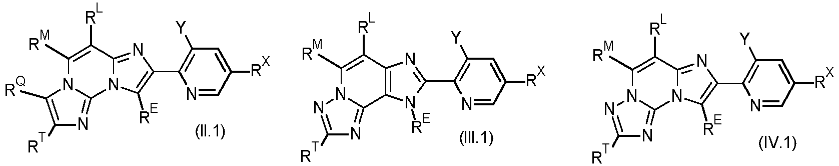 wherein all variables have a meaning as defined for formula (I).

Table A below contains combinations of meanings for variables R^{E}, Y, and R^{X} in lines S-1 to S-68. The resective numbering S-1 to S-80 of the lines of Table A is used herein below as an abbreviation for the specific combination of meanings of the variables R^{E}, Y and R^{X} in this line.

Table B below contains combinations of meanings for variables R^{L}, R^{M}, R^{Q}, and R^{T} in lines T-1 to T-29. The resective numbering T-1 to T-29 of the lines of Table B is used herein below as an abbreviation for the specific combination of meanings of the variables R^{L}, R^{M}, R^{Q}, and R^{T} in the line of Table B. Moreover, the meanings mentioned for the individual variables in Table A and Table B are *per se*, independently of the combination in which they are mentioned, a particularly preferred embodiment of the substituents in question.

**Table A: assignment of lines S-1 to S-80 to combinations of R^{E}, Y and R^{X}; "#" and "&" mean the link to the remainder of the molecule where the respective variable is situatedLine.**

| Line | R^{E} | Y | R^{X} |
|---|---|---|---|
| S-1 | H | C₂H₆-SO₂ | H |
| S-2 | H | C₂H₆-SO₂ | CF₃ |
| S-3 | H | C₂H₆-SO₂ | 4-fluorophenyl |
| S-4 | H | C₂H₆-SO₂ | Br |
| S-5 | H | C₂H₆-SO₂ | cyanomethyl |
| S-6 | H | C₂H₆-SO₂ | 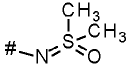 |
| S-7 | H | C₂H₆-SO₂ | 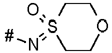 |
| S-8 | H | C₂H₆-SO₂ | 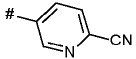 |
| S-9 | H | C₂H₆-SO₂ | 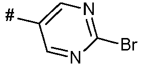 |
| S-10 | H | C₂H₆-SO₂ | 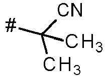 |
| S-11 | H | C₂H₆-SO₂ | cyanomethyl |
| S-12 | H | C₂H₆-SO₂ | 1-cyano-1-methyl-ethoxy |
| S-13 | H | C₂H₆-SO₂ | 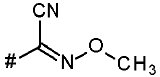 |
| S-14 | H | C₂H₆-SO₂ | 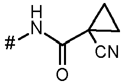 |
| S-15 | H | C₂H₆-SO₂ | 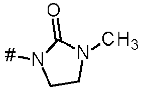 |
| S-16 | H | C₂H₆-SO₂ | 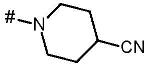 |
| S-17 | H | C₂H₆-SO₂ | 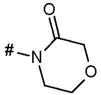 |
| S-18 | H | C₂H₆-SO₂ | -OC(CH₃)₂CN |
| S-19 | H | C₂H₆-SO₂ | 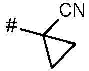 |
| S-20 | H | C₂H₆-SO₂ | 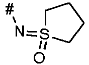 |
| S-21 | H | 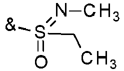 | H |
| S-22 | H | 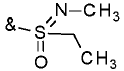 | CF₃ |
| S-23 | H | 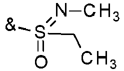 | 4-fluorophenyl |
| S-24 | H | 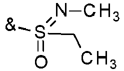 | Br |
| S-25 | H | 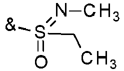 | cyanomethyl |
| S-26 | H | 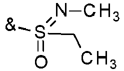 | 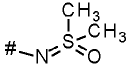 |
| S-27 | H | 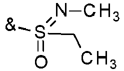 | 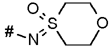 |
| S-28 | H | 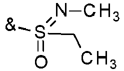 | 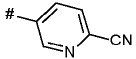 |
| S-29 | H | 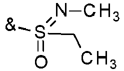 | 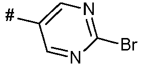 |
| S-30 | H | 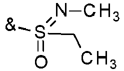 | 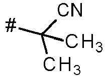 |
| S-31 | H | 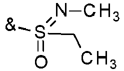 | cyanomethyl |
| S-32 | H | 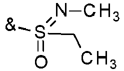 | 1-cyano-1-methyl-ethoxy |
| S-33 | H | 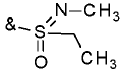 | 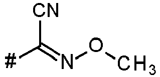 |
| S-34 | H | 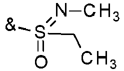 | 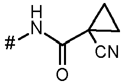 |
| S-35 | H | 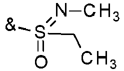 | 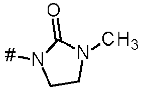 |
| S-36 | H | 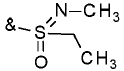 | 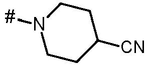 |
| S-37 | H | 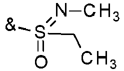 | 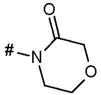 |
| S-38 | H | 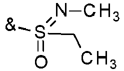 | 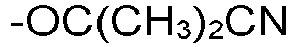 |
| S-39 | H | 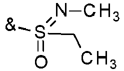 | 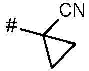 |
| S-40 | H | 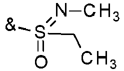 | 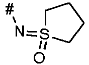 |
| S-41 | CH₃ | C₂H₆-SO₂ | H |
| S-42 | CH₃ | C₂H₆-SO₂ | CF₃ |
| S-43 | CH₃ | C₂H₆-SO₂ | 4-fluorophenyl |
| S-44 | CH₃ | C₂H₆-SO₂ | Br |
| S-45 | CH₃ | C₂H₆-SO₂ | cyanomethyl |
| S-46 | CH₃ | C₂H₆-SO₂ | 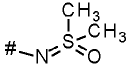 |
| S-47 | CH₃ | C₂H₆-SO₂ | 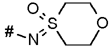 |
| S-48 | CH₃ | C₂H₆-SO₂ | 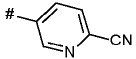 |
| S-49 | CH₃ | C₂H₆-SO₂ | 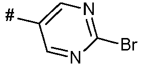 |
| S-50 | CH₃ | C₂H₆-SO₂ | 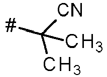 |
| S-51 | CH₃ | C₂H₆-SO₂ | cyanomethyl |
| S-52 | CH₃ | C₂H₆-SO₂ | 1-cyano-1-methyl-ethoxy |
| S-53 | CH₃ | C₂H₆-SO₂ | 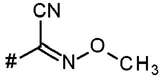 |
| S-54 | CH₃ | C₂H₆-SO₂ | 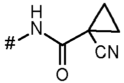 |
| S-55 | CH₃ | C₂H₆-SO₂ | 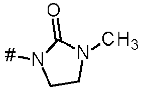 |
| S-56 | CH₃ | C₂H₆-SO₂ | 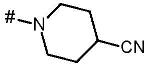 |
| S-57 | CH₃ | C₂H₆-SO₂ | 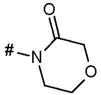 |
| S-58 | CH₃ | C₂H₆-SO₂ | -OC(CH₃)₂CN |
| S-59 | CH₃ | C₂H₆-SO₂ | 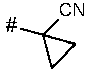 |
| S-60 | CH₃ | C₂H₆-SO₂ | 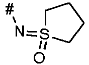 |
| S-61 | CH₃ | 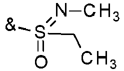 | H |
| S-62 | CH₃ | 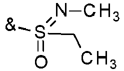 | CF₃ |
| S-63 | CH₃ | 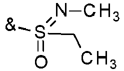 | 4-fluorophenyl |
| S-64 | CH₃ | 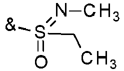 | Br |
| S-65 | CH₃ | 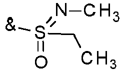 | cyanomethyl |
| S-66 | CH₃ | 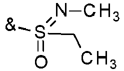 | 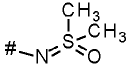 |
| S-67 | CH₃ | 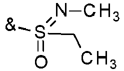 | 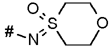 |
| S-68 | CH₃ | 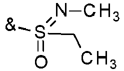 | 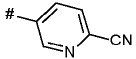 |
| S-69 | CH₃ | 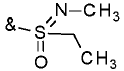 | 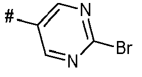 |
| S-70 | CH₃ | 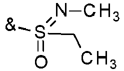 | 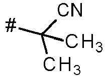 |
| S-71 | CH₃ | 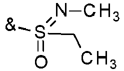 | cyanomethyl |
| S-72 | CH₃ | 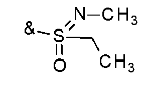 | 1-cyano-1-methyl-ethoxy |
| S-73 | CH₃ | 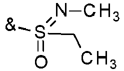 | 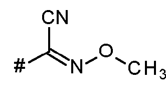 |
| S-74 | CH₃ | 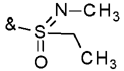 | 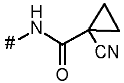 |
| S-75 | CH₃ | 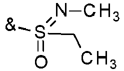 | 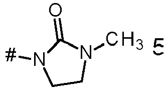 |
| S-76 | CH₃ | 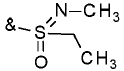 | 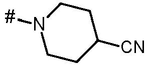 |
| S-77 | CH₃ | 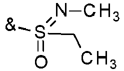 | 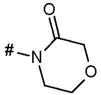 |
| S-78 | CH₃ | 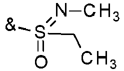 | -OC(CH₃)₂CN |
| S-79 | CH₃ | 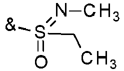 | 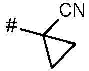 |
| S-80 | CH₃ | 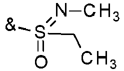 | 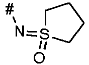 |

**Table B: assignment of lines T-1 to T-29 to combinations of R^{L}, R^{M}, R^{Q}, and R^{T}.**

| Line | R^{L} | R^{M} | R^{Q} | R^{T} |
|---|---|---|---|---|
| T-1 | H | H | H | H |
| T-2 | CF₃ | H | H | H |
| T-3 | OCF₃ | H | H | H |
| T-4 | H | CF₃ | H | H |
| T-5 | H | OCF₃ | H | H |
| T-6 | H | H | CF₃ | H |
| T-7 | H | H | OCF₃ | H |
| T-8 | H | H | H | CF₃ |
| T-9 | H | H | H | OCF₃ |
| T-10 | CF₃ | CF₃ | H | H |
| T-11 | CF₃ | H | CF₃ | H |
| T-12 | CF₃ | H | H | CF₃ |
| T-13 | H | CF₃ | CF₃ | H |
| T-14 | H | CF₃ | H | CF₃ |
| T-15 | H | H | CF₃ | CF₃ |
| T-16 | OCF₃ | OCF₃ | H | H |
| T-17 | OCF₃ | H | OCF₃ | H |
| T-18 | OCF₃ | H | H | OCF₃ |
| T-19 | H | OCF₃ | OCF₃ | H |
| T-20 | H | OCF₃ | H | OCF₃ |
| T-21 | H | H | OCF₃ | OCF₃ |
| T-22 | CF₃ | CF₃ | CF₃ | H |
| T-23 | CF₃ | H | CF₃ | CF₃ |
| T-24 | CF₃ | CF₃ | H | CF₃ |
| T-25 | H | CF₃ | CF₃ | CF₃ |
| T-26 | OCF₃ | OCF₃ | OCF₃ | H |
| T-27 | OCF₃ | H | OCF₃ | OCF₃ |
| T-28 | OCF₃ | OCF₃ | H | OCF₃ |
| T-29 | H | OCF₃ | OCF₃ | OCF₃ |

The following Tables 1 to 58 represent preferred embodiments of combinations of variables R^{L} , R^{M}, R^{Q}, R^{T}, R^{E}, Y and R^{X} with formulae (II.1), or (III.1). In case the variable does not occur in the respective formula, the respective definition of the variable is void.

Table 1: Compound of formula (II.1), wherein R^{L}, R^{M}, R^{Q}, and R^{T} are as defined as in line T-1 of Table B, and wherein the definition of the variables R^{E}, Y,, and R^{X} is as defined in a line of Table A.

Table 2: Compound of formula (II.1), wherein R^{L}, R^{M}, R^{Q}, and R^{T} are as defined as in line T-2 of Table B, and wherein the definition of the variables R^{E}, Y,, and R^{X} is as defined in a line of Table A.

Table 3: Compound of formula (II.1), wherein R^{L}, R^{M}, R^{Q}, and R^{T} are as defined as in line T-3 of Table B, and wherein the definition of the variables R^{E}, Y,, and R^{X} is as defined in a line of Table A.

Table 4: Compound of formula (II.1), wherein R^{L}, R^{M}, R^{Q}, and R^{T} are as defined as in line T-4 of Table B, and wherein the definition of the variables R^{E}, Y,, and R^{X} is as defined in a line of Table A.

Table 5: Compound of formula (II.1), wherein R^{L}, R^{M}, R^{Q}, and R^{T} are as defined as in line T-5 of Table B, and wherein the definition of the variables R^{E}, Y,, and R^{X} is as defined in a line of Table A.

Table 6: Compound of formula (II.1), wherein R^{L}, R^{M}, R^{Q}, and R^{T} are as defined as in line T-6 of Table B, and wherein the definition of the variables R^{E}, Y,, and R^{X} is as defined in a line of Table A.

Table 7: Compound of formula (II.1), wherein R^{L}, R^{M}, R^{Q}, and R^{T} are as defined as in line T-7 of Table B, and wherein the definition of the variables R^{E}, Y,, and R^{X} is as defined in a line of Table A.

Table 8: Compound of formula (II.1), wherein R^{L}, R^{M}, R^{Q}, and R^{T} are as defined as in line T-8 of Table B, and wherein the definition of the variables R^{E}, Y,, and R^{X} is as defined in a line of Table A.

Table 9: Compound of formula (II.1), wherein R^{L}, R^{M}, R^{Q}, and R^{T} are as defined as in line T-9 of Table B, and wherein the definition of the variables R^{E}, Y,, and R^{X} is as defined in a line of Table A.

Table 10: Compound of formula (II.1), wherein R^{L}, R^{M}, R^{Q}, and R^{T} are as defined as in line T-10 of Table B, and wherein the definition of the variables R^{E}, Y,, and R^{X} is as defined in a line of Table A.

Table 11: Compound of formula (II.1), wherein R^{L}, R^{M}, R^{Q}, and R^{T} are as defined as in line T-11 of Table B, and wherein the definition of the variables R^{E}, Y,, and R^{X} is as defined in a line of Table A.

Table 12: Compound of formula (II.1), wherein R^{L}, R^{M}, R^{Q}, and R^{T} are as defined as in line T-12 of Table B, and wherein the definition of the variables R^{E}, Y,, and R^{X} is as defined in a line of Table A.

Table 13: Compound of formula (II.1), wherein R^{L}, R^{M}, R^{Q}, and R^{T} are as defined as in line T-13 of Table B, and wherein the definition of the variables R^{E}, Y,, and R^{X} is as defined in a line of Table A.

Table 14: Compound of formula (II.1), wherein R^{L}, R^{M}, R^{Q}, and R^{T} are as defined as in line T-14 of Table B, and wherein the definition of the variables R^{E}, Y,, and R^{X} is as defined in a line of Table A.

Table 15: Compound of formula (II.1), wherein R^{L}, R^{M}, R^{Q}, and R^{T} are as defined as in line T-15 of Table B, and wherein the definition of the variables R^{E}, Y,, and R^{X} is as defined in a line of Table A.

Table 16: Compound of formula (II.1), wherein R^{L}, R^{M}, R^{Q}, and R^{T} are as defined as in line T-16 of Table B, and wherein the definition of the variables R^{E}, Y,, and R^{X} is as defined in a line of Table A.

Table 17: Compound of formula (II.1), wherein R^{L}, R^{M}, R^{Q}, and R^{T} are as defined as in line T-17 of Table B, and wherein the definition of the variables R^{E}, Y,, and R^{X} is as defined in a line of Table A.

Table 18: Compound of formula (II.1), wherein R^{L}, R^{M}, R^{Q}, and R^{T} are as defined as in line T-18 of Table B, and wherein the definition of the variables R^{E}, Y,, and R^{X} is as defined in a line of Table A.

Table 19: Compound of formula (II.1), wherein R^{L}, R^{M}, R^{Q}, and R^{T} are as defined as in line T-19 of Table B, and wherein the definition of the variables R^{E}, Y,, and R^{X} is as defined in a line of Table A.

Table 20: Compound of formula (II.1), wherein R^{L}, R^{M}, R^{Q}, and R^{T} are as defined as in line T-20 of Table B, and wherein the definition of the variables R^{E}, Y,, and R^{X} is as defined in a line of Table A.

Table 21: Compound of formula (II.1), wherein R^{L}, R^{M}, R^{Q}, and R^{T} are as defined as in line T-21 of Table B, and wherein the definition of the variables R^{E}, Y,, and R^{X} is as defined in a line of Table A.

Table 22: Compound of formula (II.1), wherein R^{L}, R^{M}, R^{Q}, and R^{T} are as defined as in line T-22 of Table B, and wherein the definition of the variables R^{E}, Y,, and R^{X} is as defined in a line of Table A.

Table 23: Compound of formula (II.1), wherein R^{L}, R^{M}, R^{Q}, and R^{T} are as defined as in line T-23 of Table B, and wherein the definition of the variables R^{E}, Y,, and R^{X} is as defined in a line of Table A.

Table 24: Compound of formula (II.1), wherein R^{L}, R^{M}, R^{Q}, and R^{T} are as defined as in line T-24 of Table B, and wherein the definition of the variables R^{E}, Y,, and R^{X} is as defined in a line of Table A.

Table 25: Compound of formula (II.1), wherein R^{L}, R^{M}, R^{Q}, and R^{T} are as defined as in line T-25 of Table B, and wherein the definition of the variables R^{E}, Y,, and R^{X} is as defined in a line of Table A.

Table 26: Compound of formula (II.1), wherein R^{L}, R^{M}, R^{Q}, and R^{T} are as defined as in line T-26 of Table B, and wherein the definition of the variables R^{E}, Y,, and R^{X} is as defined in a line of Table A.

Table 27: Compound of formula (II.1), wherein R^{L}, R^{M}, R^{Q}, and R^{T} are as defined as in line T-27 of Table B, and wherein the definition of the variables R^{E}, Y,, and R^{X} is as defined in a line of Table A.

Table 28: Compound of formula (II.1), wherein R^{L}, R^{M}, R^{Q}, and R^{T} are as defined as in line T-28 of Table B, and wherein the definition of the variables R^{E}, Y,, and R^{X} is as defined in a line of Table A.

Table 29: Compound of formula (II.1), wherein R^{L}, R^{M}, R^{Q}, and R^{T} are as defined as in line T-29 of Table B, and wherein the definition of the variables R^{E}, Y,, and R^{X} is as defined in a line of Table A.

Table 30: Compound of formula (III.1), wherein R^{L}, R^{M}, R^{Q}, and R^{T} are as defined as in line T-1 of Table B, and wherein the definition of the variables R^{E}, Y,, and R^{X} is as defined in a line of Table A.

Table 31: Compound of formula (III.1), wherein R^{L}, R^{M}, R^{Q}, and R^{T} are as defined as in line T-2 of Table B, and wherein the definition of the variables R^{E}, Y,, and R^{X} is as defined in a line of Table A.

Table 32: Compound of formula (III.1), wherein R^{L}, R^{M}, R^{Q}, and R^{T} are as defined as in line T-3 of Table B, and wherein the definition of the variables R^{E}, Y,, and R^{X} is as defined in a line of Table A.

Table 33: Compound of formula (III.1), wherein R^{L}, R^{M}, R^{Q}, and R^{T} are as defined as in line T-4 of Table B, and wherein the definition of the variables R^{E}, Y,, and R^{X} is as defined in a line of Table A.

Table 34: Compound of formula (III.1), wherein R^{L}, R^{M}, R^{Q}, and R^{T} are as defined as in line T-5 of Table B, and wherein the definition of the variables R^{E}, Y,, and R^{X} is as defined in a line of Table A.

Table 35: Compound of formula (III.1), wherein R^{L}, R^{M}, R^{Q}, and R^{T} are as defined as in line T-6 of Table B, and wherein the definition of the variables R^{E}, Y,, and R^{X} is as defined in a line of Table A.

Table 36: Compound of formula (III.1), wherein R^{L}, R^{M}, R^{Q}, and R^{T} are as defined as in line T-7 of Table B, and wherein the definition of the variables R^{E}, Y,, and R^{X} is as defined in a line of Table A.

Table 37: Compound of formula (III.1), wherein R^{L}, R^{M}, R^{Q}, and R^{T} are as defined as in line T-8 of Table B, and wherein the definition of the variables R^{E}, Y,, and R^{X} is as defined in a line of Table A.

Table 38: Compound of formula (III.1), wherein R^{L}, R^{M}, R^{Q}, and R^{T} are as defined as in line T-9 of Table B, and wherein the definition of the variables R^{E}, Y,, and R^{X} is as defined in a line of Table A.

Table 39: Compound of formula (III.1), wherein R^{L}, R^{M}, R^{Q}, and R^{T} are as defined as in line T-10 of Table B, and wherein the definition of the variables R^{E}, Y,, and R^{X} is as defined in a line of Table A.

Table 40: Compound of formula (III.1), wherein R^{L}, R^{M}, R^{Q}, and R^{T} are as defined as in line T-11 of Table B, and wherein the definition of the variables R^{E}, Y,, and R^{X} is as defined in a line of Table A.

Table 41: Compound of formula (III.1), wherein R^{L}, R^{M}, R^{Q}, and R^{T} are as defined as in line T-12 of Table B, and wherein the definition of the variables R^{E}, Y,, and R^{X} is as defined in a line of Table A.

Table 42: Compound of formula (III.1), wherein R^{L}, R^{M}, R^{Q}, and R^{T} are as defined as in line T-13 of Table B, and wherein the definition of the variables R^{E}, Y,, and R^{X} is as defined in a line of Table A.

Table 43: Compound of formula (III.1), wherein R^{L}, R^{M}, R^{Q}, and R^{T} are as defined as in line T-14 of Table B, and wherein the definition of the variables R^{E}, Y,, and R^{X} is as defined in a line of Table A.

Table 44: Compound of formula (III.1), wherein R^{L}, R^{M}, R^{Q}, and R^{T} are as defined as in line T-15 of Table B, and wherein the definition of the variables R^{E}, Y,, and R^{X} is as defined in a line of Table A.

Table 45: Compound of formula (III.1), wherein R^{L}, R^{M}, R^{Q}, and R^{T} are as defined as in line T-16 of Table B, and wherein the definition of the variables R^{E}, Y,, and R^{X} is as defined in a line of Table A.

Table 46: Compound of formula (III.1), wherein R^{L}, R^{M}, R^{Q}, and R^{T} are as defined as in line T-17 of Table B, and wherein the definition of the variables R^{E}, Y,, and R^{X} is as defined in a line of Table A.

Table 47: Compound of formula (III.1), wherein R^{L}, R^{M}, R^{Q}, and R^{T} are as defined as in line T-18 of Table B, and wherein the definition of the variables R^{E}, Y,, and R^{X} is as defined in a line of Table A.

Table 48: Compound of formula (III.1), wherein R^{L}, R^{M}, R^{Q}, and R^{T} are as defined as in line T-19 of Table B, and wherein the definition of the variables R^{E}, Y,, and R^{X} is as defined in a line of Table A.

Table 49: Compound of formula (III.1), wherein R^{L}, R^{M}, R^{Q}, and R^{T} are as defined as in line T-20 of Table B, and wherein the definition of the variables R^{E}, Y,, and R^{X} is as defined in a line of Table A.

Table 50: Compound of formula (III.1), wherein R^{L}, R^{M}, R^{Q}, and R^{T} are as defined as in line T-21 of Table B, and wherein the definition of the variables R^{E}, Y,, and R^{X} is as defined in a line of Table A.

Table 51: Compound of formula (III.1), wherein R^{L}, R^{M}, R^{Q}, and R^{T} are as defined as in line T-22 of Table B, and wherein the definition of the variables R^{E}, Y,, and R^{X} is as defined in a line of Table A.

Table 52: Compound of formula (III.1), wherein R^{L}, R^{M}, R^{Q}, and R^{T} are as defined as in line T-23 of Table B, and wherein the definition of the variables R^{E}, Y,, and R^{X} is as defined in a line of Table A.

Table 53: Compound of formula (III.1), wherein R^{L}, R^{M}, R^{Q}, and R^{T} are as defined as in line T-24 of Table B, and wherein the definition of the variables R^{E}, Y,, and R^{X} is as defined in a line of Table A.

Table 54: Compound of formula (III.1), wherein R^{L}, R^{M}, R^{Q}, and R^{T} are as defined as in line T-25 of Table B, and wherein the definition of the variables R^{E}, Y,, and R^{X} is as defined in a line of Table A.

Table 55: Compound of formula (III.1), wherein R^{L}, R^{M}, R^{Q}, and R^{T} are as defined as in line T-26 of Table B, and wherein the definition of the variables R^{E}, Y,, and R^{X} is as defined in a line of Table A.

Table 56: Compound of formula (III.1), wherein R^{L}, R^{M}, R^{Q}, and R^{T} are as defined as in line T-27 of Table B, and wherein the definition of the variables R^{E}, Y,, and R^{X} is as defined in a line of Table A.

Table 57: Compound of formula (III.1), wherein R^{L}, R^{M}, R^{Q}, and R^{T} are as defined as in line T-28 of Table B, and wherein the definition of the variables R^{E}, Y,, and R^{X} is as defined in a line of Table A.

Table 58: Compound of formula (III.1), wherein R^{L}, R^{M}, R^{Q}, and R^{T} are as defined as in line T-29 of Table B, and wherein the definition of the variables R^{E}, Y,, and R^{X} is as defined in a line of Table A.

Table 59: Compound of formula (IV.1), wherein R^{L}, R^{M}, R^{Q}, and R^{T} are as defined as in line T-1 of Table B, and wherein the definition of the variables R^{E}, Y,, and R^{X} is as defined in a line of Table A.

Table 60: Compound of formula (IV.1), wherein R^{L}, R^{M}, R^{Q}, and R^{T} are as defined as in line T-2 of Table B, and wherein the definition of the variables R^{E}, Y,, and R^{X} is as defined in a line of Table A.

Table 61: Compound of formula (IV.1), wherein R^{L}, R^{M}, R^{Q}, and R^{T} are as defined as in line T-3 of Table B, and wherein the definition of the variables R^{E}, Y,, and R^{X} is as defined in a line of Table A.

Table 62: Compound of formula (IV.1), wherein R^{L}, R^{M}, R^{Q}, and R^{T} are as defined as in line T-4 of Table B, and wherein the definition of the variables R^{E}, Y,, and R^{X} is as defined in a line of Table A.

Table 63: Compound of formula (IV.1), wherein R^{L}, R^{M}, R^{Q}, and R^{T} are as defined as in line T-5 of Table B, and wherein the definition of the variables R^{E}, Y,, and R^{X} is as defined in a line of Table A.

Table 64: Compound of formula (IV.1), wherein R^{L}, R^{M}, R^{Q}, and R^{T} are as defined as in line T-6 of Table B, and wherein the definition of the variables R^{E}, Y,, and R^{X} is as defined in a line of Table A.

Table 65: Compound of formula (IV.1), wherein R^{L}, R^{M}, R^{Q}, and R^{T} are as defined as in line T-7 of Table B, and wherein the definition of the variables R^{E}, Y,, and R^{X} is as defined in a line of Table A.

Table 66: Compound of formula (IV.1), wherein R^{L}, R^{M}, R^{Q}, and R^{T} are as defined as in line T-8 of Table B, and wherein the definition of the variables R^{E}, Y,, and R^{X} is as defined in a line of Table A.

Table 67: Compound of formula (IV.1), wherein R^{L}, R^{M}, R^{Q}, and R^{T} are as defined as in line T-9 of Table B, and wherein the definition of the variables R^{E}, Y,, and R^{X} is as defined in a line of Table A.

Table 68: Compound of formula (IV.1), wherein R^{L}, R^{M}, R^{Q}, and R^{T} are as defined as in line T-10 of Table B, and wherein the definition of the variables R^{E}, Y,, and R^{X} is as defined in a line of Table A.

Table 69: Compound of formula (IV.1), wherein R^{L}, R^{M}, R^{Q}, and R^{T} are as defined as in line T-11 of Table B, and wherein the definition of the variables R^{E}, Y,, and R^{X} is as defined in a line of Table A.

Table 70: Compound of formula (IV.1), wherein R^{L}, R^{M}, R^{Q}, and R^{T} are as defined as in line T-12 of Table B, and wherein the definition of the variables R^{E}, Y,, and R^{X} is as defined in a line of Table A.

Table 71: Compound of formula (IV.1), wherein R^{L}, R^{M}, R^{Q}, and R^{T} are as defined as in line T-13 of Table B, and wherein the definition of the variables R^{E}, Y,, and R^{X} is as defined in a line of Table A.

Table 72: Compound of formula (IV.1), wherein R^{L}, R^{M}, R^{Q}, and R^{T} are as defined as in line T-14 of Table B, and wherein the definition of the variables R^{E}, Y,, and R^{X} is as defined in a line of Table A.

Table 73: Compound of formula (IV.1), wherein R^{L}, R^{M}, R^{Q}, and R^{T} are as defined as in line T-15 of Table B, and wherein the definition of the variables R^{E}, Y,, and R^{X} is as defined in a line of Table A.

Table 74: Compound of formula (IV.1), wherein R^{L}, R^{M}, R^{Q}, and R^{T} are as defined as in line T-16 of Table B, and wherein the definition of the variables R^{E}, Y,, and R^{X} is as defined in a line of Table A.

Table 75: Compound of formula (IV.1), wherein R^{L}, R^{M}, R^{Q}, and R^{T} are as defined as in line T-17 of Table B, and wherein the definition of the variables R^{E}, Y,, and R^{X} is as defined in a line of Table A.

Table 76: Compound of formula (IV.1), wherein R^{L}, R^{M}, R^{Q}, and R^{T} are as defined as in line T-18 of Table B, and wherein the definition of the variables R^{E}, Y,, and R^{X} is as defined in a line of Table A.

Table 77: Compound of formula (IV.1), wherein R^{L}, R^{M}, R^{Q}, and R^{T} are as defined as in line T-19 of Table B, and wherein the definition of the variables R^{E}, Y,, and R^{X} is as defined in a line of Table A.

Table 78: Compound of formula (IV.1), wherein R^{L}, R^{M}, R^{Q}, and R^{T} are as defined as in line T-20 of Table B, and wherein the definition of the variables R^{E}, Y,, and R^{X} is as defined in a line of Table A.

Table 79: Compound of formula (IV.1), wherein R^{L}, R^{M}, R^{Q}, and R^{T} are as defined as in line T-21 of Table B, and wherein the definition of the variables R^{E}, Y,, and R^{X} is as defined in a line of Table A.

Table 80: Compound of formula (IV.1), wherein R^{L}, R^{M}, R^{Q}, and R^{T} are as defined as in line T-22 of Table B, and wherein the definition of the variables R^{E}, Y,, and R^{X} is as defined in a line of Table A.

Table 81: Compound of formula (IV.1), wherein R^{L}, R^{M}, R^{Q}, and R^{T} are as defined as in line T-23 of Table B, and wherein the definition of the variables R^{E}, Y,, and R^{X} is as defined in a line of Table A.

Table 82: Compound of formula (IV.1), wherein R^{L}, R^{M}, R^{Q}, and R^{T} are as defined as in line T-24 of Table B, and wherein the definition of the variables R^{E}, Y,, and R^{X} is as defined in a line of Table A.

Table 83: Compound of formula (IV.1), wherein R^{L}, R^{M}, R^{Q}, and R^{T} are as defined as in line T-25 of Table B, and wherein the definition of the variables R^{E}, Y,, and R^{X} is as defined in a line of Table A.

Table 84: Compound of formula (IV.1), wherein R^{L}, R^{M}, R^{Q}, and R^{T} are as defined as in line T-26 of Table B, and wherein the definition of the variables R^{E}, Y,, and R^{X} is as defined in a line of Table A.

Table 85: Compound of formula (IV.1), wherein R^{L}, R^{M}, R^{Q}, and R^{T} are as defined as in line T-27 of Table B, and wherein the definition of the variables R^{E}, Y,, and R^{X} is as defined in a line of Table A.

Table 86: Compound of formula (IV.1), wherein R^{L}, R^{M}, R^{Q}, and R^{T} are as defined as in line T-28 of Table B, and wherein the definition of the variables R^{E}, Y,, and R^{X} is as defined in a line of Table A.

Table 87: Compound of formula (IV.1), wherein R^{L}, R^{M}, R^{Q}, and R^{T} are as defined as in line T-29 of Table B, and wherein the definition of the variables R^{E}, Y,, and R^{X} is as defined in a line of Table A.

In one embodiment, the compounds of formula (I) are compounds of formula (I-A), or (I-B) wherein
R^{E} is H; CH₃, which is unsubstituted or halogenated;
R^{L}, R^{M}, R^{Q}, and R^{T} are independently H; C₁-C₃-alkyl, C₁-C₃-alkoxy, which groups are unsubstituted or halogenated;
X is phenyl or a heteroaryl A1;
Y is C₂H₅-S, C₂H₅-SO₂, or S(=O)(=NCH₃)C₂H₅;
each R^{X} is independently halogen;
C₁-C₃-alkyl, C₁-C₃-alkoxy, which groups are unsubstituted or substituted with CN or halogen (such as 1-cyanoisopropyl);
NR³C(=O)R⁷, C(R⁸)=N-O(R⁹);
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents selected from halogen, CN, C₁-C₃-alkyl, and C₁-C₃-haloalkyl and wherein two substituents may form together with the carbon-atom to which they are bound a group (C=O), and wherein said N- and S-atoms are independently oxidized, or non-oxidized; or
a group of formula (S) 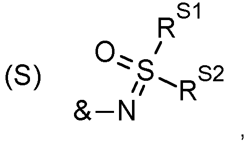
wherein each R^{S1}, R^{S2} is independently selected from C₁-C₃-alkyl, which groups are unsubstituted, or halogenated;
or two substituents R^{S1} R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to; and
n is 0 or 1.

In another embodiment, the compounds of formula (I) are compounds of formula (I-A), or (I-B) wherein
R^{E} is H; CH₃, which is unsubstituted or halogenated;
R^{L}, R^{M}, R^{Q}, and R^{T} are independently H; CH₃, OCH₃, CF₃, or OCF₃;
X is phenyl or a heteroaryl A1;
Y is C₂H₅-SO₂, or S(=O)(=NCH₃)C₂H₅;
each R^{X} is independently halogen;
C₁-C₃-alkyl, C₁-C₃-alkoxy, which groups are unsubstituted or substituted with CN or halogen (such as 1-cyanoisopropyl);
NHC(=O)R⁷, C(R⁸)=N-OR⁹);
phenyl, which is unsubstituted or halogenated;
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents selected from halogen, CN, C₁-C₃-alkyl, C₁-C₃-alkoxy, C₁-C₃-haloalkyl, and C₁-C₃-haloalkoxy, and wherein two substituents may form together with the carbon-atom to which they are bound a group (C=O), and wherein said N- and S-atoms are independently oxidized, or non-oxidized; or a group of formula (S) 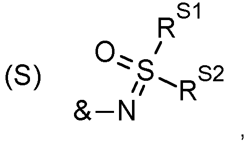
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 6-membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein two substituents may form together with the carbon-atom to which they are bound a group (C=O), and wherein said heterocycle comprises no, one or more, same or different heteroatoms O, N, or S in addition to the sulfur atom to which R^{S1} and R^{S2} are bound to.
R⁷ is C₁-C₃-alkyl or C₃-C₆-cycloalkyl, which groups are unsubstituted or substituted with one or more, same or different substituents selected from halogen and CN;
R⁸ is H, CN, C₁-C₃-alkyl, or C₁-C₃-haloalkyl;
R⁹ is H, C₁-C₃-alkyl, C₁-C₃-haloalkyl; and
n is 0 or 1.

In another embodiment, the compounds of formula (I) are compounds of formula (I-A), or (I-B) wherein
R^{E} is H or CH₃;
R^{L}, R^{M}, R^{Q}, and R^{T} are independently H or CF₃;
X is a heteroaryl A1;
Y is C₂H₅-SO₂, or S(=O)(=NCH₃)C₂H₅;
each R^{X} is independently halogen;
C₁-C₃-alkyl, C₁-C₃-alkoxy, which groups are unsubstituted or substituted with CN or halogen (such as 1-cyanoisopropyl);
NHC(=O)R⁷, C(R⁸)=N-OR⁹);
phenyl, which is unsubstituted or halogenated;
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents selected from halogen, CN, C₁-C₃-alkyl, or C₁-C₃-haloalkyl, and wherein two substituents may form together with the carbon-atom to which they are bound a group (C=O), and wherein said N- and S-atoms are independently oxidized, or non-oxidized; or
a group of formula (S) 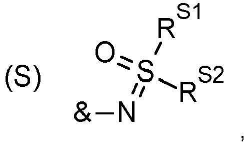
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 6-membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to;
R⁷ is C₃-C₆-cycloalkyl, which groups are unsubstituted or substituted with CN;
R⁸ is CN;
R⁹ is H, C₁-C₃-alkyl, C₁-C₃-haloalkyl; and
n is 0 or 1.

In one embodiment, the compounds of formula (I) are compounds of formula (II.1), (II.1), or (IV.1)
wherein
R^{E} is H; CH₃, which is unsubstituted or halogenated;
R^{L}, R^{M}, R^{Q}, and R^{T} are independently H; C₁-C₃-alkyl, C₁-C₃-alkoxy, which groups are unsubstituted or halogenated;
X is phenyl or a heteroaryl A1;
Y is C₂H₅-S, C₂H₅-SO₂, or S(=O)(=NCH₃)C₂H₅;
each R^{X} is independently halogen;
C₁-C₃-alkyl, C₁-C₃-alkoxy, C₃-C₆-cyclopropyl; which groups are unsubstituted or substituted with CN or halogen (such as 1-cyanoisopropyl);
NR³C(=O)R⁷;
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents selected from halogen, CN, C₁-C₃-alkyl, and C₁-C₃-haloalkyl and wherein two substituents may form together with the carbon-atom to which they are bound a group (C=O), and wherein said N- and S-atoms are independently oxidized, or non-oxidized; or
a group of formula (S) 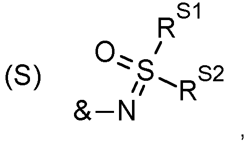
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to; and
n is 0 or 1.

In one embodiment, the compounds of formula (I) are compounds of formula (II.1), (II.1), or (IV.1)
wherein
R^{E} is H; CH₃, which is unsubstituted or halogenated;
R^{L}, R^{M}, R^{Q}, and R^{T} are independently H; C₁-C₃-alkyl, C₁-C₃-alkoxy, which groups are unsubstituted or halogenated;
X is phenyl or a heteroaryl A1;
Y is C₂H₅-S, C₂H₅-SO₂, or S(=O)(=NCH₃)C₂H₅;
each R^{X} is independently halogen;
C₁-C₃-alkyl, C₁-C₃-alkoxy, C₃-C₆-cyclopropyl; which groups are unsubstituted or substituted with CN or halogen (such as 1-cyanoisopropyl);
a 5- or 6-membered saturated, partially unsaturated, or fully unsaturated heterocyclic ring,
wherein said heterocyclic ring comprises one or more, same or different heteroatoms O, N, or S, and is unsubstituted, or substituted with one or more, same or different substituents selected from halogen, CN, C₁-C₃-alkyl, and C₁-C₃-haloalkyl and wherein two substituents may form together with the carbon-atom to which they are bound a group (C=O); or a group of formula (S) 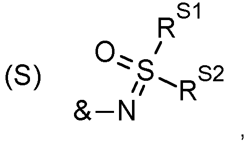
wherein each R^{S1}, R^{S2} is independently selected from C₁-C₃-alkyl, which groups are unsubstituted, or halogenated;
or two substituents R^{S1} R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy,; and
n is 0 or 1.

In another embodiment, the compounds of formula (I) are compounds of formula (I-A), wherein
R^{E}, R^{L}, R^{M}, R^{Q}, and R^{T} are independently H; or
   C₁-C₃-alkyl, which is unsubstituted or halogenated;
each R^{X} is independently halogen, C₁-C₃-alkyl, C₁-C₃-haloalkyl; or
phenyl, which is unsubstituted or halogenated;
X is a heteroaryl A1;
Y is C₂H₅-SO₂;
n is 0 or 1.

The term "compound(s) of the invention" refers to compound(s) of formula (I), or "compound(s) (I)", and includes their salts, tautomers, stereoisomers, and N-oxides.

The invention also relates to agrochemical compositions comprising an auxiliary and at least one compound (I).

An agrochemical composition comprises a pesticidally effective amount of a compound (I).

The compounds I can be converted into customary types of agro-chemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International. The compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders. Suitable solvents and liquid carriers are water and organic solvents. Suitable solid carriers or fillers are mineral earths.

Suitable surfactants are surface-active compounds, e.g. anionic, cationic, nonionic, and amphoteric surfactants, block polymers, polyelectrolytes. Such surfactants can be used as emusifier, dispersant, solubilizer, wetter, penetration enhancer, protective colloid, or adjuvant. Surfactants are listed in McCutcheon's, Vol.1: Emulsifiers & Detergents, McCutcheon's Directories, Glen Rock, USA, 2008 (International or North American Ed.). Suitable anionic surfactants are alkali, alkaline earth, or ammonium salts of sulfonates, sulfates, phosphates, carboxylates. Suitable nonionic surfactants are alkoxylates, N-subsituted fatty acid amides, amine oxides, esters, sugar-based surfactants, polymeric surfactants. Suitable cationic surfactants are qua-ternary surfactants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100%.

Various types of oils, wetters, adjuvants, or fertilizer may be added to the active substances or the compositions comprising them as premix or, if appropriate not until immediately prior to use (tank mix). These agents can be admixed with the compositions according to the invention in a weight ratio of 1:100 to 100:1.

The user applies the composition according to the invention usually from a predosage device, a knapsack sprayer, a spray tank, a spray plane, or an irrigation system. Usually, the agro-chemical composition is made up with water, buffer, and/or further auxiliaries to the desired application concentration and the ready-to-use spray liquor or the agrochemical composition according to the invention is thus obtained. Usually, 20 to 2000 liters, of the ready-to-use spray liquor are applied per hectare of agricultural useful area.

The compounds I are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound (I).

The compounds I are also suitable for use in combating or controlling animal pests. There-fore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound (I). The compounds I are effective through both contact and ingestion to any and all developmental stages, such as egg, larva, pupa, and adult.

The compounds I can be applied as such or in form of compositions comprising them.

The application can be carried out both before and after the infestation of the crops, plants, plant propagation materials by the pests.

The term "contacting" includes both direct contact (applying the compounds/compositions directly on the animal pest or plant) and indirect contact (applying the compounds/compositions to the locus).

The term "animal pest" includes arthropods, gastropods, and nematodes. Preferred animal pests according to the invention are arthropods, preferably insects and arachnids, in particular insects.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grape-fruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, , shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "seed" embraces seeds and plant propagules including true seeds, seed pieces, suckers, corms, bulbs, fruit, tubers, grains, cuttings, cut shoots, and means preferably true seeds.

"Pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare.

The compounds I are also suitable for use against non-crop insect pests. For use against said non-crop pests, compounds I can be used as bait composition, gel, general insect spray, aerosol, as ultra-low volume application and bed net (impregnated or surface applied).

The term "non-crop insect pest" refers to pests, which are particularly relevant for non-crop targets, e.g. ants, termites, wasps, flies, ticks, mosquitoes, bed bugs, crickets, or cockroaches, such as: *Aedes aegypti, Musca domestica, Tribolium* spp.; termites such as *Reticulitermes flavipes, Coptotermes formosanus;* roaches such as *Blatella germanica, Periplaneta Americana;* ants such as *Solenopsis invicta, Linepithema humile,* and *Camponotus pennsylvanicus.*

The bait can be a liquid, a solid or a semisolid preparation (e.g. a gel). For use in bait compositions, the typical content of active ingredient is from 0.001 wt% to 15 wt%, desirably from 0.001 wt% to 5 wt% of active compound.

The compounds I and its compositions can be used for protecting wooden materials such as trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 wt%, preferably from 0.1 to 45 wt%, and more preferably from 1 to 25 wt% of at least one repellent and/or insecticide.

### Pests

The compounds of the invention are especially suitable for efficiently combating animal pests e.g. arthropods, and nematodes including:
insects from the sub-order of *Auchenorrhyncha,* e.g. *Amrasca biguttula, Empoasca* spp., *Nephotettix virescens, Sogatella furcifera, Mahanarva* spp., *Laodelphax striatellus, Nilaparvata lugens, Diaphorina citri;*
*Lepidoptera,* e.g. *Helicoverpa* spp., *Heliothis virescens, Lobesia botrana, Ostrinia nubilalis, Plutella xylostella, Pseudoplusia includens, Scirpophaga incertulas, Spodoptera* spp., *Trichoplusia ni, Tuta absoluta, Cnaphalocrocis medialis, Cydia pomonella, Chilo suppressalis, Anticarsia gemmatalis, Agrotis ipsilon, Chrysodeixis includens;*
True bugs, e.g. *Lygus* spp., Stink bugs such as *Euschistus* spp., *Halyomorpha halys, Nezara viridula, Piezodorus guildinii, Dichelops furcatus;*
Thrips, e.g. *Frankliniella* spp., Thrips spp., *Dichromothrips corbettii;*
Aphids, e.g. *Acyrthosiphon pisum, Aphis* spp., *Myzus persicae, Rhopalosiphum* spp., *Schizaphis graminum, Megoura viciae;*
Whiteflies, e.g. *Trialeurodes vaporariorum, Bemisia* spp.;
*Coleoptera,* e.g. *Phyllotreta* spp., *Melanotus* spp., *Meligethes aeneus, Leptinotarsa decimlineata, Ceutorhynchus* spp., *Diabrotica* spp., *Anthonomus grandis, Atomaria linearia, Agriotes* spp., *Epilachna spp.; Limonius spp., Popillia spp. (e.g. P.japonica);*
Flies, e.g. *Delia* spp., *Ceratitis capitate, Bactrocera* spp., *Liriomyza* spp.;
*Coccoidea,* e.g. *Aonidiella aurantia, Ferrisia* virgate;
Anthropods of class *Arachnida* (Mites), e.g. *Penthaleus major, Tetranychus* spp.;
Nematodes, e.g. *Heterodera glycines, Meloidogyne* spp., *Pratylenchus* spp., *Caenorhabditis elegans.*

### Animal health

The compounds I are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound (I).

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound (I).

The compounds of the invention are further suitable for use in combating or controlling parasites in and on animals. Furthermore, the invention relates to a method of combating or controlling parasites in and on animals, which comprises contacting the parasites with a parasitically effective amount of a compound (I).

The invention also relates to the non-therapeutic use of compounds I for controlling or combating parasites. Moreover, the invention relates to a non-therapeutic method of combating or controlling parasites, which comprises applying to a locus a parasiticidally effective amount of a compound (I).

The compounds I can be effective through both contact (via soil, glass, wall, bed net, carpet, blankets or animal parts) and ingestion (e.g. baits). Furthermore, the compounds I can be applied to any and all developmental stages.

The compounds I can be applied as such or in form of compositions comprising them.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating the following parasites: *Cimex lectularius, Rhipicephalus sanguineus,* and *Ctenocephalides felis.*

As used herein, the term "animal" includes warm-blooded animals (including humans) and fish. Preferred are mammals, such as cattle, sheep, swine, camels, deer, horses, pigs, poultry, rabbits, goats, dogs and cats, water buffalo, donkeys, fallow deer and reindeer, and also in furbearing animals such as mink, chinchilla and raccoon, birds such as hens, geese, turkeys and ducks and fish such as fresh- and salt-water fish such as trout, carp and eels. Particularly preferred are domestic animals, such as dogs or cats.

The compounds I may be applied in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. For oral administration, the dosage form chosen should provide the animal with 0.01 mg/kg to 100 mg/kg of animal body weight per day of the compounds I, preferably with 0.5 mg/kg to 100 mg/kg of animal body weight per day. Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Oral solutions are administered directly.

Solutions for use on the skin are trickled on, spread on, rubbed in, sprinkled on or sprayed on.

Gels are applied to or spread on the skin or introduced into body cavities.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures.

Emulsions can be administered orally, dermally or as injections.

Suspensions can be administered orally or topically/dermally.

Semi-solid preparations can be administered orally or topically/dermally.

For the production of solid preparations, the active compound is mixed with suitable excipients, if appropriate with addition of auxiliaries, and brought into the desired form.

The compositions which can be used in the invention can comprise generally from about 0.001 to 95% of the compound I.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula (I) against endoparasites in concentrations of 10 ppm to 2% by weight, preferably of 0.05 to 0.9% by weight, very particularly preferably of 0.005 to 0.25% by weight.

Solid formulations which release compounds of the invention may be applied in total amounts of 10 mg/kg to 300 mg/kg, preferably 20 mg/kg to 200 mg/kg, most preferably 25 mg/kg to 160 mg/kg body weight of the treated animal in the course of three weeks.

The following examples illustrate the invention.

### A. Preparation of Compounds

Materials: Unless otherwise noted, reagents and solvents were purchased at highest commercial quality and used without further purification. Dry tetrahydrofuran (THF), ethylacetate (EtOAc), dimethylsulfoxide (DMSO), acetone, ethanol (EtOH), benzene, dimethylformamide (DMF), diisopropylethylamine (DIPEA), hexafluorophosphate azabenzotriazole tetramethyl uronium (HATU), pyridine, and CH₂Cl₂ were purchased from commercial providers.

All reactions were monitored by thin-layer chromatography (TLC) using Merck silica gel 60 F₂54 pre-coated plates (0.25 mm). Flash chromatography was carried out with Kanto Chemical silica gel (Kanto Chemical, silica gel 60N, spherical neutral, 0.040-0.050 mm, Cat.-No. 37563-84). If not otherwise indicated, ¹H NMR spectra were recorded on JEOL JNM-ECA-500 (500 MHz). Chemical shifts are expressed in ppm downfield from the internal solvent peaks for acetone-d₆ (¹H; δ = 2.05 ppm) and CD₃OD (¹H; δ = 3.30 ppm), and *J* values are given in Hertz. The following abbreviations were used to explain the multiplicities: s = singlet, d = doublet, t = triplet, q = quartet, dd = double doublet, dt = double triplet, m = multiplet, br = broad. High-resolution mass spectra were measured on a JEOL JMS-T100LP.

Characterization: The compounds were characterized by coupled High Performance Liquid Chromatography with mass spectrometry (HPLC/MS).

Method A: UHPLC-MS on Shimadzu Nexera UHPLC & Shimadzu LCMS 20-20 ESI. Analytical UHPLC column: Phenomenex Kinetex 1,7 µm XB-C18 100A; 50 x 2.1 mm; mobile phase: A: water + 0.1% TFA; B: acetonitrile; gradient: 5-100% B in 1.50 minutes; 100% B 0.20 min; flow: 0,8-1,0mL/min in 1,50 minutes at 60°C. MS-method: ESI positive; mass range (m/z) 100-700. Method B: Agilent 1260 HPLC MSD:6125B single quadrupole MSD Column: Luna C18,2.0*50mm, 5µm Column Temp: 40 Mobile Phase:A:0.04%TFA in H2O Mobile Phase:B:0.02%TFA in ACN Flow Rate: 1ml/min

Method C: Shimadzu LC-20AD. Analytical UHPLC column: C-18, 30 mm, 2.1 mm, 5 micron; mobile phase: A: 0.04% TFA in Water. B: 0.02 % TFA in Acetonitrile. Flow Rate: 1.5 mL/min, Injection Vol: 0.5 µL; Gradient: 5%B to 95 %B in 1.16 min,5% B for 34 sec . Run time: 1.5 min at 40°C.

Method D: Shimadzu LC-20AB. Analytical UHPLC column: C-18, 50 mm, 2.1 mm, 5 micron; mobile phase: A: 0.04% TFA in Water. B: 0.02 % TFA in Acetonitrile. Flow Rate: 1.2 mL/min, Injection Vol: 0.3 µL; Gradient: 10%B to 80 %B in 4 min,10% B for 30 sec . Run time: 4.5 min at 40°C.

### Synthesis Example 1: preparation of 8-(5-bromo-3-(ethylsulfonyl)pyridin-2-yl)-9-methyl-5-(trifluoromethyl)-9H-imidazo[4,5-c][1,2,4]triazolo[1,5-a]pyridine (compound of formula (I.2).

### Part A: preparation of N⁸-methyl-5-(trifluoromethyl)-[1,2,4]triazolo[1,5-a]pyridine-7,8-diamine

### Step 1: preparation of 3-nitro-6-(trifluoromethyl)pyridin-2-amine:

A composition containing 2-chloro-3-nitro-6-(trifluoromethyl)pyridine (43.3 mmol), an aqueous solution of NH₃ (30 % in H₂O, 13 mL) and CH₃CN (40 mL) was stirred at 80 °C for 16 hours. The reaction mixture was then cooled down to 20 to 25 °C and concentrated under reduced pressure. The resulting residue was purified by flash column chromatography to afford give 3-nitro-6-(trifluoromethyl)pyridin-2-amine (8.34 g). ¹H-NMR (400 MHz, CDCl₃): *δ* 8.60 (d, *J* = 8.6 Hz, 1H), 7.09 (d, *J =* 8.6 Hz, 1H).

### Step 2: preparation of N'-hydroxy-N-(3-nitro-6-(trifluoromethyl)pyridin-2-yl)formimidamide:

A solution of 3-nitro-6-(trifluoromethyl)pyridin-2-amine (40.2 mmol) and dimethylformamidime-thylacetal (52.3 mmol) in (CH₃)₂CHOH (200 mL) was heated to 80 °C and stirred for 4 hours. The reaction mixture was then cooled to 20 to 25 °C, upon which hydroxylamine hydrochloride (52.3 mmol) was added. After the resulting reaction mixture was stirred at 80 °C for 4 hours, the mixture was cooled to 20 to 25 °C. The reaction was then quenched with H₂O and the resulting composition was extracted. The combined organic layers were dried, filtered and concentrated under reduced pressure ito afford *N*'-hydroxy-*N*-(3-nitro-6-(trifluoromethyl)pyridin-2-yl)formimidamide (9.85 g). ¹H-NMR (400 MHz, DMSO-*d*₆): *δ* 11.56 (s, 1H), 10.45 (d, *J* = 9.2 Hz, 1H), 8.83 (d, *J* = 8.4 Hz, 1H), 8.06 (d, *J* = 9.2 Hz, 1H), 7.57 (d, *J* = 8.4 Hz, 1H).

### Step 3: preparation of 8-nitro-5-(trifluoromethyl)-[1,2,4]triazolo[1,5-a]pyridine:

A solution of *N*'-hydroxy-*N*-(3-nitro-6-(trifluoromethyl)pyridin-2-yl)formimidamide (39.3 mmol) and polyphosphoric acid (268.0 mmol) in 1,4-dioxane (150 mL) was stirred at 100 °C for 16 hours. The reaction mixture was then cooled to 20 to 25 °C and concentrated to remove the contained 1,4-dioxane. The resulting residue was treated with H₂O, and it the pH value was adjusted to 9 at a temperature of °C. The resulting composition was extracted and the combined organic layers were dried, filtered and concentrated under reduced pressure. The resulting residue was purified by flash column chromatography to afford 8-nitro-5-(trifluoromethyl)-[1,2,4]triazolo[1,5-a]pyridine (5.93 g). ¹H-NMR (400 MHz, CDCl₃): *δ* 8.71 (s, 1H), 8.60 (d, *J* = 8.2 Hz, 1H), 7.68 (d, *J* = 8.2 Hz, 1H). LCMS (ESI) *m*/*z* 233.0 [M + H]⁺.

### Step 4: preparation of 5-(trifluoromethyl)-[1,2,4]triazolo[1,5-a]pyridin-8-amine:

A solution of 8-nitro-5-(trifluoromethyl)-[1,2,4]triazolo[1,5-a]pyridine (25.5 mmol) in CH₃CH₂OH (100 mL) was added to a solution of Pd/C (593 mg, 10 % w/w) in CH₃CH₂OH (20 mL). The resulting reaction mixture was stirred at 20 to 25 °C under H₂ (1 atm) for 16 hours. The reaction mixture was then filtered through a pad of celite, and the filtrate was concentrated under reduced pressure to afford crude 5-(trifluoromethyl)-[1,2,4]triazolo[1,5-a]pyridin-8-amine (5.01 g). ¹H-NMR (400 MHz, CDCl₃): δ 8.35 (s, 1H), 7.28 (d, *J =* 8.0 Hz, 1H), 6.59 (d, *J =* 8.0 Hz, 1H), 5.00 (Br s, 2H).

### Step 5: preparation of N-(5-(trifluoromethyl)-[1,2,4]triazolo[1,5-a]pyridin-8-yl)acetamide:

To a solution of 5-(trifluoromethyl)-[1,2,4]triazolo[1,5-a]pyridin-8-amine (7.32 mmol) in 1,2-dichloroethane (50 mL) was added acetyl chloride (36.6 mmol) and pyridine (36.6 mmol) at 0 °C. The resulting reaction mixture was stirred at 20 to 25 °C for 2 hours. The reaction mixture was quenched with H₂O and the resulting composition was extracted. The combined organic layers were dried and concentrated under reduced pressure. The residue was purified by flash column chromatography to afford *N*-(5-(trifluoromethyl)-[1,2,4]triazolo[1,5-a]pyridin-8-yl)acetamide (1.54 g, 70 % yield). ¹H-NMR (400 MHz, CDCl₃): *δ* 8.55 (d, *J* = 8.2 Hz, 1H), 8.51 (br s, 1H), 8.39 (s, 1H), 7.46 (d, *J =* 8.2 Hz, 1H), 2.35 (s, 3H). LCMS (ESI) *m*/*z* found, 245.1 [M + H]⁺.

### Step 6: preparation of N-methyl-N-(5-(trifluoromethyl)-[1,2,4]triazolo[1,5-a]pyridin-8-yl)acetamide:

To a solution of *N*-(5-(trifluoromethyl)-[1,2,4]triazolo[1,5-a]pyridin-8-yl)acetamide (30.3 mmol) in DMF (80 mL) was added NaH (60 % in mineral oil, 1.45 g, 60.7 mmol) at 0 °C. After the resulting composition was stirred at 0 °C for 1 hours, CH₃I (46 mmol) was added. The resulting reaction mixture was stirred at 20 to 25 °C for another 1 hours. The reaction mixture was then quenched by H₂O and the resulting composition was extracted. The combined organic layers were dried and concentrated under reduced pressure to afford a residue. The residue was purified by flash column chromatography to afford *N*-methyl-*N*-(5-(trifluoromethyl)-[1,2,4]triazolo[1,5-a]pyridin-8-yl)acetamide (5.53 g). ¹H-NMR (400 MHz, CDCl₃): *δ* 8.50 (s, 1H), 7.55-7.50 (m, 2H), 3.48 (s, 3H), 2.10 (s, 3H). LCMS (ESI) *m*/*z* found, 259.1 [M + H]⁺.

### Step 7: preparation of N-methyl-5-(trifluoromethyl)-[1,2,4]triazolo[1,5-a]pyridin-8-amine:

To a solution of *N*-methyl-*N*-(5-(trifluoromethyl)-[1,2,4]triazolo[1,5-a]pyridin-8-yl)acetamide (21.4 mmol) in CH₃OH (100 mL) was added HCl (6 N aqueous, 60 mL) at 20 to 25 °C. After the resulting reaction mixture had been stirred at 50 °C for 16 hours, the reaction mixture was cooled to 20 to 25 °C. The reaction mixture was then concentrated under reduced pressure, and the residue was treated with H₂O, and the resulting composition was extracted. The combined organic layers were dried, filtered, and concentrated under reduced pressure to afford crude N-methyl-5-(trifluoromethyl)-[1,2,4]triazolo[1,5-a]pyridin-8-amine (4.24 g). LCMS (ESI) *m*/*z* found, 217.1 [M + H]⁺.

Step 8: preparation of *N*-methyl-7-nitro-5-(trifluoromethyl)-[1,2,4]triazolo[1,5-*a*]pyridin-8-amine:

To a solution of *N*-methyl-5-(trifluoromethyl)-[1,2,4]triazolo[1,5-a]pyridin-8-amine (19.6 mmol) in sulfuric acid (30 mL) was added nitric acid (19.6 mmol) at 0 °C. After the resulting reaction mixture had been stirred at 0 °C for 30 minutes, the reaction mixture was poured into ice water. The resulting composition was treated with H₂O and extracted. The combined organic layers were dried and concentrated under reduced pressure to afford a residue. The residue was purified by flash column chromatography to afford N-methyl-7-nitro-5-(trifluoromethyl)-[1,2,4]triazolo[1,5-a]pyridin-8-amine (4.69 g). ¹H-NMR (400 MHz, CDCl₃): *δ* 8.69 (s, 1H), 8.18 (s, 1H), 3.95 (s, 3H).

### Step 9: preparation of N⁸-methyl-5-(trifluoromethyl)-[1,2,4]triazolo[1,5-a]pyridine-7,8-diamine:

To a solution of *N*-methyl-7-nitro-5-(trifluoromethyl)-[1,2,4]triazolo[1,5-a]pyridin-8-amine (17.9 mmol) in CH₃CH₂OH (100 mL) was added SnCl₂·2H₂O (71.8 mmol) at 20 to 25 °C. After the resulting reaction mixture was stirred at 80 °C for 2 hours, the reaction was quenched with an aqueous saturated solution of NaHCO₃. The resulting composition was filtered by a pad of Celite, and the filtrate was extracted. The combined organic layers were dried and concentrated under reduced pressure to afford a residue. The residue was purified by flash column chromatography to afford *N*⁸-methyl-5-(trifluoromethyl)-[1,2,4]triazolo[1,5-*a*]pyridine-7,8-diamine (1.66 g). ¹H-NMR (400 MHz, CDCl₃): *δ* 8.23 (s, 1H), 6.98 (s, 1H), 3.06 (s, 3H).

LCMS (ESI) *m*/*z* found, 232.1 [M + H]⁺.

### Part B: preparation of 5-bromo-3-(ethylsulfonyl)picolinic acid

### Step 1: preparation of methyl 5-bromo-3-chloropicolinate:

To a solution of 5-bromo-3-chloropicolinic acid (85.0 mmol) in CH₃OH (200 mL) was added thionyl chloride (102 mmol) dropwise at 20 to 25 °C. The resulting reaction mixture was stirred at 75 °C for 2.5 hours. The solvent was removed under reduced pressure to afford a residue. The residue was treated with CH₂CL₂ and and aqueous saturated solution of NaHCO₃. The resulting mixture was extracted and the combined organic layers were dried, filtered and concentrated under reduced pressure to afford methyl 5-bromo-3-chloropicolinate (20.9 g) which was used to next step without further purification. ¹H-NMR (400 MHz, CDCl₃): *δ* 8.63 (d, *J* = 1.8 Hz, 1H), 8.01 (d, *J =* 1.8 Hz, 1H), 4.01 (s, 3H).

### Step 2: methyl 5-bromo-3-(ethylthio)picolinate::

To a solution of methyl 5-bromo-3-chloropicolinate (59.9 mmol) in THF (260 mL) was added sodium ethanethiolate (59.9 mmol) at 0 °C, and the resulting reaction mixture was stirred at 0 °C for 1 hour. Subsequently, the the reaction mixture was stirred at 20 to 25 °C for 16 hours. Then, the reaction mixture was concentrated under reduced pressure to afford a residue, which was treated with H₂O and extracted. The combined organic layers were dried, filtered and concentrated under reduced pressure. The resulting residue was purified by silica gel column chromatography to afford methyl 5-bromo-3-(ethylthio)picolinate (2.58 g). ¹H-NMR (400 MHz, CDCl₃): *δ* 8.46 (s, 1H), 7.78 (s, 1H), 3.99 (s, 3H), 2.94 (q, *J =* 7.4 Hz, 2H), 1.41 (t, *J =* 7.4 Hz, 3H). LCMS (ESI) *m*/*z* 276.0 [M + H] ⁺ and 298.0 [M + Na] ⁺.

### Step 3: methyl 5-bromo-3-(ethylsulfonyl)picolinate:

To a solution of methyl 5-bromo-3-(ethylthio)picolinate (7.31 mmol) in CH₂Cl₂ (50 mL) was added 3-chloroperbenzoic acid (16.8 mmol) at 0 °C. After stirring the resultin reaction mixture at 0 °C for 1 hour, the reaction mixture was quenched, washed, and extracted. The combined organic layer was dried, filtered and concentrated under reduced pressure to afford a residue. The residue was purified by silica gel column chromatography to afford methyl 5-bromo-3-(ethylsulfonyl)picolinate (2.61 g). ¹H-NMR (400 MHz, CDCl₃): *δ* 8.90 (d, *J* = 2.0 Hz, 1H), 8.51 (d, *J* = 2.0 Hz, 1H), 4.04 (s, 3H), 3.57 (q, *J =* 7.6 Hz, 2H), 1.37 (t, *J =* 7.6 Hz, 3H).

### Step 4: 5-bromo-3-(ethylsulfonyl)picolinic acid:

To a solution of methyl 5-bromo-3-(ethylsulfonyl)picolinate (8.47 mmol) in THF/H₂O (80 mL, 3:1 (v/v)) was added LiOH (12.7 mmol) at 0 °C and the resulting reaction mixture was stirred at 20 to 25 °C for 1 hour. The pH of the reaction mixture was then adjusted with Dowex H⁺ (pH = 6), which reaction mixture was then filtered, and concentrated under reduced pressure to afford 5-bromo-3-(ethylsulfonyl)picolinic acid (2.63g), which was used to next step without further purification. ¹H-NMR (400 MHz, DMSO-*d*₆): *δ* 8.78 (d, *J* = 2.4 Hz, 1H), 8.18 (d, *J =* 2.4 Hz, 1H), 3.75 (q, *J* = 7.6 Hz, 2H), 1.08 (t, *J =* 7.6 Hz, 3H).

### Part C: Synthesis of 8-(5-bromo-3-(ethylsulfonyl)pyridin-2-yl)-9-methyl-5-(trifluoromethyl)-9H-imidazo[4,5-c][1,2,4]triazolo[1,5-a]pyridine (compound of formula (I.2)

### Step 1: 5-bromo-3-(ethylsulfonyl)-N-(8-(methylamino)-5-(trifluoromethyl)-[1,2,4]triazolo[1,5-a]pyridin-7-yl)picolinamide:

A solution of 5-bromo-3-(ethylsulfonyl)picolinic acid (3.24 mmol) in thionyl chloride (8 mL) was heated to 80 °C for 2 hours. The reaction mixture was then concentrated in vacue to remove residual thionyl chloride. A solution of *N*⁸-methyl-5-(trifluoromethyl)-[1,2,4]triazolo[1,5-*a*]pyridine-7,8-diamine (2.16 mmol) and (CH₃CH₂)₃N (3.2 mmol) in CH₂Cl₂ (10 mL) was added to the reaction mixture at 0 °C. After the resulting reaction mixture was stirred at 20 to 25 °C for 1 hour, the reaction was quenched with H₂O and extracted. The combined organic layers were dried, filtered and concentrated under reduced pressure to afford crude 5-bromo-3-(ethylsulfonyl)-*N*-(8-(methylamino)-5-(trifluoromethyl)-[1,2,4]triazolo[1,5-a]pyridin-7-yl)picolinamide (1.3 g), which was used in the next step without purification.

### Step 2: 8-(5-bromo-3-(ethylsulfonyl)pyridin-2-yl)-9-methyl-5-(trifluoromethyl)-9H-imidazo[4,5-c][1,2,4]triazolo[1,5-a]pyridine:

A solution of 5-bromo-3-(ethylsulfonyl)-*N*-(8-(methylamino)-5-(trifluoromethyl)-[1,2,4]triazolo[1,5-a]pyridin-7-yl)picolinamide (2.56 mmol) in CH₃COOH (4 mL) was stirred at 110 °C for 16 hours. After the reaction mixture was cooled to 20 to 25 °C, it was treated with H₂O and extracted. The combined organic layers were dried, filtered and concentrated under reduced pressure. The resulting residue was purified by flash column chromatography. The product was washed with CH₃OH to afford 8-(5-bromo-3-(ethylsulfonyl)pyridin-2-yl)-9-methyl-5-(trifluoromethyl)-9H-imidazo[4,5-c][1,2,4]triazolo[1,5-a]pyridine (549 mg). ¹H-NMR (400 MHz, CDCl₃): *δ* 9.06 (s, 1H), 8.68 (s, 1H), 8.48 (s, 1H), 7.88 (s, 1H), 4.29 (s, 3H), 3.88 (q, *J* = 7.6 Hz, 2H), 1.41 (t, *J* = 7.6 Hz, 3H). LCMS (ESI) *m*/*z* found, 489.0 [M + H]⁺; HPLC purity: 99.53 %, *t*_{R} (retention time) = 21.13 min.

### Synthesis Example 2: preparation of 11-(4-bromo-2-ethylsulfonyl-phenyl)-12-methyl-7-(trifluoromethyl)-3,5,6,10,12-pentazatricyclo[7.3.0.02,6]dodeca-1(9),2,4,7,10-pentaene

### Step-1: preparation of 3-bromo-6-(trifluoromethyl)pyridin-2-amine (compound I.8)

To a solution of 6-(trifluoromethyl)pyridin-2-amine (333 g) in CH₂Cl₂ (2.30 L) was added Br₂ (344 g). The resulting reaction mixture was stirred at 0-25 °C for 2 hours. The reaction mixture was poured into water. Then the aqueous layer was washed with a saturated aqueous solution of NaHCO₃. The organic layer was dried, and concentrated under reduced pressure. The resulting residue was purified by column chromatography to afford a crude product. The crude product was triturated with n-hexane : 2-methoxy-2-methylpropane = 10:1 (500 mL) at 5 °C for 1 hour to afford 3-bromo-6-(trifluoromethyl)pyridin-2-amine (708 g) as a white solid. ¹H-NMR: (400 MHz, CDCl₃) *δ* 7.78-7.96 (m, 1H), 6.88-6.91 (m, 1H), 5.26-5.48 (s, 2H).

### Step-2: Preparation of N-[3-bromo-6-(trifluoromethyl)-2-pyridyl]-N'-hydroxy-formamidine

To a solution of 3-bromo-6-(trifluoromethyl)pyridin-2-amine (354 g) in (CH₃)₂CHOH (2.40 L) was added dimethylformamide-dimethylacetal (350 g) at 25 °C. The resulting mixture was stirred at 85 °C for 3 hours. The mixture was then cooled to 50 °C, upon which hydroxylamine; hydrochloride (204 g) was added. Subsequently, the mixture was stirred at 90 °C for 9 hrours. The solvent was then evaporated under reduced pressure to afford a residue, to which water (2.00 L) was added. The resulting mixture was extracted and the combined organic phaseses dried. The solvent was evaporated under reduced pressure to afford N-[3-bromo-6-(trifluoromethyl)-2-pyridyl]-N'-hydroxy-formamidine (640 g) as a white solid. ¹H-NMR: (400 MHz, CDCl₃) *δ* 8.43-8.46 (d, *J* = 12 Hz, 1H), 8.16-8.18 (d, *J* = 8.0 Hz, 1H), 7.95-7.96 (d, *J =* 4.0 Hz, 1H), 7.14-7.16 (d, *J =* 8.0 Hz, 2H).

### Step-3: preparation of 8-bromo-5-(trifluoromethyl)-[1,2,4]triazolo[1,5-a]pyridine

A mixture of N-[3-bromo-6-(trifluoromethyl)-2-pyridyl]-N'-hydroxy-formamidine (320 g) and polyphosphoric acid (761 g) in dioxane (2.24 L) was stirred at 100 °C for 12 hours. The mixture was then poured into ice water (1.50 L), then the mixture was adjusted to pH = 8-9 with saturated aqueous solution of NaOH. Then, the resulting mixture was extracted, and the combined organic phases were washed, dried, filtered and concentrated to afford a crude product. The crude product was triturated with n-heptane (1.50 L) at 25 °C for 1 hour to afford 8-bromo-5-(trifluoromethyl)-[1,2,4]triazolo[1,5-a]pyridine (490 g) as a brown solid. ¹H-NMR:(400 MHz, CDCl₃) *δ* 8.51 (s, 1H), 7.87-7.89 (d, *J =* 8.0 Hz, 1H), 7.36-7.38 (d, *J =* 8.0 Hz, 1H).

### Step-4: Preparation of tert-butyl N-methyl-N-[5-(trifluoromethyl)-[1,2,4]triazolo[1,5-a]pyridin-8-yl]carbamate

To a solution of 8-bromo-5-(trifluoromethyl)-[1,2,4]triazolo[1,5-a]pyridine (245 g), tert-butyl N-methylcarbamate (181 g), and Cs₂CO₃ (450 g) in dioxane (1.70 L) was added (5-diphenylphosphanyl-9,9-dimethyl-xanthen-4-yl)-diphenyl-phosphane (53.2 g) and (1E,4E)-1,5-diphenylpenta-1,4-dien-3-one;palladium (42.1 g,) at 25 °C. The resulting reaction mixture was stirred at 100 °C for 12 hours under N₂. The reaction mixture was then filtered, and the filter cake was washed with EtOAc. The filtrate was concentrated under reduced pressure to afford a residue. The residue was purified by column chromatography to give tert-butyl N-methyl-N-[5-(trifluoromethyl)-[1,2,4]triazolo[1,5-a]pyridin-8-yl]carbamate (380 g) as a yellow solid. ¹H-NMR: (400 MHz, DMSO) *δ* 8.70 (s, 1H), 7.82-7.83 (d, *J* = 4.0 Hz, 1H), 7.75-7.77 (d, *J =* 4.0 Hz, 1H), 3.33 (s, 3H), 1.33 (s, 9H).

### Step-5: preparation of N-methyl-5-(trifluoromethyl)-[1,2,4]triazolo[1,5-a]pyridin-8-amine

A mixture of tert-butyl N-methyl-N-[5-(trifluoromethyl)-[1,2,4]triazolo[1,5-a]pyridin-8-yl]carbamate (224 g) and CF₃COOH (808 g) in CH₂Cl₂ (1.61 L) was stirred at 25 °C for 12 hours. The mixture was then poured into water (1.00 L), and the separated organic layer was further washed with water (1.00 L x 2). Then, the organic phase was washed with saturated aqueous solution of NaHCO₃ (about 1.00 L). The combined organic phases were washed with brine, dried, filtered and concentrated to N-methyl-5-(trifluoromethyl)-[1,2,4]triazolo[1,5-a]pyridin-8-amine (133 g, 84.6% yield, 97.4% purity) as a yellow solid. ¹H NMR: (400 MHz, DMSO) *δ* 8.52 (s, 1H), 7.52-7.54 (d, *J* = 8.0 Hz, 1H), 7.35-7.36 (d, *J* = 4.0 Hz, 1H), 6.37-6.39 (d, *J* = 8.0 Hz, 1H), 2.89-2.91 (d, *J* = 8.0 Hz, 3H).

### Step-6: Preparation of N-methyl-7-nitro-5-(trifluoromethyl)-[1,2,4]triazolo[1,5-a]pyridin-8-amine

A composition was prepared comprising N-methyl-5-(trifluoromethyl)-[1,2,4]triazolo[1,5-a]pyridin-8-amine (47.0 g) in dichloroethane (470 mL), to which was added H₂SO₄ (127 g) at 20 °C. Then, HNO₃ (42.8 g, 68% purity) was added to the composition at 0-5 °C, and the resulting reaction mixture was stirred at 0 °C for 1 hour. Then, HNO₃ (6.00 g, 68% purity) was added to the reaction mixture at 0 °C, which was subsequently stirred at 0 °C for 1 hour. Two parallel reactions were combined for work-up. The reaction mixture was poured into 1.50 kg iced water (1.00 kg ice + 500 g water) with CH₂Cl₂ (300 mL). The resulting mixture was allowed to warm to 25 °C with stirring. The aqueous phase was exctraced, the organic phases combined and concentrated to afford a crude product. The crude product was triturated with 2-methoxy-2-methylpropan / n-heptane = 1/1 (300 mL) at 25 °C for 2 hours to give N-methyl-7-nitro-5-(trifluoromethyl)-[1,2,4]triazolo[1,5-a]pyridin-8-amine (75.7 g) as a yellow solid. LC-MS: mass calculated for C₈H₇F₃N₅O₂ [M]⁺ 262, found 263

### Step-7: Preparation of N8-methyl-5-(trifluoromethyl)-[1,2,4]triazolo[1,5-a]pyridine-7,8-diamine

To a solution of N-methyl-7-nitro-5-(trifluoromethyl)-[1,2,4]triazolo[1,5-a]pyridin-8-amine (55.0 g, 210 mmol) in EtOH (385 mL) was added SnCl₂•2H₂O (166 g, 737 mmol) and HCl (12 M, 165 mL) at 25 °C. Then the mixture was stirred at 25-80 °C for 12 hrs. HPLC showed starting material was consumed completely. The reaction was quenched by addition of ice water (1.00 L), then the mixture was adjusted to pH = 9-10 with NaOH (solid) at 5 °C, and then the mixture was extracted with EtOAc (300 mL x 3). The combined organic layers were washed with H₂O (150 mL x 2), concentrated under reduced pressure to give a residue (31.0 g crude). The crude product was purified by prep-HPLC (column: Phenomenex luna C18 (250*70mm, 15 um); mobile phase: [water (TFA)-ACN]; B%: 5%-35%, 27 min) to give N8-methyl-5-(trifluoromethyl)-[1,2,4]triazolo[1,5-a]pyridine-7,8-diamine (17.1 g, 33.5% yield, 95.6% purity) as a pink solid. ¹H NMR: (400 MHz, CDCl₃) *δ* 8.21 (s, 1H), 6.97 (s, 1H), 7.35-7.36 (d, *J =* 4.0 Hz, 1H), 3.07 (s, 3H).

### Step -8: Preparation of 4-bromo-2-ethylsulfonyl-N-[8-(methylamino)-5-(trifluoromethyl)-[1,2,4]triazolo[1,5-a]pyridin-7-yl]benzamide

To a solution of N8-methyl-5-(trifluoromethyl)-[1,2,4]triazolo[1,5-a]pyridine-7,8-diamine (2.00 g) and diisopropylamine (1.79 g) in dimethylformamide (100 mL) was added 4-bromo-2-ethylsulfonyl-benzoic acid (2.53 g) (synthesized as described in WO 2016/023954, p.86) followed by HATU(4.27 g,11.25 mmol) in small portions. The resulting reaction mixture was stirred at 20 to 25 °C. for 24 hours. The reaction mixture was then poured into water (50.0 mL) and a brown solution was obtained, which was extracted with dichloromethane, then concentrated and purified with column chromatography to give 4-bromo-2-ethylsulfonyl-N-[8-(methylamino)-5-(trifluoromethyl)-[1,2,4]triazolo[1,5-a]pyridin-7-yl]benzamide (4.2 g, 96% yield). LC-MS: mass calculated for C₁₇H₁₅BrF₃N₅O₃S[M+2]⁺ 508, found 508 at 1.106 mins (Method B)

### Step -9: Preparation of 11-(4-bromo-2-ethylsulfonyl-phenyl)-12-methyl-7-(trifluoromethyl)-3,5,6,10,12-pentazatricyclo[7.3.0.^{2,6}]dodeca-1(9),2,4,7,10-pentaene (compound I.8)

A mixture of 4-bromo-2-ethylsulfonyl-N-[8-(methylamino)-5-(trifluoromethyl)-[1,2,4]triazolo[1,5-a]pyridin-7-yl]benzamide (2 g) in CH₃COOH (20 mL) was degassed and purged with N₂ for 3 times, and then the mixture was stirred at 110 °C for 12 hours under N₂ atmosphere. The reaction mixture was then poured into H₂O (50 mL). Formed crystalline solid was separated by suction filtration. The collected fiter cakewas purified by column chromatography to give compound I.8 (0.8 g, 41.4% yield, 98% purity) as an off-white solid. LC-MS: mass calculated for C₁₇H₁₃BrF₃N₅O₂S [M+1]⁺ 489, found 489 at 1.093 mins with Method B

Accordingly, by the above Synthesis Examples 1-2, or by analogous procedures to the procedure described above, the following examples of formula I-A.1 were prepared, 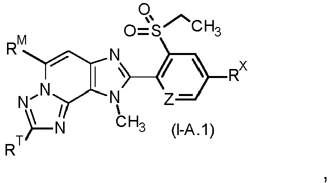 wherein wherein the variables R^{M}, R^{T}, R^{X}, and Z have a meaning as defined in Table E:

**Table E: Compounds of formula ()).1 to I.19 with their physical characterization. *retention time in minutes, mass charge ratio m/z; n.m. means not measured; (a) is HPLC Method A; (c) is HPLC Method C**

| Compound | R^{M} | R^{T} | R^{X} | Z | Phys. Chem. Data*: HPLC Retention time [min]; m/z | Chemical shift in ¹H-NMR (δ); Solvent CDCl₃ |
|---|---|---|---|---|---|---|
| I.1 | CF₃ | H | H | N | 17.32, 411.1^{(a)} | 9.02(d, *J* = 4.8 Hz, 1H), 8.55 (d, *J* = 8.0 Hz, 1H), 8.48 (s, 1H), 7.89 (s, 1H), 7.75 (dd, *J* = 8.0, 4.8 Hz, 1H), 4.27 (s, 3H), 3.81 (q, *J =* 7.6 Hz, 2H), 1.38 (t, *J =* 7.6 Hz, 3H) |
| I.2 | CF₃ | H | Br | N | 21.13, 489.0^{(a)} | 9.06 (s, 1H), 8.68 (s, 1H), 8.48 (s, 1H), 7.88 (s, 1H), 4.29 (s, 3H), 3.88 (q, *J* = 7.6 Hz, 2H), 1.41 (t, *J* = 7.6 Hz, 3H) |
| I.3 | CF₃ | H | CF₃ | N | 22.27, 479.1^{(a)} | 9.26 (s, 1H), 8.79 (s, 1H), 8.50 (s, 1H), 7.90 (s, 1H), 4.34 (s, 3H), 3.95 (q, *J* = 7.2 Hz, 2H), 1.43 (t, *J* = 7.2 Hz, 3H) |
| I.4 | CF₃ | H | 4-fluorophenyl | N | n.m. | 9.18 (s, 1H), 8.66 (s, 1H), 8.49 (s, 1H), 7.90 (s, 1H), 7.74 -7.71 (m, 2H), 7.29 (t, *J* = 8.4 Hz, 2H), 4.32 (s, 3H), 3.88 (q, *J* = 7.6 Hz, 2H), 1.41 (t, *J* = 7.6 Hz, 3H). |
| I.5 | CF₃ | H | 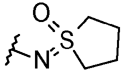 | N | 1.003; 528.3 (M+H)(a) | n.m. |
| I.6 | CF₃ | H | 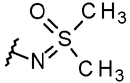 | N | 0.917; 501.9 (M+H) (a) | n.m. |
| I.7 | CF₃ | H | 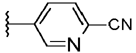 | N | 1.069; 513 (M+H) (a) | n.m. |
| I.8 | CF₃ | H | Br | CH | 1.093; 489^{(c)} (M+H) | (400 MHz, CDCl₃)δ 9.07-9.08 (d, J = 4.0 Hz, 1H), 8.68-8.69 (d, J = 4.0 Hz, 1H), 8.49 (s, 1H), 7.89 (s, 1H), 4.30 (s, 3H), 3.86-3.91 (m, 2H), 1.40-1.44 (m, 3H). |
| I.9 | CF₃ | H | 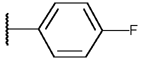 | CH | 1.214; 503.9 (M+)^{(a)} | n.m. |
| I.10 | CF₃ | H | 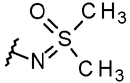 | CH | 0.942; 501.1 (M+H)^{(a)} | n.m. |
| I.11 | CF₃ | H | 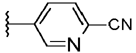 | CH | 1.099; 512.1 (M+)^{(a)} | n.m. |
| I.12 | CF₃ | H | 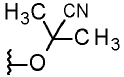 | N | 1.138; 493.4 (M+)^{(a)} | n.m. |
| I.13 | CF₃ | H | 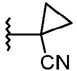 | N | 1.029; 475.2 (M+H) (a) | n.m. |
| I.14 | CF₃ | H | 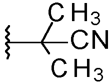 | N | 1.06; 477.9 (M+H) (a) | n.m. |
| I.15 | CF₃ | H | 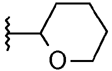 | N | 1.181; 495.2 (M+H) (a) | n.m. |
| I.16 | CF₃ | H | 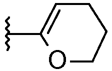 | N | 1.225; 493.2 (M+H) (a) | n.m. |
| I.17 | CF₃ | H | 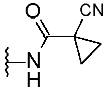 | N | 1.038; 519.0 (M+H) (a) | n.m. |
| I.18 | CF₃ | H | 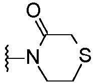 | N | 0.979; 526 (M+H) (a) | n.m. |
| I.19 | CF₃ | H | 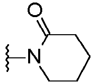 | N | 1.024; 508 (M+H) (a) | n.m. |

### Synthesis Example 3: Preparation of 11-(5-bromo-3-ethylsulfonyl-2-pyridyl)-7-(trifluoromethyl)-1,3,5,6,10-pentazatricyclo[7.3.0.0^{2,6}]dodeca-2,4,7,9,11-pentaene) (compound I.20)

### Step-1: preparation of 7-(trifluoromethyl)-4H-[1,2,4]triazolo[1,5-a]pyrimidin-5-one

A composition was prepared comprising 1H-1,2,4-triazol-3-amine (1 g) in dioxane (10 mL). Ethyl 4,4,4-trifluorobut-2-ynoate (2.37 g) was added to the composition, which was subsequently stirred at 110 °C for 2 hours. The reaction was then quenched with water, the aqueous layer was extracted with ethylacetate. The combined organic layers were washed, dried, and concentrated to afford a crud product. The crude product was purified by HPLC to give 7-(trifluoromethyl)-4H-[1,2,4]triazolo[1,5-a]pyrimidin-5-one (375 mg) as yellow solid.¹H-NMR: (400 MHz, DMSO-d₆) δ = 8.24 (s, 1H), 6.87 (s, 1H)

### Step-2: Preparation of 4-[2-(5-bromo-3-ethylsulfonyl-2-pyridyl)-2-oxo-ethyl]-7-(trifluoromethyl)-[1,2,4]triazolo[1,5-a]pyrimidin-5-one

A composition was prepared comprisingf 7-(trifluoromethyl)-4H-[1,2,4]triazolo[1,5-a]pyrimidin-5-one (3.1 g) in CH₃CN (150 mL). 2-bromo-1-(5-bromo-3-ethylsulfonyl-2-pyridyl)ethenone (6.2 g) (synthesized as described in WO2021204577, page 79) and K₂CO₃ (5.2 g) were added to the composition, which was subsequently stirred at 60 °C for 3 hours. The reaction was quenched with water, and the resulting composition extracted with EtOAc. The combined organic layers were washed, dried, and concentrated to afford a crude product. The crude product was purified by column to give 4-[2-(5-bromo-3-ethylsulfonyl-2-pyridyl)-2-oxo-ethyl]-7-(trifluoromethyl)-[1,2,4]triazolo[1,5-a]pyrimidin-5-one (3 g) as yellow solid. ¹H-NMR:(400 MHz, CDCl₃) δ = 9.01 (d, *J* = 2.1 Hz, 1H), 8.68 (d, *J =* 2.1 Hz, 1H), 8.03 (s, 1H), 6.74 (s, 1H), 5.87 (s, 2H), 3.60 (q, *J* = 7.6 Hz, 2H), 1.33 (t, *J* = 7.4 Hz, 3H).

### Step-3: Preparation of 11-(5-bromo-3-ethylsulfonyl-2-pyridyl)-7-(trifluoromethyl)-1,3,5,6,10-pentazatricyclo[7.3.0.0^{2,6}]dodeca-2,4,7,9,11-pentaene

To a solution of 4-[2-(5-bromo-3-ethylsulfonyl-2-pyridyl)-2-oxo-ethyl]-7-(trifluoromethyl)-[1,2,4]triazolo[1,5-a]pyrimidin-5-one (500 mg) in CH₃CH₂CH₂CH₂OH (2.5 mL) was added NH₄(CF₃COO) (1.3 g), CH₃COOH (600 mg) and a molecular sieve (4A-MS; 500 mg), and the resulting mixture was stirred at 145 °C for 8 hours. The reaction was quenched with water, and extracted with EtOAc. The combined organic layers were washed, dried, and concentrated to afford a crude product. The crude product was purified by column to give compound I.20 (60 mg) as yellow solid. ¹H-NMR: (400 MHz, CDCl₃) δ = 8.96 (d, *J* = 2.0 Hz, 1H), 8.72 (s, 1H), 8.69 (d, *J* = 2.0 Hz, 1H), 8.32 (s, 1H), 7.69 (s, 1H), 4.03 (q, *J =* 7.4 Hz, 2H), 1.43 (t, *J =* 7.4 Hz, 3H).

Accordingly, by the above Synthesis Example 3 or by analogous procedures to the procedure described above, the following examples of formula I-G.1 were prepared, 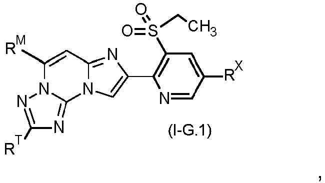 wherein wherein the variables R^{M}, R^{T}, R^{X}, and Z have a meaning as defined in Table F:

**Table F: Compounds of formula ()).1 to I.19 with their physical characterization. *retention time in minutes, mass charge ratio m/z; n.m. means not measured; (a) is HPLC Method A; (b) is HPLC Method B; (d) is HPLC Method D;**

| Compound | R^{M} | R^{T} | R^{X} | Phys. Chem. Data*: HPLC Retention time [min]; m/z | Chemical shift in ¹H-NMR (δ); Solvent CDCl₃ |
|---|---|---|---|---|---|
| I.20 | CF₃ | H | Br | n.m. | (400 MHz) δ = 8.96 (d, *J =* 2.0 Hz, 1H), 8.72 (s, 1H), 8.69 (d, *J =* 2.0 Hz, 1H), 8.32 (s, 1H), 7.69 (s, 1H), 4.03 (q, *J* = 7.4 Hz, 2H), 1.43 (t, *J =* 7.4 Hz, 3H). |
| I.21 | CF₃ | H | 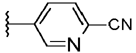 | 1.456; 499.1 (M+H)(a) | n.m. |
| 1.22 | CF₃ | H | 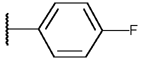 | 2.742; 491.1 (M+H) ^{(b)} | n.m. |
| I.23 | CF₃ | H | 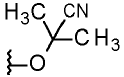 | 1.47; 480 (M+H) ^{(d)} | n.m. |

### Synthesis Example 4: preparation of [5-ethylsulfonyl-6-[7-(trifluoromethyl)-1,3,6,10-tetrazatricyclo[7.3.0.02,6]dodeca-2,4,7,9,11-pentaen-11-yl]-3-pyridyl]imino-dimethyl-oxo-λ6-sulfane (compound I.24)

### Step 1: Preparation of 1-benzyl-3-[(4-methoxyphenyl)methyl]-6-(trifluoromethyl)pyrimidine-2,4-dione

To the stirred solution of 3-[(4-methoxyphenyl)methyl]-6-(trifluoromethyl)-1H-pyrimidine-2,4-dione (20 g) (prepared as described in WO2018206479, page 65) and K₂CO₃ (18.41 g) in DMF (150 mL), chloromethylbenzene (12.64 g) was added dropwise at 0 °C. The resulting reaction mixture was heated to 95 °C for 12 hours. After completion of the reaction, the reaction mixture was cooled and diluted with ice water (300 mL). Formed precipitate was filtered through buck-ner funnel and dried over high vacuum to afford 1-benzyl-3-[(4-methoxyphenyl)methyl]-6-(trifluoromethyl)pyrimidine-2,4-dione as a white solid (20 g). ¹H-NMR (300 MHz, DMSO-d₆) δ 7.45 (d, *J* = 8 Hz, 2H), 7.1-7.4 (m, 5H), 6.85 (d, *J* = 8 Hz, 2H), 6.35 (s, 1H), 5.2 (s, 2H), 5.25 (s, 2H), 3.82 (s, 3H), 1.21 (t, *J* = 7.4 Hz, 3H).

### Step-2: Preparation of 1-benzyl-6-(trifluoromethyl)pyrimidine-2,4-dione

To a stirred solution of 1-benzyl-3-[(4-methoxyphenyl)methyl]-6-(trifluoromethyl)pyrimidine-2,4-dione (20 g) in 20% H₂O in CH₃CN (100 mL) was added ceric ammonium nitrate (35.11g). The resultant reaction mixture was stirred at 25°C for 12 hours. After the completion of the reaction, the reaction mixture was diluted with water, and extracted. The organic layers from the extraction were dried, and concentrated dryness under reduced pressure. The obtained crude product was purified by flash column chromatography to afford 1-benzyl-6-(trifluoromethyl)pyrimidine-2,4-dione as white solid (6g). ¹H-NMR (300 MHz, DMSO-d₆) δ 11.63(s,1H), 7.31 - 7.09 (m, 2H), 7.03 - 6.94 (m, 2H), 6.83 - 6.73 (m, 1H), 4.61 (s, 1H), 3.36 (d, *J =* 2.7 Hz, 2H).

### Step-3: Preparation of 4-amino-1-benzyl-6-(trifluoromethyl)pyrimidin-2-one

To a composition of 1-benzyl-6-(trifluoromethyl)-3H-pyrimidine-2,4-dione (2 g, 7.407 mmol) in CH₂Cl₂ (20 V) at 0 °C, pyridine (1.733 g) was added and stirred for 10 minutes. Then, trifluoromethanesulfonic anhydride (4.17 g) was added drop wise to the composition over a period of 20 minutes. The resultant reaction mixture was stirred at 20-25 °C for 4 hours. Subsequently, a solution of NH₃ in CH₃OH (7 N) was added to the reaction mixture, and the resulting mixture was continued to stir for another 4-5 hours. The reaction mixture was then partitioned between CH₂Cl₂ (250 mL x 2) and brine solution (100 mL), Organic layer was separated, dried and concentrated under reduced pressure to get a crude product. The crude product was purified by column chromatography to afford 4-amino-1-benzyl-6-(trifluoromethyl)pyrimidin-2-one as an off white solid (1.0 g, 50 % yield). ¹H-NMR (300 MHz, DMSO-d₆) δ 7.8 (s, 1H), 7.9 (s, 1H), 7.1-7.5 (m, 5H), 6.4(s, 1H), 5.1 (s, 2H). LC-MS: mass calculated C₁₂H₁₀F₃N₃O [M-H]¹⁻ 269, found 269.

### Step-4: Preparation of 6-benzyl-2-(5-bromo-3-ethylsulfonyl-2-pyridyl)-7-(trifluoromethyl)imidazo-[1,2-c]pyrimidin-5-one

To a stirred solution of 2-bromo-1-(4-bromo-2-ethylsulfonyl-phenyl)ethanone (1.64 g) in (CH₃)₃COH (2 V) was added 4-amino-1-benzyl-6-(trifluoromethyl)pyrimidin-2-one (1 g). A Molecular sieve was added to the obtained reaction mixture (0.4 g) and the resultant composition was heated to 120 °C for 24 hours. After the completion of the reaction, the composition was filtered through a celite bed, and celite bed was washed with EtOAc. The obtained filtrate was collected and concentrated under reduced pressure to get crude product, which was purified by flash column chromatography to afford 6-benzyl-2-(5-bromo-3-ethylsulfonyl-2-pyridyl)-7-(trifluoromethyl)imidazo[1,2-c]pyrimidin-5-one as a yellow solid (0.65 g). ¹H NMR (300 MHz, DMSO-d₆) δ 9.09 (d, *J =* 2.2 Hz, 1H), 8.54 (d, *J* = 2.2 Hz, 1H), 8.37 (s, 1H), 7.67 (s, 1H), 7.39 - 7.19 (m, 5H), 5.30 (s, 2H), 4.08 (q, *J =* 7.4 Hz, 2H), 1.25 (t, *J* = 7.4 Hz, 3H).

### Step 5: Preparation of 6-benzyl-2-[5-[[dimethyl(oxo)-λ6-sulfanylidene]amino]-3-ethylsulfonyl-2-pyridyl]-7-(trifluoromethyl)imidazo[1,2-c]pyrimidin-5-one

To the stirred solution of 6-benzyl-2-(5-bromo-3-ethylsulfonyl-2-pyridyl)-7-(trifluoromethyl)-imidazo[1,2-c]pyrimidin-5-one (0.5 g) in dioxane (5 mL) was added imino-dimethyl-oxo-λ6-sulfane (0.129 g) and Cs₂CO₃ (0.451 g)). The resulting composition stirred under continuous purging of N₂ for a period of 5 minutes. Under continued inert condition, tris-(dibenzyliden-aceton)-dipalladium(0) (0.042 g) was added to the composition, followed by Xantphos (0.053 g), and the resulting reaction mixture was stirred at 120 °C for a period of 3-4 hours. After completion of the reaction, the reaction mixture was cooled and filtered through a celite followed by washing with 5% CH₃OH in CH₂Cl₂. The obtained filtrate was concentrated to get a brown colored crude residue, which was purified by column chromatography to afford 6-benzyl-2-[5-[[dimethyl(oxo)-λ6-sulfanylidene]amino]-3-ethylsulfonyl-2-pyridyl]-7-(trifluoromethyl)imidazo[1,2-c]pyrimidin-5-one as a pale yellow solid (0.282 g , 52% yield). ¹H NMR (300 MHz, DMSO-d₆) δ 8.46 (d, *J* = 2.5 Hz, 1H), 8.18 (s, 1H), 7.90 (d, *J* = 2.5 Hz, 1H), 7.62 (s, 1H), 7.39 - 7.18 (m, 5H), 5.30 (s, 2H), 3.96 (q, *J =* 7.4 Hz, 2H), 3.37 (s, 6H), 1.21 (t, *J =* 7.4 Hz, 3H).

### Step-6: Preparation of 2-[5-[[dimethyl(oxo)-λ6-sulfanylidene]amino]-3-ethylsulfonyl-2-pyridyl]-7-(trifluoromethyl)-6H-imidazo[1,2-c]pyrimidin-5-one

A composition of 6-benzyl-2-[5-[[dimethyl(oxo)-λ6-sulfanylidene]amino]-3-ethylsulfonyl-2-pyridyl]-7-(trifluoromethyl)imidazo[1,2-c]pyrimidin-5-one (0.25g) in CH₃OH (3 mL) was produced. Ammonium formate (0.285 g) was then added to the composition, followed by 10% Pd/C (0.020 g, 0.0226 mmol) at 20 to 25 °C under inert conditions. The mixture was stirred at 25 °C for 12 hours. The reaction mixture was then filtered through a celite bed at 25 °C. The celite bed was subsequently washed with CH₃OH, and the filtrate containing the reaction mass was concentrated under reduced pressure to dryness, The obtained crude mass was purified by column chromatography to afford 2-[5-[[dimethyl(oxo)-λ6-sulfanylidene]amino]-3-ethylsulfonyl-2-pyridyl]-7-(trifluoromethyl)-6H-imidazo[1,2-c]pyrimidin-5-one as a pale yellow solid (0.190 g). ¹H NMR (500 MHz, DMSO-d₆) δ 12.92 (s, 1H), δ 8.48 (d, *J* = 2.5 Hz, 1H), 8.14 (s, 1H), 7.91 (d, *J* = 2.5 Hz, 1H), 7.35 (s, 1H), 3.94 (s, 2H), 3.40 (s, 6H), 1.19 (dt, *J* = 10.9, 7.2 Hz, 3H).

### Step-7: Preparation of 2-[5-[[dimethyl(oxo)-λ6-sulfanylidene]amino]-3-ethylsulfonyl-2-pyridyl]-7-(trifluoromethyl)imidazo[1,2-c]pyrimidin-5-amine

To a solution of 2-[5-[[dimethyl(oxo)-λ6-sulfanylidene]amino]-3-ethylsulfonyl-2-pyridyl]-7-(trifluoromethyl)-6H-imidazo[1,2-c]pyrimidin-5-one (0.05 g) in CH₂Cl₂ (2 mL) and pyridine (0.026 g) was added dropwise trifluoromethanesulfonic anhydride (0.061g) at 0°C. The resulting mixture was then stirred at 0 °C to 25 °C for 3 hours. A solution of NH₃ in CH₃OH (7 N) was added to the mixture for 10 minutes at 0 °C. The resulting mixture was then stirred at 25 °C for 12 hours, upon which the mixture was concentrated under reduced pressure. The remaining residue was purified by column chromatography to give 2-[5-[[dimethyl(oxo)-λ6-sulfanylidene]amino]-3-ethylsulfonyl-2-pyridyl]-7(trifluoromethyl)imidazo[1,2-c]pyrimidin-5-amine(0.03 g, 60 %) as yellow solid. ¹H NMR (300 MHz, DMSO-d₆) δ 8.44 - 8.34 (m, 2H), 8.19 (s, 2H), 7.85 (d, *J* = 2.5 Hz, 1H), 7.30 (s, 1H), 4.06 (q, *J =* 6.4, 5.3 Hz, 2H), 3.41(s, 6H), 1.21 - 1.14 (m, 3H). LC-MS: mass calculated C₁₆H₁₇F₃N₆O₃S₂[M+H]⁺ 463, found 463 at 1.851 mins ( method A).

### Step-8: Preparation of [5-ethylsulfonyl-6-[7-(trifluoromethyl)-1,3,6,10-tetrazatricyclo[7.3.0.02,6]dodeca-2,4,7,9,11-pentaen-11-yl]-3-pyridyl]imino-dimethyl-oxo-λ6-sulfane (compound I.24)

2-[5-[[dimethyl(oxo)-λ6-sulfanylidene]amino]-3-ethylsulfonyl-2-pyridyl]-7-(trifluoromethyl)-imidazo[1,2-c]pyrimidin-5-amine (0.1 g) was dissolved in CH₃CH₂OH (1 mL) in a 10 mL microwave vial with a magnetic stir bar. Then, a 50% solution of 2-chloroacetaldehyde in H₂O (0.034 g) and NaHCO₃ (0.036 g) were added to the above mixture. The obtained reaction mixture was heated to 120 °C for 2 hours in microwave. The reaction mixture was then concentrated in vacuo, and rhe residue was purified by flash chromatography to obtain [5-ethylsulfonyl-6-[7-(trifluoromethyl)-1,3,6,10-tetrazatricyclo[7.3.0.02,6]dodeca-2,4,7,9,11-pentaen-11-yl]-3-pyridyl]imino-dimethyl-oxo-λ6-sulfane. (0.06 g, 56 %). ¹H-NMR (300 MHz, DMSO-d₆) δ 8.56 - 8.47 (m, 2H), 8.05 (s, 1H), 7.91 (dd, *J* = 19.5, 2.2 Hz, 2H), 7.50 (d, *J =* 1.7 Hz, 1H), 4.02 (q, *J* = 7.4 Hz, 2H), 3.41 (s, 6H), 1.24 (t, *J* = 7.4 Hz, 3H).

### B. Biological Examples

The activity of the compounds of formula (I) of the present invention could be demonstrated and evaluated in biological tests described in the following. If not otherwise specified, the test solutions are prepared as follows: The active compound of formula ())s dissolved at the desired concentration in a mixture of 1:1 (vol:vol) distilled water : acteone. The test solution is prepared at the day of use. Test solutions are prepared in general at concentrations of 1000 ppm, 500 ppm, 300 ppm, 100 ppm and 30 ppm (wt/vol).

### Yellow fever mosquito (Aedes aegypti)

For evaluating control of yellow fever mosquito (*Aedes aegypti*) the test unit consisted of 96-well-microtiter plates containing 200µl of tap water per well and 5-15 freshly hatched *A. aegypti* larvae. The active compounds were formulated using a solution containing 75% (v/v) water and 25% (v/v) DMSO. Different concentrations of formulated compounds or mixtures were sprayed onto the insect diet at 2.5µl, using a custom built micro atomizer, at two replications.

After application, microtiter plates were incubated at 28 + 1°C, 80 + 5 % RH for 2 days. Larval mortality was then visually assessed. In this test, compounds of formula I..2, I.3, and I.4 at 10 ppm showed over 70% mortality in comparison with untreated controls. In this test, compounds of formula I.6, I.7, I.8, I.9, I.11, I.12, I.13, I.16, I.20, I.21, I.22 at 800 ppm showed over 70% mortality in comparison with untreated controls.

### Boll weevil (Anthonomus grandis)

For evaluating control of boll weevil (*Anthonomus grandis*) the test unit consisted of 96-well-microtiter plates containing an insect diet and 5-10 *A. grandis* eggs. The compounds were formulated using a solution containing 75% v/v water and 25% v/v DMSO. Different concentrations of formulated compounds were sprayed onto the insect diet at 5 µl, using a custom built micro atomizer, at two replications. After application, microtiter plates were incubated at about 25 ± 1°C and about 75 ± 5 % relative humidity for 5 days. Egg and larval mortality was then visually assessed. In this test, compounds of formula I.2 at 30 ppm showed over 70% mortality in comparison with untreated controls. In this test, compounds of formula I.3 and I.4 at 30 ppm showed over 70% mortality in comparison with untreated controls. In this test, compounds of formula I.6, I.7, I.8, I.9, I.10, I.11, I.12, I.13, I.14, I.20, I.21, I.22, I.24, at 800 ppm showed over 70% mortality in comparison with untreated controls.

### Tobacco budworm (Heliothis virescens)

For evaluating control of tobacco budworm (*Heliothis virescens*) the test unit consisted of 96-well-microtiter plates containing an insect diet and 15-25 *H. virescens* eggs.

The compounds were formulated using a solution containing 75% v/v water and 25% v/v DMSO. Different concentrations of formulated compounds were sprayed onto the insect diet at 10 µl, using a custom built micro atomizer, at two replications. After application, microtiter plates were incubated at about 28 ± 1°C and about 80 ± 5 % relative humidity for 5 days. Egg and larval mortality was then visually assessed. In this test, compounds of formula I.2, I.3, and I.4 at 10 ppm showed over 70% mortality in comparison with untreated controls. In this test, compounds of formula I.5, I.7, I.8, I.9, I.11, I.12, I.13, I.14, I.15, I.16, I.20, I.21, I.22 at 800 ppm showed over 70% mortality in comparison with untreated controls.

### Green Peach Aphid (Myzus persicae)

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 10or 20ml glass vials. A nonionic surfactant (Kinetic^{®}) was included in the solution at a volume of 0.01% (v/v). The vials were then inserted into an automated electrostatic sprayer equipped with an atomizing nozzle for application to plants/insects. Bell pepper plants at the first true-leaf stage were infested prior to treatment by placing heavily infested leaves from the main colony on top of the treatment plants. Aphids were allowed to transfer overnight to accomplish an infestation of 30-50 aphids per plant and the host leaves were removed. The infested plants were then sprayed by an automated electrostatic plant sprayer equipped with an atomizing spray nozzle. The plants were dried in the sprayer fume hood, removed, and then maintained in a growth room under fluorescent lighting in a 14:10 light:dark photoperiod at about 25°C and about 20-40% relative humidity. Aphid mortality on the treated plants, relative to mortality on untreated control plants, was determined after 5 days. In this test, compound of formula I.1 at 10 ppm showed over 70% mortality in comparison with untreated controls. In this test, compounds of formula I.2, I.3, and I.4 at 10 ppm showed over 70% mortality in comparison with untreated controls. In this test, compounds of formula I.5, I.6, I.7, I.8, I.9, I.10, I.11, I.12, I.13, I.14, I.15, I.16, I.20, I.21, and I.22, at 800 ppm showed over 70% mortality in comparison with untreated controls.

### Red spider Mite (Tetranychus kanzawai)

The active compound of formula ())s dissolved at the desired concentration in a mixture of 1:1 (vol:vol) distilled water : acetone. Add surfactant (Kinetic) is added at a rate of 0.01% (vol/vol).The test solution is prepared at the day of use.Potted cowpea beans of 4-5 days of age are cleaned with tap water and sprayed with 1-2 ml of the test solution using air driven DeVilbiss^{®} hand atomizer at 20- 30 psi (≈ 1,38 to 2,07 bar). The treated plants are allowed to air dry and afterwards inoculated with 30 or more mites by clipping a cassava leaf section from rearing population. Treated plants are placed inside a holding room at about 25-26°C and about 65-70% relative humidity. Estimate percent mortality is assessed 72 hours after treatment. In this test, compounds of formula I.2, I.3 and I.4 at 10 ppm showed over 70% mortality in comparison with untreated controls. In this test, compounds of formula I.5, I.6, I.7, I.8, I.9, I.10, I.12, I.13, I.14, I.16, I.20, I.24 at 800 ppm showed over 70% mortality in comparison with untreated controls.

### Green Soldier Stink Bug (Nezara viridula)

The active compound is dissolved at the desired concentration in a mixture of 1:1 (vol:vol) distilled water : aceteone. Surfactant (Kinetic) is added at a rate of 0.01% (vol/vol).The test solution is prepared at the day of use. Soybean pods are placed in 90 x 50 mm glass Petri dishes lined with moistened filter paper and inoculated with ten late 3rd instar *N. viridula.* Using a hand atomizer, an approximately 2 ml solution is sprayed into each Petri dish. Treated set-up is kept at about 25-26°C and relative humidity of about 65-70%. Percent mortality is recorded after 5 days. In this test, compounds I.5, I.6 at 300 ppm showed over 70% mortality in comparison with untreated controls.

## Claims

1. Compounds of formula (I), or an agrochemically or veterinarily acceptable salt, stereoisomer, tautomer, or N-oxide thereof 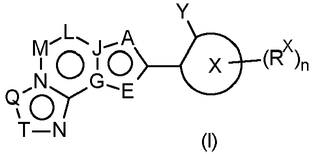
wherein the variables in formula (I) have the following meaning
A is CH, N, or NH;
E is N, O, S, NR^{E}, or CR^{E};
G, J are independently C or N, provided that only one of E or G is N;
L is N or CR^{L};
M is N or CR^{M};
Q is N or CR^{Q}
T is N or CR^{T};
X is phenyl, or a 5- or 6-membered heteroaryl;
Y is SR^{Y1}, S(O)R^{Y1}, S(O)₂R^{Y1}, S(=O)(=NR^{Y2})R^{Y1}, or S(=NR^{Y2})(=NR^{Y3})R^{Y1};
R^{E}, R^{L}, R^{M}, R^{Q} and R^{T} are independently H, halogen, N₃, CN, NO₂, SCN, SF₅;
C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, which groups are halogenated or non-halogenated,
C(=O)OR¹, NR²R³, C₁-C₆-alkylen-NR²R³, O-C₁-C₆-alkylen-NR²R³, C₁-C₆-alkylen-CN, NH-C₁-C₆-alkylen-NR²R³, C(=O)NR²R³, C(=O)R⁴, SO₂NR²R³, S(=O)ₘR⁵, OR⁶, SR⁶, or CH₂R⁶;
phenyl, which is unsubstituted or substituted with one or more, same or different substituents R¹¹;
R¹ H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which groups are halogenated or non-halogenated;
C₁-C₆-alkylen-NR²R³, C₁-C₆-alkylen-CN, or CH₂R⁶; or
phenyl, which is unsubstituted, or substituted with one or more, same or different substituents R¹¹;
R¹¹ is halogen, N₃, OH, CN, NO₂, SCN, SF₅;
C₁-C₆-alkyl, C₁-C₆-alkoxy, C₂-C₆-alkenyl , C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are halogenated or non-halogenated;
R² is H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted, or substituted with one or more, same or different substitutent selected from halogen, CN and HO.
C(=O)R²¹, C(=O)OR²¹, C(=O)NR²¹, C₁-C₆-alkylen-CN, or CH₂R⁶; or
phenyl, which is unsubstituted or substituted with one or more, same or different substituents R¹¹;
R²¹ is H;
C₁-C₆-alkyl, C₁-C₆-haloalkyl, C₂-C₆-alkenyl, C₂-C₆-alkynyl, C₁-C₆-alkoxy-C₁-C₄-alkyl, C₃-C₆-cycloalkyl;
C₃-C₆-cycloalkyl-C₁-C₄-alkyl, C₃-C₆-cycloalkoxy-C₁-C₄ alkyl, phenyl, or a saturated, partially-, or fully unsaturated 5- or 6-membered heterocycle, wherein the cyclic moieties are unsubstituted or substituted with one or more, same or different substituents R¹¹;
R³ is H;
C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are halogenated or non-halogenated;
C₁-C₆-alkylen-CN, or CH₂R⁶;
phenyl, which is unsubstituted or substituted with one or more, same or different substituents R¹¹; or
NR²R³ may also form an N-bound, saturated 3- to 8-membered heterocycle, which in addition to the nitrogen atom may have 1 or 2 further heteroatoms or heteroatom moieties selected from O, S(=O)ₘ, NH, and N-C₁-C₆-alkyl, and wherein the N-bound heterocycle is unsubstituted or substituted with one or more, same or different substituents selected from halogen, C₁-C₄-alkyl, C₁-C₄-haloalkyl, C₁-C₄-alkoxy and C₁-C₄-haloalkoxy;
R⁴ is H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which are unsubstituted or substituted with one or more, same of different substituents selected from halogen, CN, and OH;
CH₂R⁶, or phenyl, which is unsubstituted, or substituted with one or more, same or different substituents R¹¹;
R⁵ is C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, or C₃-C₆-cycloalkoxy-C₁-C₄-alkyl, which groups are halogenated or non-halogenated;
C₁-C₆-alkylen-NR²R³, C₁-C₆-alkylen-CN, CH₂R⁶; or
phenyl, which is unsubstituted, or substituted with one or more, same or different substituents R¹¹;
R⁶ is phenyl, which is unsubstituted or substituted with one or more, same or different substituents R¹¹;
each R^{X} is independently halogen, N₃, OH, CN, NO₂, SCN, SF₅;
C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which groups are unsubstituted or substituted with CN or halogen;
C(=O)OR¹, NR²R³, C(=O)NR²R³, C(=O)R⁴, SO₂NR²R³, S(=O)ₘR¹, OR⁶, SR⁶, CH₂R⁶, OC(=O)R⁴, NR³C(=O)R⁴, OC(=O)OR¹, OC(=O)NR²R³, OC(=O)SR¹, OC(=S)NR²R³, OC(=S)SR¹, ONR²R³, ON=CR¹R⁴, N=CR¹R⁴, NNR², NR³C(=O)R⁷, SC(=O)SR¹, SC(=O)NR²R³, C(=S)R⁶, C(=S)OR⁴, C(=NR²)R⁴, C(R⁸)=NO(R⁹);
phenyl, which is unsubstituted or substituted with one or more, same or different substituents R¹¹;
a 5- or 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents R³¹, and wherein said N- and S-atoms are independently oxidized, or non-oxidized; or
a group of formula (S) 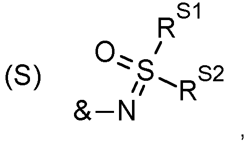
wherein each R^{S1}, R^{S2} is independently selected from C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or halogenated;
a 3- to 6-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said N- and S-atoms are independently oxidized, or non-oxidized;
phenyl, which is unsubstituted, or substituted with one or more, same or different substituents selected from halogen, C₁-C₃-alkyl, C₁-C₃-alkoxy, C₁-C₃-haloalkyl, and C₁-C₃-haloalkoxy;
or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1}, and R^{S2} are bound to;
R³¹ is halogen, N₃, OH, CN, NO₂, SCN, SF₅;
C₁-C₆-alkyl, C₁-C₆-haloalkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₁-C₆-alkoxycarbonyl, C₃-C₆-cycloalkyl;
C₃-C₆-cycloalkoxy, C₃-C₆-cycloalkyl-C₁-C₄-alkyl, C₃-C₆-cycloalkoxy-C₁-C₄ alkyl, phenyl, or a saturated, partially-, or fully unsaturated 5- or 6-membered heterocycle, wherein the cyclic moieties are unsubstituted or substituted with one or more, same or different substituents R¹¹; or
two geminal substituents R³¹ form together with the atom to which they are bound a group =O or =S;
each R⁷ is independently C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from CN and halogen;
each R⁸ is independently H, CN, OH, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with halogen;
phenyl or benzyl, wherein the phenyl ring is unsubstituted or substituted with one or more, same or different substituents R¹¹;
each R⁹ is independently H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which groups are unsubstituted or substituted with one or more, same or different substituents selected from halogen and CN;
phenyl or benzyl, wherein the phenyl ring is unsubstituted or substituted with one or more, same or different substitutents R¹¹;
each R^{Y1} R^{Y2}, R^{Y3} is independently selected from H, C₁-C₆-alkyl, C₃-C₆-cycloalkyl, C₂-C₆-alkenyl, C₃-C₆-cycloalkenyl, C₂-C₆-alkynyl, C₃-C₆-cycloalkyl-C₁-C₃-alkyl, which groups are unsubstituted, or substituted with one or more, same or different substituents selected from halogen and CN;
a 3- to 12-membered saturated, partially unsaturated, or fully unsaturated heterocyclic ring or ring system, wherein said heterocyclic ring or ring system comprises one or more, same or different heteroatoms O, N, or S, and is unsubstituted, or substituted with one or more, same or different substituents selected from halogen, CN, C₁-C₃-alkyl, C₁-C₃-alkoxy, C₁-C₃-haloalkyl, C₁-C₃-haloalkoxy, and wherein said N- and S-atoms are independently oxidized, or non-oxidized;
phenyl, which is unsubstituted, or substituted with one or more, same or different substituents selected from halogen, CN, C₁-C₃-alkyl, C₁-C₃-alkoxy, C₁-C₃-haloalkyl, C₁-C₃-haloalkoxy;
or two substituents selected from R^{Y1}, R^{Y2}, R^{Y3} form, together with the N- or S-atoms to which they are bound, a 5- or 6- membered saturated, partially unsaturated, or fully unsaturated heterocycle, which heterocycle is unsubstituted, or substituted with one or more, same or different substituents selected from halogen, CN, C₁-C₃-alkyl, C₁-C₃-alkoxy, C₁-C₃-haloalkyl, C₁-C₃-haloalkox, and wherein said heterocycle comprises no, one or more, same or different heteroatoms O, N, or S in addition to the S- or N-atoms to which the two substituents selected from R^{Y1},R^{Y2} and R^{Y3} are bound to;
the index n is 0, 1, 2, 3, or 4 if X is phenyl or a 6-membered heteroaryl; or 0, 1, 2, or 3 if X is a 5-membered heteroaryl; and
the index m is 0, 1, or 2.

2. Compounds of formula (I) according to claim 1, wherein A is N.

3. Compounds of formula (I) according to any of claims 1 or 2, wherein formula (I) is of formula (I-A), (I-B), or (I-G)

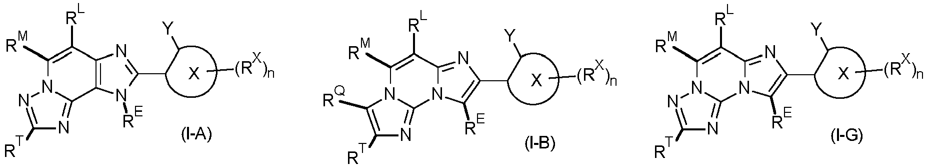

4. Compounds of formula (I) according to any of claims 1 to 3, wherein X is phenyl or 2-pyridyl.

5. Compounds of formula (I) according to any of claims 1 to 4, wherein R^{L}, R^{M}, R^{Q} and R^{T} are independently H, C₁-C₃-alkyl, C₁-C₃-alkoxy, C₁-C₃-haloalkyl, or C₁-C₃-haloalkoxy.

6. Compounds of formula (I) according to any of claims 1 to 5, wherein Y is SO₂R^{Y1}, or S(=O)(=NR^{Y2})R^{Y1}, wherein R^{Y1} is C₁-C₃-alkyl or C₁-C₃-haloalkyl, and R^{Y2} is H, C₁-C₃-alkyl, or C₁-C₃-haloalkyl.

7. Compounds of formula (I) according to any of claims 1 to 6, wherein
each R^{X} is independently halogen;
C₁-C₃-alkyl, C₁-C₃-alkoxy, C₃-C₆-alkoxy, which groups are unsubstituted or substituted with CN or halogen;
NR³C(=O)R⁷, C(R⁸)=N-O(R⁹);
phenyl, which is unsubstituted or halogenated;
a 5- or 6-membered saturated, partially unsaturated, or fully unsaturated heterocyclic ring, wherein said heterocyclic ring comprises one or more, same or different heteroatoms O, N, or S, and is unsubstituted, or substituted with one or more, same or different substituents selected from halogen, CN, C₁-C₃-alkyl, or C₁-C₃-haloalkyl, and wherein two substituents may form together with the carbon-atom to which they are bound a group (C=O), and wherein said N- and S-atoms are independently oxidized, or non-oxidized; or
a group of formula (S) 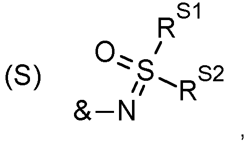
wherein each R^{S1}, R^{S2} is independently selected from C₁-C₃-alkyl, which groups are unsubstituted, or halogenated;
or two substituents R^{S1}, R^{S2} form, together with the sulfur atom to which they are bound, a 5- or 6- membered saturated, partially unsaturated, or fully unsaturated heterocycle, which heterocycle is unsubstituted, or substituted with one or more, same or different substituents selected from halogen, C₁-C₃-alkyl, C₁-C₃-alkoxy, C₁-C₃-haloalkyl, and C₁-C₃-haloalkoxy, and wherein said heterocycle comprises no, one or more, same or different heteroatoms O, N, or S in addition to the sulfur atom to which R^{S1} and R^{S2} are bound to.

8. Compounds of formula (I) according to any of claims 1 to 7, wherein R^{E} is H; or CH₃, which is unsubstituted or halogenated.

9. Use of compounds of formula (I) as defined in any of claims 1 to 8 as an agrochemical pesticide, wherein methods for treatment of the human or animal body by surgery or therapy are excluded.

10. Pesticidal mixture comprising a compound of formula (I) as defined in any of claims 1 to 8, and another agrochemically active ingredient, preferably a pesticide, more preferably an insecticide and/or fungicide.

11. Agrochemical or veterinary compositions comprising a compound of formula (I) as defined in any of claims 1 to 8, or a pesticidal mixture as defined in claim 9, and a liquid or solid carrier.

12. Methods for controlling invertebrate pests, infestation, or infection by invertebrate pests, comprising contacting the pests, their food supply, habitat, breeding grounds or their locus with a compound of formula (I) as defined in any of claims 1 to 8 or a pesticidal mixture as defined in claim 10 in pesticidally effective amounts, wherein methods for treatment of the human or animal body by surgery or therapy are excluded.

13. Seed, comprising a compound of formula (I) as defined in any of claims 1 to 8, or a pesticidal mixture as defined in claim 10 in an amount of from 0.1 g to 10 kg per 100 kg of seeds.

## Patentansprüche

1. Verbindungen der Formel (I) oder agrochemisch oder tiermedizinisch unbedenkliches Salz, Stereoisomer, Tautomer oder N-Oxid davon, 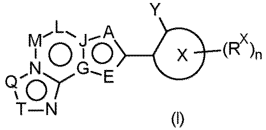 wobei die Variablen in Formel (I) die folgenden Bedeutungen aufweisen:
A steht für CH, N oder NH;
E steht für N, O, S, NR^{E} oder CR^{E};
G, J stehen unabhängig für C oder N, mit der Maßgabe, dass nur eines von E oder G für N steht;
L steht für N oder CR^{L};
M steht für N oder CR^{M};
Q steht für N oder CR^{Q};
T steht für N oder CR^{T};
X steht für Phenyl oder ein 5- oder 6-gliedriges Heteroaryl;
Y steht für SR^{Y1}, S(O)R^{Y1}, S(O)₂R^{Y1}, S(=O)(=NR^{Y2})R^{Y1} oder S(=NR^{Y2})(=NR^{Y3})R^{Y1};
R^{E}, R^{L}, R^{M}, R^{Q} und R^{T} stehen unabhängig für H, Halogen, N₃, CN, NO₂, SCN, SF₅;
C₁-C₆-Alkyl, C₁-C₆-Alkoxy, C₂-C₆-Alkenyl, Tri-C₁-C₆-alkylsilyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄-alkyl, C₃-C₆-Cycloalkoxy-C₁-C₄-alkyl, wobei diese Gruppen halogeniert oder nicht halogeniert sind,
C(=O)OR¹, NR²R³, C₁-C₆-Alkylen-NR²R³, O-C₁-C₆-Alkylen-NR²R³, C₁-C₆-Alkylen-CN, NH-C₁-C₆-Alkylen-NR²R³, C(=O)NR²R³, C(=O)R⁴, SO₂NR²R³, S(=O)ₘR⁵, OR⁶, SR⁶ oder CH₂R⁶;
Phenyl, das unsubstituiert oder durch einen oder mehrere, gleiche oder verschiedene Substituenten R¹¹ substituiert ist;
R¹ H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen halogeniert oder nicht halogeniert sind;
C₁-C₆-Alkylen-NR²R³, C₁-C₆-Alkylen-CN, oder CH₂R⁶; oder
Phenyl, das unsubstituiert oder durch einen oder mehrere, gleiche oder verschiedene Substituenten R¹¹ substituiert ist;
R¹¹ steht für Halogen, N₃, OH, CN, NO₂, SCN, SF₅;
C₁-C₆-Alkyl, C₁-C₆-Alkoxy, C₂-C₆-Alkenyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄-alkyl, C₃-C₆-Cycloalkoxy-C₁-C₄-alkyl, wobei diese Gruppen halogeniert oder nicht halogeniert sind;
R² steht für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch einen oder mehrere, gleiche oder verschiedene Substituenten, die aus Halogen, CN und HO ausgewählt sind, substituiert sind;
C(=O)R²¹, C(=O)OR²¹, C(=O)NR²¹, C₁-C₆-Alkylen-CN oder CH₂R⁶; oder
Phenyl, das unsubstituiert oder durch einen oder mehrere, gleiche oder verschiedene Substituenten R¹¹ substituiert ist;
R²¹ steht für H;
C₁-C₆-Alkyl, C₁-C₆-Halogenalkyl, C₂-C₆-Alkenyl, C₂-C₆-Alkinyl, C₁-C₆-Alkoxy-C₁-C₄-alkyl, C₃-C₆-Cycloalkyl;
C₃-C₆-Cycloalkyl-C₁-C₄-alkyl, C₃-C₆-Cycloalkoxy-C₁-C₄-alkyl, Phenyl oder einen gesättigten, teilweise oder vollständig ungesättigten 5- oder 6-gliedrigen Heterocyclus, wobei die cyclischen Gruppierungen unsubstituiert oder durch einen oder mehrere, gleiche oder verschiedene Substituenten R¹¹ substituiert sind;
R³ steht für H;
C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen halogeniert oder nicht halogeniert sind;
C₁-C₆-Alkylen-CN oder CH₂R⁶;
Phenyl, das unsubstituiert oder durch einen oder mehrere, gleiche oder verschiedene Substituenten R¹¹ substituiert ist; oder
NR²R³ kann auch einen N-gebundenen, gesättigten 3- bis 8-gliedrigen Heterocyclus bilden, der zusätzlich zu dem Stickstoffatom 1 oder 2 weitere Heteroatome oder Heteroatomgruppierungen, die aus O, S(=o)ₘ, NH und N-C₁-C₆-Alkyl ausgewählt sind, aufweisen kann, und wobei der N-gebundene Heterocyclus unsubstituiert oder durch einen oder mehrere, gleiche oder verschiedene Substituenten, die aus Halogen, C₁-C₄-Alkyl, C₁-C₄-Halogenalkyl, C₁-C₄-Alkoxy and C₁-C₄-Halogenalkoxy ausgewählt sind, substituiert ist;
R⁴ steht für H, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl oder C₃-C₆-Cycloalkoxy-C₁-C₄-alkyl, die unsubstituiert oder durch einen oder mehrere, gleiche oder verschiedene Substituenten, die aus Halogen, CN und OH ausgewählt sind, subsituiert sind;
CH₂R⁶ oder Phenyl, das unsubstituiert oder durch einen oder mehrere, gleiche oder verschiedene Substituenten R¹¹ substituiert ist;
R⁵ steht für C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl oder C₃-C₆-Cycloalkoxy-C₁-C₄-alkyl, wobei diese Gruppen halogeniert oder nicht halogeniert sind;
C₁-C₆-Alkylen-NR²R³, C₁-C₆-Alkylen-CN, CH₂R⁶; oder
Phenyl, das unsubstituiert oder durch einen oder mehrere, gleiche oder verschiedene Substituenten R¹¹ substituiert ist;
R⁶ steht für Phenyl, das unsubstituiert oder durch einen oder mehrere, gleiche oder verschiedene Substituenten R¹¹ substituiert ist;
R^{X} steht jeweils unabhängig für Halogen, N₃, OH, CN, NO₂, SCN, SF₅;
C₁-C₆-Alkyl, C₁-C₆-Alkoxy, C₂-C₆-Alkenyl, Tri-C₁-C₆-alkylsilyl, C₂-C₆-Alkinyl, C₁-C₆-Alkoxy-C₁-C₄-alkyl, C₁-C₆-Alkoxy-C₁-C₄-alkoxy, C₃-C₆-Cycloalkyl, C₃-C₆-Cycloalkoxy, C₃-C₆-Cycloalkyl-C₁-C₄-alkyl, C₃-C₆-Cycloalkoxy-C₁-C₄-alkyl, wobei diese Gruppen unsubstituiert oder durch CN oder Halogen substituiert sind,
C(=O)OR¹, NR²R³, C(=O)NR²R³, C(=O)R⁴, SO₂NR²R³, S(=O)ₘR¹, OR⁶, SR⁶, CH₂R⁶, OC (=O) R⁴, NR³C(=O)R⁴, OC(=O)OR¹, OC (=O) NR²R³, OC(=O)SR¹, OC(=S)NR²R³, OC(=S)SR¹, ONR²R³, ON=CR¹R⁴, N=CR¹R⁴, NNR², NR³C(=O) R⁷, SC (=O) SR¹, SC(=O)NR²R³, C(=S)R⁶, C(=S)OR⁴, C(=NR²)R⁴, C(R⁸)=NO(R⁹);
Phenyl, das unsubstituiert oder durch einen oder mehrere, gleiche oder verschiedene Substituenten R¹¹ substituiert ist;
einen 5- oder 6-gliedrigen gesättigten, teilweise ungesättigten oder vollständig ungesättigten heterocyclischen Ring, wobei der heterocyclische Ring ein oder mehrere, gleiche oder verschiedene Heteroatome O, N oder S umfasst und unsubstituiert oder durch einen oder mehrere, gleiche oder verschiedene Substituenten R³¹ substituiert ist und wobei die N- und S-Atome unabhängig oxidiert oder nicht oxidiert sind; oder
eine Gruppe der Formel (S) 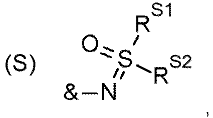
wobei R^{S1}, R^{S2} jeweils unabhängig ausgewählt sind aus C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkyl-C₁-C₃-alkyl , wobei diese Gruppen unsubstituiert oder halogeniert sind;
einem 3- bis 6-gliedrigen gesättigten, teilweise ungesättigten oder vollständig ungesättigten heterocyclischen Ring oder Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten, die aus Halogen, C₁-C₃-Alkyl, C₁-C₃-Alkoxy, C₁-C₃-Halogenalkyl und C₁-C₃-Halogenalkoxy ausgewählt sind, substituiert ist und wobei die N- und S-Atome unabhängig oxidiert oder nicht oxidiert sind;
Phenyl, das unsubstituiert oder durch einen oder mehrere, gleiche oder verschiedene Substituenten, die aus Halogen, C₁-C₃-Alkyl, C₁-C₃-Alkoxy, C₁-C₃-Halogenalkyl und C₁-C₃-Halogenalkoxy ausgewählt sind, substituiert ist;
oder zwei Substituenten R^{S1}, R^{S2} zusammen mit dem Schwefelatom, an das sie gebunden sind, einen 5- oder 6-gliedrigen gesättigten oder teilweise ungesättigten oder vollständig ungesättigten Heterocyclus bilden, wobei der Heterocyclus unsubstituiert oder durch einen oder mehrere, gleiche oder verschiedene Substituenten, die aus Halogen, C₁-C₃-Alkyl, C₁-C₃-Alkoxy, C₁-C₃-Halogenalkyl und C₁-C₃-Halogenalkoxy ausgewählt sind, substituiert ist und wobei der Heterocyclus kein, ein oder mehrere, gleiche oder verschiedene Heteroatome O, N oder S zusätzlich zu dem Schwefelatom, an das R^{S1} und R^{S2} gebunden sind, umfasst;
R³¹ steht für Halogen, N₃, OH, CN, NO₂, SCN, SF₅;
C₁-C₆-Alkyl, C₁-C₆-Halogenalkyl, C₁-C₆-Alkoxy, C₂-C₆-Alkenyl, C₂-C₆-Alkinyl, C₁-C₆-Alkoxy-C₁-C₄-alkyl, C₁-C₆-Alkoxy-C₁-C₄-alkoxy, C₁-C₆-Alkoxycarbonyl, C₃-C₆-Cycloalkyl;
C₃-C₆-Cycloalkoxy, C₃-C₆-Cycloalkyl-C₁-C₄-alkyl, C₃-C₆-Cycloalkoxy-C₁-C₄-alkyl, Phenyl oder einen gesättigten, teilweise oder vollständig ungesättigten 5- oder 6-gliedrigen Heterocyclus, wobei die cyclischen Gruppierungen unsubstituiert oder durch einen oder mehrere, gleiche oder verschiedene Substituenten R¹¹ substituiert sind; oder
zwei geminale Substituenten R³¹ bilden zusammen mit dem Atom, an das sie gebunden sind, eine =O- oder =S-Gruppe; R⁷ steht jeweils unabhängig für C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch eine oder mehrere, gleiche oder verschiedene Substituenten, die aus CN und Halogen ausgewählt sind, substituiert sind;
R⁸ steht jeweils unabhängig für H, CN, OH, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl, C₃-C₆-Cycloalkoxy-C₁-C₄-alkyl, wobei diese Gruppen unsubstituiert oder durch Halogen substituiert sind;
Phenyl oder Benzyl, wobei der Phenylring unsubstituiert oder durch einen oder mehrere, gleiche oder verschiedene Substituenten R¹¹ substituiert ist;
R⁹ steht jeweils unabhängig für H, C₁-C₆-Alkyl, C₂-C₆-Alkenyl, C₂-C₆-Alkinyl, C₁-C₆-Alkoxy-C₁-C₄-alkyl, C₃-C₆-Cycloalkyl, C₃-C₆-Cycloalkyl-C₁-C₄-alkyl oder C₃-C₆-Cycloalkoxy-C₁-C₄-alkyl, wobei diese Gruppen unsubstituiert oder durch einen oder mehrere, gleiche oder verschiedene Substituenten, die aus Halogen und CN ausgewählt sind, substituiert sind;
Phenyl oder Benzyl, wobei der Phenylring unsubstituiert oder durch einen oder mehrere, gleiche oder verschiedene Substituenten R¹¹ substituiert ist;
R^{Y1}, R^{Y2}, R^{Y3} sind jeweils unabhängig ausgewählt aus H, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkyl-C₁-C₃-alkyl, wobei die Gruppen unsubstituiert oder durch einen oder mehrere, gleiche oder verschiedene Substituenten, die aus Halogen und CN ausgewählt sind, substituiert sind;
einem 3- bis 12-gliedrigen gesättigten, teilweise ungesättigten oder vollständig ungesättigten heterocyclischen Ring oder Ringsystem, wobei der heterocyclische Ring bzw. das heterocyclische Ringsystem ein oder mehrere, gleiche oder verschiedene Heteroatome O, N oder S umfasst und unsubstituiert oder durch einen oder mehrere, gleiche oder verschiedene Substituenten, die aus Halogen, CN, C₁-C₃-Alkyl, C₁-C₃-Alkoxy, C₁-C₃-Halogenalkyl, C₁-C₃-Halogenalkoxy ausgewählt sind, substituiert ist und wobei die N- und S-Atome unabhängig oxidiert oder nicht oxidiert sind;
Phenyl, das unsubstituiert oder durch einen oder mehrere, gleiche oder verschiedene Substituenten, die aus Halogen, CN, C₁-C₃-Alkyl, C₁-C₃-Alkoxy, C₁-C₃-Halogenalkyl, C₁-C₃-Halogenalkoxy ausgewählt sind, substituiert ist;
oder zwei Substituenten, die aus R^{Y1}, R^{Y2}, R^{Y3} ausgewählt sind, bilden zusammen mit den N- oder S-Atomen, an die sie gebunden sind, einen 5- oder 6-gliedrigen gesättigten, teilweise ungesättigten oder vollständig ungesättigten Heterocyclus, wobei der Heterocyclus unsubstituiert oder durch einen oder mehrere, gleiche oder verschiedene Substituenten, die aus Halogen, CN, C₁-C₃-Alkyl, C₁-C₃-Alkoxy, C₁-C₃-Halogenalkyl, C₁-C₃-Halogenalkoxy ausgewählt sind, substituiert ist und wobei der Heterocyclus kein, ein oder mehrere, gleiche oder verschiedene Heteroatome O, N oder S zusätzlich zu dem S- oder N-Atomen, an die die zwei aus R^{Y1}, R^{Y2} und R^{Y3} ausgewählten Substituenten gebunden sind, umfasst;
der Index n steht für 0, 1, 2, 3 oder 4, wenn X für Phenyl oder ein 6-gliedriges Heteroaryl steht;
oder für 0, 1, 2 oder 3, wenn X für ein 5-gliedriges Heteroaryl steht; und der Index m steht für 0, 1 oder 2.

2. Verbindungen der Formel (I) nach Anspruch 1, wobei A für N steht.

3. Verbindungen der Formel (I) nach Anspruch 1 oder 2, wobei Formel (I) der Formel (I-A), (I-B) oder (I-G) entspricht.

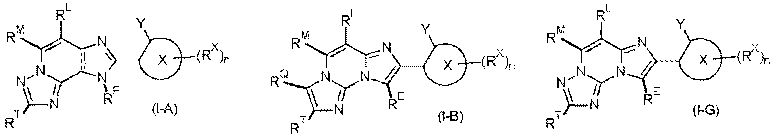

4. Verbindungen der Formel (I) nach einem der Ansprüche 1 bis 3, wobei X für Phenyl oder 2-Pyridyl steht.

5. Verbindungen der Formel (I) nach einem der Ansprüche 1 bis 4, wobei R^{L}, R^{M}, R^{Q} und R^{T} unabhängig für H, C₁-C₃-Alkyl, C₁-C₃-Alkoxy, C₁-C₃-Halogenalkyl oder C₁-C₃-Halogenalkoxy stehen.

6. Verbindungen der Formel (I) nach einem der Ansprüche 1 bis 5, wobei Y für SO₂R^{Y1} oder S(=O) (=NR^{Y2})R^{Y1} steht, wobei R^{Y1} für C₁-C₃-Alkyl oder C₁-C₃-Halogenalkyl steht und R^{Y2} für H, C₁-C₃-Alkyl oder C₁-C₃-Halogenalkyl steht.

7. Verbindungen der Formel (I) nach einem der Ansprüche 1 bis 6, wobei
R^{X} jeweils unabhängig für Halogen;
C₁-C₃-Alkyl, C₁-C₃-Alkoxy, C₃-C₆-Alkoxy, wobei diese Gruppen unsubstituiert oder durch CN oder Halogen substituiert sind;
NR³C(=O)R⁷, C(R⁸)=N-O(R⁹);
Phenyl, das unsubstituiert oder halogeniert ist;
einen 5- oder 6-gliedrigen gesättigten, teilweise ungesättigten oder vollständig ungesättigten heterocyclischen Ring, wobei der heterocyclische Ring ein oder mehrere, gleiche oder verschiedene Heteroatome O, N oder S umfasst und unsubstituiert oder durch einen oder mehrere, gleiche oder verschiedene Substituenten, die aus Halogen, CN, C₁-C₃-Alkyl oder C₁-C₃-Halogenalkyl ausgewählt sind, substituiert ist und wobei zwei Substituenten zusammen mit dem Kohlenstoffatom, an das sie gebunden sind, eine (C=O)-Gruppe bilden können und wobei die N- oder S-Atome unabhängig oxidiert oder nicht oxidiert sind; oder
eine Gruppe der Formel (S) 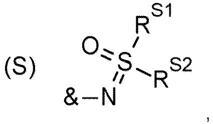
steht, wobei R^{S1}, R^{S2} jeweils unabhängig aus C₁-C₃-Alkyl ausgewählt sind, wobei diese Gruppen unsubstituiert oder halogeniert sind;
oder zwei Substituenten R^{S1}, R^{S2} zusammen mit dem Schwefelatom, an das sie gebunden sind, einen 5- oder 6-gliedrigen gesättigten oder teilweise ungesättigten oder vollständig ungesättigten Heterocyclus bilden, wobei der Heterocyclus unsubstituiert oder durch einen oder mehrere, gleiche oder verschiedene Substituenten, die aus Halogen, C₁-C₃-Alkyl, C₁-C₃-Alkoxy, C₁-C₃-Halogenalkyl und C₁-C₃-Halogenalkoxy ausgewählt sind, substituiert ist und wobei der Heterocyclus kein, ein oder mehrere, gleiche oder verschiedene Heteroatome O, N oder S zusätzlich zu dem Schwefelatom, an das R^{S1} und R^{S2} gebunden sind, umfasst.

8. Verbindungen der Formel (I) nach einem der Ansprüche 1 bis 7, wobei R^{E} für H oder CH₃, das unsubstituiert oder halogeniert ist, steht.

9. Verwendung von Verbindungen der Formel (I) gemäß einem der Ansprüche 1 bis 8 als agrochemisches Pestizid, wobei Verfahren zur chirurgischen oder therapeutischen Behandlung des menschlichen oder tierischen Körpers ausgeschlossen sind.

10. Pestizide Mischung, umfassend eine Verbindung der Formel (I) gemäß einem der Ansprüche 1 bis 8 und einen anderen agrochemisch Wirkstoff, vorzugsweise ein Pestizid, weiter bevorzugt ein Insektizid und/oder Fungizid.

11. Agrochemische oder tiermedizinische Zusammensetzungen, umfassend eine Verbindung der Formel (I) gemäß einem der Ansprüche 1 bis 8 oder eine pestizide Mischung gemäß Anspruch 9 und einen flüssigen oder festen Träger.

12. Verfahren zum Bekämpfen von wirbellosen Schädlingen, Befall oder Infektion durch wirbellose Schädlinge, umfassend das In-Kontakt-Bringen der Schädlinge, ihrer Nahrungsquelle, ihres Lebensraums, ihrer Brutstätten oder ihres Standorts mit einer Verbindung der Formel (I) gemäß einem der Ansprüche 1 bis 8 oder einer pestiziden Mischung gemäß Anspruch 10 in pestizid wirksamen Mengen, wobei Verfahren zur chirurgischen oder therapeutischen Behandlung des menschlichen oder tierischen Körpers ausgeschlossen sind.

13. Saatgut, umfassend eine Verbindung der Formel (I) gemäß einem der Ansprüche 1 bis 8 oder eine pestizide Mischung gemäß Anspruch 10 in einer Menge von 0,1 g bis 10 kg pro 100 kg Saatgut.

## Revendications

1. Composés de formule (I), ou sel acceptable sur le plan agrochimique ou sur le plan vétérinaire, stéréoisomère, forme tautomère ou N-oxyde correspondant (e) 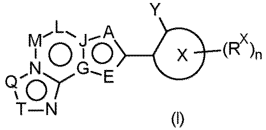 les variables dans la formule (I) ayant la signification suivante
A étant CH, N, ou NH ;
E étant N, O, S, NR^{E}, ou CR^{E} ;
G, J étant indépendamment C ou N, à condition que seulement un parmi E ou G soit N ;
L étant N ou CR^{L} ;
M étant N ou CR^{M} ;
Q étant N ou CR^{Q}
T étant N ou CR^{T} ;
X étant phényle, ou un hétéroaryle à 5 ou 6 chaînons ; Y étant SR^{Y1}, S(O)R^{Y1}, S(O)₂R^{Y1}, S(=O) (=NR^{Y2})R^{Y1}, ou S (=NR^{Y2})(=NR^{Y3})R^{Y1} ;
R^{E}, R^{L}, R^{M}, R^{Q} et R^{T} étant indépendamment H, halogène, N₃, CN, NO₂, SCN, SF₅ ;
C₁-C₆-alkyle, C₁-C₆-alcoxy, C₂-C₆-alcényle, tri-C₁-C₆-alkylsilyle, C₂-C₆-alcynyle, C₁-C₆-alcoxy-C₁-C₄-alkyle, C₁-C₆-alcoxy-C₁-C₄-alcoxy, C₃-C₆-cycloalkyle, C₃-C₆-cycloalcoxy, C₃-C₆-cycloalkyl-C₁-C₄-alkyle, C₃-C₆-cycloalcoxyx-C₁-C₄-alkyle, lesquels groupes étant halogénés ou non halogénés,
C(=O)OR¹, NR²R³, C₁-C₆-alkylène-NR²R³, O-C₁-C₆-alkylène-NR²R³, C₁-C₆-alkylène-CN, NH-C₁-C₆-alkylène-NR²R³, C(=O)NR²R³, C(=O)R⁴, SO₂NR²R³, S(=O)ₘR⁵, OR⁶, SR⁶, ou CH₂R⁶ ; phényle, qui est non substitué ou substitué par un ou plusieurs substituants identiques ou différents R¹¹ ;
R¹ H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, ou C₃-C₆-cycloalcoxy-C₁-C₄-alkyle, lesquels groupes étant halogénés ou non halogénés ;
C₁-C₆-alkylène-NR²R³, C₁-C₆-alkylène-CN, ou CH₂R⁶ ; ou
phényle, qui est non substitué ou substitué par un ou plusieurs substituants identiques ou différents R¹¹ ;
R¹¹ étant halogène, N₃, OH, CN, NO₂, SCN, SF₅ ;
C₁-C₆-alkyle, C₁-C₆-alcoxy, C₂-C₆-alcényle, C₂-C₆-alcynyle, C₁-C₆-alcoxy-C₁-C₄-alkyle, C₁-C₆-alcoxy-C₁-C₄-alcoxy, C₃-C₆-cycloalkyle, C₃-C₆-cycloalcoxy, C₃-C₆-cycloalkyl-C₁-C₄-alkyle, C₃-C₆-cycloalcoxy-C₁-C₄-alkyle, lesquels groupes étant halogénés ou non halogénés ;
R² étant H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par un ou plusieurs substituants identiques ou différents choisis parmi halogène, CN et HO.
C(=O)R²¹, C(=O)OR²¹, C(=O)NR²¹ C₁-C₆-alkylène-CN, ou CH₂R⁶ ; ou
phényle, qui est non substitué ou substitué par un ou plusieurs substituants identiques ou différents R¹¹ ;
R²¹ étant H ;
C₁-C₆-alkyle, C₁-C₆-halogénoalkyle, C₂-C₆-alcényle, C₂-C₆-alcynyle, C₁-C₆-alcoxy-C₁-C₄-alkyle, C₃-C₆-cycloalkyle ;
C₃-C₆-cycloalkyl-C₁-C₄-alkyle, C₃-C₆-cycloalcoxy-C₁-C₄ alkyle, phényle, ou un hétérocycle saturé, partiellement ou totalement insaturé à 5 ou 6 chaînons, les groupements cycliques étant non substitués ou substitués par un ou plusieurs substituants identiques ou différents R¹¹ ;
R³ étant H ;
C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant halogénés ou non halogénés ;
C₁-C₆-alkylène-CN, ou CH₂R⁶ ;
phényle, qui est non substitué ou substitué par un ou plusieurs substituants identiques ou différents R¹¹ ; ou NR²R³ pouvant également former un hétérocycle N-lié, saturé à 3 à 8 chaînons, qui en plus de l'atome d'azote peut avoir 1 ou 2 hétéroatomes ou groupements d'hétéroatomes supplémentaires choisis parmi O, S(=O)_{m,} NH, et N-C₁-C₆-alkyle, et l'hétérocycle N-lié étant non substitué ou substitué par un ou plusieurs substituants identiques ou différents choisis parmi halogène, C₁-C₄-alkyle, C₁-C₄-halogénoalkyle, C₁-C₄-alcoxy et C₁-C₄-halogénoalcoxy ;
R⁴ étant H, C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, ou C₃-C₆-cycloalcoxy-C₁-C₄-alkyle, qui sont non substitués ou substitués par un ou plusieurs substituants identiques ou différents choisis parmi halogène, CN, et OH ;
CH₂R⁶, ou phényle, qui est non substitué ou substitué par un ou plusieurs substituants identiques ou différents R^{m} ;
R⁵ ; étant C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, ou C₃-C₆-cycloalcoxy-C₁-C₄-alkyle, lesquels groupes étant halogénés ou non halogénés ;
C₁-C₆-alkylène-NR²R³, C₁-C₆-alkylène-CN, CH₂R⁶ ; ou
phényle, qui est non substitué ou substitué par un ou plusieurs substituants identiques ou différents R¹¹ ;
R⁶ étant phényle, qui est non substitué ou substitué par un ou plusieurs substituants identiques ou différents R¹¹ ;
chaque R^{X} étant indépendamment halogène, N₃, OH, CN, NO₂, SCN, SF₅ ;
C₁-C₆-alkyle, C₁-C₆-alcoxy, C₂-C₆-alcényle, tri-C₁-C₆-alkylsilyle, C₂-C₆-alcynyle, C₁-C₆-alcoxy-C₁-C₄-alkyle, C₁-C₆-alcoxy-C₁-C₄-alcoxy, C₃-C₆-cycloalkyle, C₃-C₆-cycloalcoxy, C₃-C₆-cycloalkyl-C₁-C₄-alkyle, C₃-C₆-cycloalcoxy-C₁-C₄-alkyle, lesquels groupes étant non substitués ou substitués par CN ou halogène ;
C(=O)OR¹, NR²R³, C(=O)NR²R³, C(=O)R⁴, SO₂NR²R³, S(=O)ₘR¹, OR⁶, SR⁶, CH₂R⁶, OC(=O)R⁴, NR³C(=O)R⁴, OC(=O)OR¹, OC (=O) NR²R³, OC(=O)SR¹, OC(=S)NR²R³, OC(=S)SR¹, ONR²R³, ON=CR¹R⁴, N=CR¹R⁴, NNR², NR³C(=O)R⁷, SC(=O)SR¹, SC(=O)NR²R³, C(=S)R⁶, C(=S)OR⁴, C(=NR²)R⁴, C(R⁸)=NO(R⁹) ;
phényle, qui est non substitué ou substitué par un ou plusieurs substituants identiques ou différents R¹¹ ;
un cycle hétérocyclique saturé, partiellement insaturé ou totalement insaturé à 5 ou 6 chaînons, ledit cycle hétérocyclique comprenant un ou plusieurs hétéroatomes identiques ou différents O, N, ou S, et étant non substitué ou substitué par un ou plusieurs substituants identiques ou différents R³¹, et lesdits atomes de N et de S étant indépendamment oxydés ou non oxydés ; ou
un groupe de formule (S) 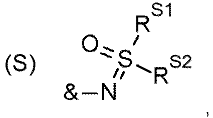
chaque R^{S1}, R^{S2} étant indépendamment choisi parmi C₁-C₆-alkyle, C₃-C₆-cycloalkyle, C₂-C₆-alcényle, C₃-C₆-cycloalcényle, C₂-C₆-alcynyle, C₃-C₆-cycloalkyl-C₁-C₃-alkyle, lesquels groupes étant non substitués ou halogénés ;
un cycle ou système cyclique hétérocyclique saturé, partiellement insaturé ou totalement insaturé à 3 à 6 chaînons, ledit cycle ou système cyclique hétérocyclique comprenant un ou plusieurs hétéroatomes identiques ou différents O, N, ou S, et étant non substitué ou substitué par un ou plusieurs substituants identiques ou différents choisis parmi halogène, C₁-C₃-alkyle, C₁-C₃-alcoxy, C₁-C₃-halogénoalkyle, et C₁-C₃-halogénoalcoxy, et lesdits atomes de N et de S étant indépendamment oxydés ou non oxydés ;
phényle, qui est non substitué ou substitué par un ou plusieurs substituants identiques ou différents choisis parmi halogène, C₁-C₃-alkyle, C₁-C₃-alcoxy, C₁-C₃-halogénoalkyle, et C₁-C₃-halogénoalcoxy ;
ou deux substituants R^{S1}, R^{S2} formant, conjointement avec l'atome de soufre auquel ils sont liés, un hétérocycle saturé, partiellement insaturé ou totalement insaturé à 5 ou 6 chaînons, lequel hétérocycle étant non substitué ou substitué par un ou plusieurs substituants identiques ou différents choisis parmi halogène, C₁-C₃-alkyle, C₁-C₃-alcoxy, C₁-C₃-halogénoalkyle, et C₁-C₃-halogénoalcoxy, et ledit hétérocycle ne comprenant pas de, ou comprenant un ou plusieurs hétéroatomes identiques ou différents O, N, ou S en plus de l'atome de soufre auquel R^{S1}, et R^{S2} sont liés ;
R³¹ étant halogène, N₃, OH, CN, NO₂, SCN, SF₅ ; C₁-C₆-alkyle, C₁-C₆-halogénoalkyle, C₁-C₆-alcoxy, C₂-C₆-alcényle, C₂-C₆-alcynyle, C₁-C₆-alcoxy-C₁-C₄-alkyle, C₁-C₆-alcoxy-C₁-C₄-alcoxy, C₁-C₆-alcoxycarbonyle, C₃-C₆-cycloalkyle ;
C₃-C₆-cycloalcoxy, C₃-C₆-cycloalkyl-C₁-C₄-alkyle, C₃-C₆-cycloalcoxy-C₁-C₄ alkyle, phényle, ou un hétérocycle saturé, partiellement ou totalement insaturé à 5 ou 6 chaînons, les groupements cycliques étant non substitués ou substitués par un ou plusieurs substituants identiques ou différents R¹¹ ; ou
deux substituants géminaux R³¹ formant conjointement avec l'atome auquel ils sont liés un groupe =O ou =S ;
chaque R⁷ étant indépendamment C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par un ou plusieurs substituants identiques ou différents choisis parmi CN et halogène ;
chaque R⁸ étant indépendamment H, CN, OH, C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par halogène ;
phényle ou benzyle, le cycle phényle étant non substitué ou substitué par un ou plusieurs substituants identiques ou différents R¹¹ ;
chaque R⁹ étant indépendamment H, C₁-C₆-alkyle, C₂-C₆-alcényle, C₂-C₆-alcynyle, C₁-C₆-alcoxy-C₁-C₄-alkyle, C₃-C₆-cycloalkyle, C₃-C₆-cycloalkyl-C₁-C₄-alkyle, C₃-C₆-cycloalcoxy-C₁-C₄-alkyle, lesquels groupes étant non substitués ou substitués par un ou plusieurs substituants identiques ou différents choisis parmi halogène et CN ;
phényle ou benzyle, le cycle phényle étant non substitué ou substitué par un ou plusieurs substituants identiques ou différents R¹¹ ;
chaque R^{Y1}, R^{Y2}, R^{Y3} étant indépendamment choisi parmi H, C₁-C₆-alkyle, C₃-C₆-cycloalkyle, C₂-C₆-alcényle, C₃-C₆-cycloalcényle, C₂-C₆-alcynyle, C₃-C₆-cycloalkyl-C₁-C₃-alkyle, lesquels groupes étant non substitués ou substitués par un ou plusieurs substituants identiques ou différents choisis parmi halogène et CN ;
un cycle ou système cyclique hétérocyclique saturé, partiellement insaturé ou totalement insaturé à 3 à 12 chaînons, ledit cycle ou système cyclique hétérocyclique comprenant un ou plusieurs hétéroatomes identiques ou différents O, N, ou S, et étant non substitué ou substitué par un ou plusieurs substituants identiques ou différents choisis parmi halogène, CN, C₁-C₃-alkyle, C₁-C₃-alcoxy, C₁-C₃-halogénoalkyle, C₁-C₃-halogénoalcoxy, et lesdits atomes de N et de S étant indépendamment oxydés ou non oxydés ;
phényle, qui est non substitué ou substitué par un ou plusieurs substituants identiques ou différents choisis parmi halogène, CN, C₁-C₃-alkyle, C₁-C₃-alcoxy, C₁-C₃-halogénoalkyle, C₁-C₃-halogénoalcoxy ;
ou deux substituants choisis parmi R^{Y1}, R^{Y2}, R^{Y3} formant, conjointement avec les atomes de N ou de S auxquels ils sont liés, un hétérocycle saturé, partiellement insaturé ou totalement insaturé à 5 ou 6 chaînons, lequel hétérocycle étant non substitué ou substitué par un ou plusieurs substituants identiques ou différents choisis parmi halogène, CN, C₁-C₃-alkyle, C₁-C₃-alcoxy, C₁-C₃-halogénoalkyle, C₁-C₃-halogénoalcoxy, et ledit hétérocycle ne comprenant pas de, ou comprenant un ou plusieurs hétéroatomes identiques ou différents O, N, ou S en plus des atomes de S ou de N auxquels les deux substituants choisis parmi R^{Y1}, R^{Y2} et R^{Y3} sont liés ;
l'indice n étant 0, 1, 2, 3, ou 4 si X est phényle ou un hétéroaryle à 6 chaînons ;
ou 0, 1, 2, ou 3 si X est un hétéroaryle à 5 chaînons ; et l'indice m étant 0, 1, ou 2.

2. Composés de formule (I) selon la revendication 1, A étant N.

3. Composés de formule (I) selon l'une quelconque des revendications 1 ou 2, la formule (I) étant de formule (I-A), (I-B), ou (I-G)

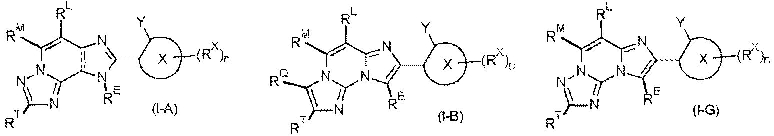

4. Composés de formule (I) selon l'une quelconque des revendications 1 à 3, X étant phényle ou 2-pyridinyle.

5. Composés de formule (I) selon l'une quelconque des revendications 1 à 4, R^{L}, R^{M}, R^{Q} et R^{T} étant indépendamment H, C₁-C₃-alkyle, C₁-C₃-alcoxy, C₁-C₃-halogénoalkyle, ou C₁-C₃-halogénoalcoxy.

6. Composés de formule (I) selon l'une quelconque des revendications 1 à 5, Y étant SO₂R^{Y1}, ou S(=O)(=NR^{Y2})R^{Y1}, R^{Y1} étant C₁-C₃-alkyle ou C₁-C₃-halogénoalkyle, et R^{Y2} étant H, C₁-C₃-alkyle, ou C₁-C₃-halogénoalkyle.

7. Composés de formule (I) selon l'une quelconque des revendications 1 à 6,
chaque R^{X} étant indépendamment halogène ;
C₁-C₃-alkyle, C₁-C₃-alcoxy, C₃-C₆-alcoxy, lesquels groupes étant non substitués ou substitués par CN ou halogène ; NR³C (=O) R⁷, C(R⁸)=N-O(R⁹) ;
phényle, qui est non substitué ou halogéné ;
un cycle hétérocyclique saturé, partiellement insaturé ou totalement insaturé à 5 ou 6 chaînons, ledit cycle hétérocyclique comprenant un ou plusieurs hétéroatomes identiques ou différents O, N, ou S, et étant non substitué ou substitué par un ou plusieurs substituants identiques ou différents choisis parmi halogène, CN, C₁-C₃-alkyle, ou C₁-C₃-halogénoalkyle, et deux substituants pouvant former conjointement avec l'atome de carbone auquel ils sont fixés un groupe (C=O), et lesdits atomes de N et de S étant indépendamment oxydés ou non oxydés ; ou
un groupe de formule (S) 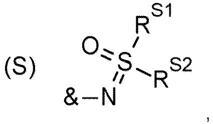
chaque R^{S1}, R^{S2} étant indépendamment choisi parmi C₁-C₃-alkyle, lesquels groupes étant non substitués ou halogénés ;
ou deux substituants R^{S1}, R^{S2} formant, conjointement avec l'atome de soufre auquel ils sont liés, un hétérocycle saturé, partiellement insaturé ou totalement insaturé à 5 ou 6 chaînons, lequel hétérocycle étant non substitué ou substitué par un ou plusieurs substituants identiques ou différents choisis parmi halogène, C₁-C₃-alkyle, C₁-C₃-alcoxy, C₁-C₃-halogénoalkyle, et C₁-C₃-halogénoalcoxy, et ledit hétérocycle ne comprenant pas de, ou comprenant un ou plusieurs hétéroatomes identiques ou différents O, N, ou S en plus de l'atome de soufre auquel R^{S1} et R^{S2} sont liés.

8. Composés de formule (I) selon l'une quelconque des revendications 1 à 7, R^{E} étant H ; ou CH₃, qui est non substitué ou halogéné.

9. Utilisation de composés de formule (I) tels que définis dans l'une quelconque des revendications 1 à 8 en tant que pesticide agrochimique, dans laquelle les procédés de traitement du corps humain ou animal par chirurgie ou thérapie sont exclus.

10. Mélange pesticide comprenant un composé de formule (I) tel que défini dans l'une quelconque des revendications 1 à 8, et un autre ingrédient actif sur le plan agrochimique, de préférence un pesticide, plus préférablement un insecticide et/ou un fongicide.

11. Compositions agrochimiques ou vétérinaires comprenant un composé de formule (I) tel que défini dans l'une quelconque des revendications 1 à 8, ou un mélange pesticide tel que défini dans la revendication 9, et un support liquide ou solide.

12. Procédés pour lutter contre les organismes nuisibles invertébrés, une infestation ou une infection par des organismes nuisibles invertébrés, comprenant la mise en contact des organismes nuisibles, de leur approvisionnement alimentaire, de leur habitat, de leurs aires de reproduction ou de leur site avec un composé de formule (I) tel que défini dans l'une quelconque des revendications 1 à 8 ou un mélange pesticide tel que défini dans la revendication 10 en des quantités efficaces du point de vue pesticide, dans lesquels les procédés pour le traitement du corps humain ou animal par chirurgie ou thérapie sont exclus.

13. Graine comprenant un composé de formule (I), tel que défini dans l'une quelconque des revendications 1 à 8, ou un mélange pesticide tel que défini dans la revendication 10 en une quantité allant de 0,1 g à 10 kg pour 100 kg de graines.
